# FASCICULE DE BREVET EUROPEEN

(11) **EP 3 707 132 B1**
(45) Date de publication et mention de la délivrance du brevet: **17.07.2024**
(21) Numéro de dépôt: 18830902.5
(22) Date de dépôt: 28.09.2018
(51) Int. Cl.: C07D 317/58, C07C 209/16, C07C 211/21, C07C 211/27, C07C 211/29, C07C 213/02, C07C 319/20, C07C 217/58, C07C 217/84, C07C 323/32, C07D 307/52

(54) **NOUVEAU PROCÉDÉ DE SYNTHÈSE D'AMINES TERTIAIRES DISSYMÉTRIQUES**
NEUES VERFAHREN ZUR SYNTHESE VON UNSYMMETRISCHEN TERTIÄREN AMINEN
NEW METHOD FOR THE SYNTHESIS OF UNSYMMETRICAL TERTIARY AMINES

(30) Priorité: 29.09.2017 FR 1759117
(43) Date de publication de la demande: 16.09.2020
(73) Titulaire: Université Paris-Saclay, 91190 Gif-sur-Yvette (FR); Centre National de la Recherche Scientifique, 75794 Paris Cedex 16 (FR)
(72) Inventeur: BOUR, Christophe, 91410 Dourdan (FR); VAYER, Marie Jenny Jacqueline, 02520 Flavy le Martel (FR); GANDON, Vincent, 91290 Arpajon (FR)
(74) Mandataire: Grosset-Fournier, Chantal Catherine
(86) Numéro de dépôt international: PCT/FR2018/052396
(87) Numéro de publication internationale: WO 2019/063952

(56) Documents cités:
- JESÚS CAMPOS ET AL: "Methanol Dehydrogenation by Iridium N-Heterocyclic Carbene Complexes", INORGANIC CHEMISTRY, vol. 54, no. 11, 15 June 2015 (2015-06-15), EASTON, US, pages 5079 - 5084, XP055483083, ISSN: 0020-1669, DOI: 10.1021/ic502521c
- SOLENNE MOULIN ET AL: "Bifunctional (Cyclopentadienone)Iron-Tricarbonyl Complexes: Synthesis, Computational Studies and Application in Reductive Amination", CHEMISTRY - A EUROPEAN JOURNAL, vol. 19, no. 52, 15 November 2013 (2013-11-15), DE, pages 17881 - 17890, XP055483506, ISSN: 0947-6539, DOI: 10.1002/chem.201302432

## Description

La présente invention concerne un nouveau procédé de synthèse d'amines tertiaires dissymétriques utilisant un alcool et une imine, ainsi que de nouvelles amines tertiaires, tels que définis dans les revendications.

La mise au point de réactions simples pour la formation de liaisons C-N représente un défi important pour les chimistes. Les amines substituées ont de larges applications dans la synthèse des médicaments, colorants, détergents, parfums, produits pharmaceutiques, émulsifiants, agents de protection des cultures, etc.

Parmi les différentes stratégies de synthèse d'amines, le développement de réactions d'amination directe d'alcools permettant la génération rapide d'amines secondaires ou tertiaires, à partir de matériaux de départ simples et facilement disponibles, est à la pointe de la synthèse chimique fine et implique un fort potentiel de développement industriel.

Parmi ces méthodes, l'amination directe des alcools par emprunt d'hydrogène (ou transfert d'hydrogène) a été reconnue comme l'une des plus pratiques pour la production industrielle d'alkylamines trisubstituées. Cette réaction économe en atome ainsi que la large disponibilité des alcools, combinée avec le fait que l'eau est le seul sous-produit de ces réactions, répondant aux principes généraux de la chimie verte, sont les raisons de son utilisation à grande échelle.

Cette amination a pour la première fois été réalisée avec des métaux de transition tardifs de deuxième et troisième périodes, qui ont été suivis rapidement par des complexes bien plus élaborés (Ru, Rh, Ir, Os). A titre d'exemple, la préparation d'amines tertiaires à partir d'imine et de méthanol en présence d'un catalyseur d'iridium est divulguée par Jesus Campos et al. (Inorg. Chem. 2015, 54, 5079-5084). La tendance actuelle est de remplacer ces éléments nobles par des métaux de transition de la première période moins coûteux et plus abondants. A cet égard, des améliorations significatives ont été récemment réalisées en utilisant des catalyseurs de manganèse, de fer ou de cobalt. Moulin et al. (Chem. Eur. J., 2013, 19, 17881-17890) décrit des catalyseurs à base de fer dans des réactions d'amination réductrice. Cependant, il existe encore un besoin important de catalyseurs encore plus actifs qui travaillent dans des conditions plus douces, qui permettent une plus grande tolérance fonctionnelle du substrat et qui améliorent la sélectivité et le TON (Turn-Over Number).

Si l'on souhaite accéder à la synthèse d'amines tertiaires par cette stratégie, on peut avoir accès uniquement à des amines trisubstituées avec 1 ou 2 groupements différenciés. En revanche, il n'existe pas de méthodes d'amination directe pour l'accès aux amines tertiaires possédant 3 substituants différents. La transformation directe d'amines primaires via la synthèse d'imines en amines tertiaires portant trois substituants différents est ainsi inconnue à ce jour.

L'un des buts de l'invention est la mise à disposition d'un nouveau procédé de synthèse d'amines tertiaires trisubstituées dissymétriques simple à mettre en oeuvre, rapide et applicable à une large variété de substrats.

L'un des autres buts de l'invention est la possibilité d'utiliser un catalyseur dérivé d'un métal abondant et peu coûteux et permettant de travailler dans des conditions douces.

L'un des autres buts de l'invention est la possibilité d'accéder à la synthèse d'une large variété d'amines tertiaires, et ce de façon simple.

Ce nouveau procédé présente les avantages d'être simple à mettre en oeuvre, rapide, applicable à une large variété de substrats, d'utiliser un catalyseur dérivé d'un métal abondant et peu coûteux et permettant de travailler dans des conditions douces.

La présente invention concerne l'utilisation d'un alcool de formule (C) et d'une imine de formule (D) dans la mise en oeuvre d'un procédé de préparation d'amines tertiaires de formule (E), telle que définie dans la revendication 1.

Au sens de l'invention, l'expression «alcool primaire» désigne un composé de formule R-O-H pour lequel l'atome de carbone portant le groupement hydroxyle porte également deux atomes d'hydrogène.

Au sens de l'invention, l'expression «alcool secondaire» désigne un composé de formule R-O-H pour lequel l'atome de carbone portant le groupement hydroxyle porte un seul atome d'hydrogène.

Au sens de l'invention, l'expression «amine tertiaire» désigne un composé de formule N-R₃ pour lequel R n'est pas un atome d'hydrogène.

L'invention concerne l'utilisation telle que définie dans la revendication 1 d'un alcool de formule (**C**)

**(C)** R₄OH

dans laquelle R₄ représente un alkyle en C₂ à C₁₀, ou un cycloalkyle en C₃ à C₁₀ et d'une imine de formule (**D**) 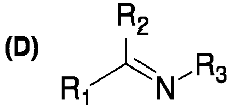
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
dans la mise en oeuvre d'un procédé de préparation d'amines tertiaires de formule (**E**) 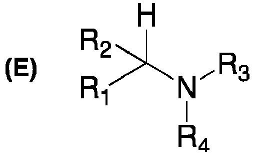
dans laquelle R₁, R₂, R₃, R₄ sont tels que définis ci-dessus dans laquelle la préparation d'amines tertiaires est réalisée en présence d'un catalyseur comprenant un complexe de Fer(0).

Au sens de l'invention, l'expression «alkyle en C₁ à C₁₀» désigne une chaîne carbonée acyclique, saturée, linéaire ou ramifiée, comprenant 1 à 10 atomes de carbone. Des exemples d'alkyles en C₁ à C₁₀ incluent les groupes méthyle, éthyle, propyle, butyle, pentyle, hexyle ou heptyle.

Au sens de l'invention, l'expression «alkyle en C₂ à C₁₀» désigne une chaîne carbonée acyclique, saturée, linéaire ou ramifiée, comprenant 2 à 10 atomes de carbone. Des exemples d'alkyles en C₂ à C₁₀ incluent les groupes éthyle, propyle, butyle, pentyle, hexyle ou heptyle. La définition de propyle, butyle, pentyle, hexyle ou heptyle inclut tous les isomères possibles.

Par exemple, le terme butyle comprend n-butyle, iso-butyle, sec-butyle et tert-butyle. L'alkyle peut être substitué à différentes positions par un ou plusieurs groupements fonctionnels tels que halogène, alcoxyle, amino, nitro, cyano, trifluorométhyle ou ester carboxylique.

Au sens de la présente invention, l'expression «cycloalkyle en C₃ à C₁₀» désigne un mono-, bi- ou tri-cycle saturé ou partiellement saturé, comprenant de 3 à 10 atomes de carbone.

Au sens de l'invention, l'expression «dérivé carbonylé en C₁ à C₁₀» désigne un composé comprenant 1 à 10 atomes de carbone et comportant une double liaison entre un atome de carbone et un atome d'oxygène.

Au sens de l'invention, l'expression «formiate en C₃ à C₁₀» désigne un composé de formule HCOOR comprenant 3 à 10 atomes de carbone.

Selon le mode de réalisation ci-dessus, l'invention concerne l'utilisation d'un alcool de formule (**C**) pouvant être un alcool primaire ou secondaire et d'une imine de formule (**D**) pouvant être une aldimine ou une cétimine.

Au sens de l'invention, l'expression «aldimine» désigne une imine de formule (**D**) 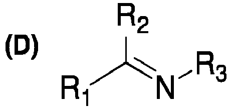 pour laquelle R₂ représente un hydrogène.

Au sens de l'invention, l'expression «cétimine» désigne une imine de formule (**D**) 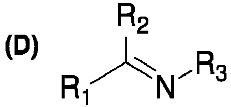 pour laquelle R₂ n'est pas un atome d'hydrogène.

Selon un mode de réalisation particulier, l'invention concerne l'utilisation d'un alcool primaire de formule (**C**)

**(C)** R₄OH

dans laquelle R₄ représente un alkyle en C₂ à C₁₀
et d'une imine de formule (**D**) 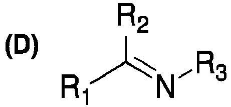
dans laquelle R₁ représente un aryle, allyle, alkyle en C₁ à C₁₀, ou cycloalkyle en C₃ à C₁₀,
R₂ représente un hydrogène,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
dans la mise en oeuvre d'un procédé de préparation d'amines tertiaires de formule (**E**) 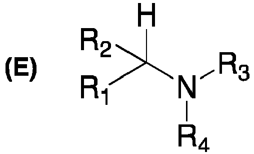
dans laquelle R₁, R₂, R₃, R₄ sont tels que définis ci-dessus, dans laquelle la préparation d'amines tertiaires est réalisée en présence d'un catalyseur comprenant un complexe de Fer(0).

Selon un autre mode de réalisation particulier, l'invention concerne l'utilisation d'un alcool primaire de formule (**C**)

**(C)** R₄OH

dans laquelle R₄ représente un alkyle en C₂ à C₁₀,
et d'une imine de formule (**D**) 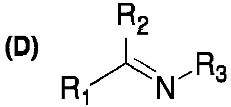
dans laquelle R₁ et R₂ représentent un aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
dans la mise en oeuvre d'un procédé de préparation d'amines tertiaires de formule (**E**) 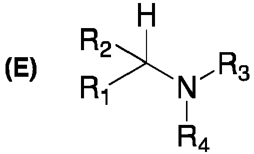
dans laquelle R₁, R₂, R₃, R₄ sont tels que définis ci-dessus, dans laquelle la préparation d'amines tertiaires est réalisée en présence d'un catalyseur comprenant un complexe de Fer(0).

Selon un autre mode de réalisation particulier, l'invention concerne l'utilisation d'un alcool secondaire de formule (**C**)

**(C)** R₄OH

dans laquelle R₄ représente un alkyle en C₂ à C₁₀, ou un cycloalkyle en C₃ à C₁₀,
et d'une imine de formule (**D**) 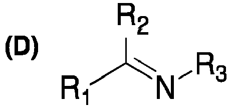
dans laquelle R₁ représente un aryle, allyle, alkyle en C₁ à C₁₀, ou cycloalkyle en C₃ à C₁₀,
R₂ représente un hydrogène,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
dans la mise en oeuvre d'un procédé de préparation d'amines tertiaires de formule (**E**) 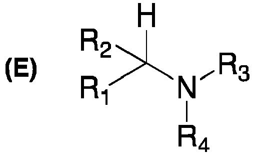
dans laquelle R₁, R₂, R₃, R₄ sont tels que définis ci-dessus, dans laquelle la préparation d'amines tertiaires est réalisée en présence d'un catalyseur comprenant un complexe de Fer(0).

Selon un autre mode de réalisation particulier, l'invention concerne l'utilisation d'un alcool secondaire de formule (**C**)

**(C)** R₄OH

dans laquelle R₄ représente un alkyle en C₂ à C₁₀, ou un cycloalkyle en C₃ à C₁₀, et d'une imine de formule (**D**) 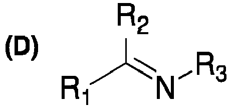
dans laquelle R₁ et R₂ représentent un aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
dans la mise en oeuvre d'un procédé de préparation d'amines tertiaires de formule (**E**) 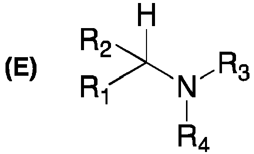
dans laquelle R₁, R₂, R₃, R₄ sont tels que définis ci-dessus, dans laquelle la préparation d'amines tertiaires est réalisée en présence d'un catalyseur comprenant un complexe de Fer(0).

Selon un autre mode de réalisation, l'invention concerne l'utilisation d'un alcool, notamment primaire ou secondaire et d'une imine dans la mise en oeuvre d'un procédé de préparation d'amines tertiaires dans laquelle la préparation d'amines tertiaires est réalisée en présence d'un catalyseur comprenant un complexe de Fer(0) tel que défini dans la revendication 1.

Au sens de l'invention, l'expression «catalyseur» désigne un composé qui permet une réaction chimique ou en augmente la vitesse.

Au sens de l'invention, l'expression « complexe de Fer(0) » désigne un édifice chimique où un atome de Fer au degré d'oxydation 0 est lié à plusieurs ligands.

En effet, les Inventeurs sont parvenus à aller au-delà de la réactivité classique des alcools en utilisant des catalyseurs de Fer(0) pouvant catalyser l'addition d'alcools primaires et secondaires sur des imines par N-alkylation. Ce procédé n'a aucun précédent dans la littérature et permet de synthétiser des amines trisubstituées avec trois groupements différents inaccessibles par des méthodes conventionnelles.

Selon un autre mode de réalisation, l'invention concerne l'utilisation d'un alcool, notamment primaire ou secondaire et d'une imine dans la mise en oeuvre d'un procédé de préparation d'amines tertiaires dans laquelle la préparation d'amines tertiaires est réalisée en présence d'un catalyseur comprenant un complexe de Fer(0), choisi parmi les formules suivantes : 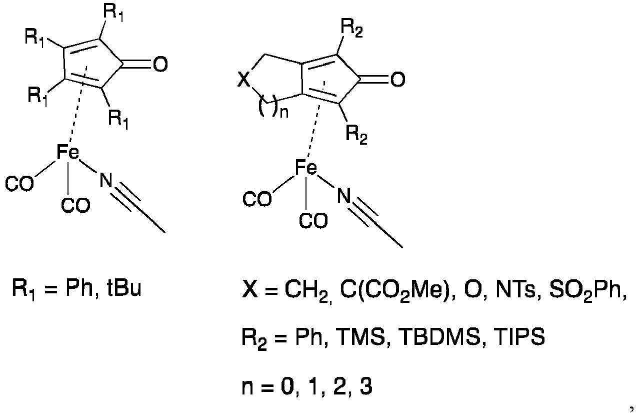 dans lesquelles
Ts = tosyle 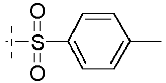
TMS = triméthylsilyle 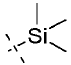
TBDMS = tert-butyldiméthylsilyle 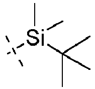
TIPS = triisopropylsilyle 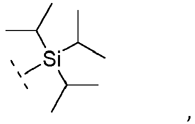
et de préférence répondant à la formule (**B**) suivante : 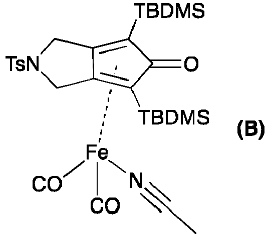 dans laquelle

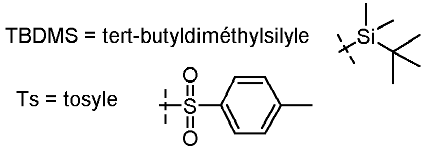

Le catalyseur utilisé est soit le catalyseur de formule (**B**) préparé préalablement à la préparation des amines tertiaires, soit le catalyseur formé *in situ* pendant cette préparation via l'ajout d'oxyde de triméthylamine sur le complexe de formule (**A**) :

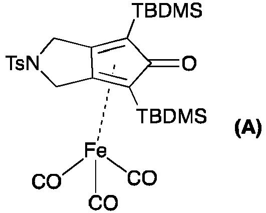

Ce catalyseur de type cyclopentadiènone-tricarbonyl de formule (**A**) doit préalablement être activé par ajout d'un additif, l'oxyde de triméthylamine, afin d'éliminer un ligand de type CO, ce qui le rend actif mais cette étape nécessite l'utilisation d'une boîte à gant. Les Inventeurs ont démontré que le catalyseur préformé de formule (**B**), par stabilisation d'un ligand acétonitrile, était manipulable à l'air ambiant et était tout aussi actif en catalyse.

Selon un autre mode de réalisation, l'invention concerne l'utilisation d'un alcool, notamment primaire ou secondaire et d'une imine dans la mise en oeuvre d'un procédé de préparation d'amines tertiaires dans laquelle la préparation d'amines tertiaires est réalisée en présence d'un catalyseur comprenant un complexe de Fer(0), choisi parmi les formules suivantes : 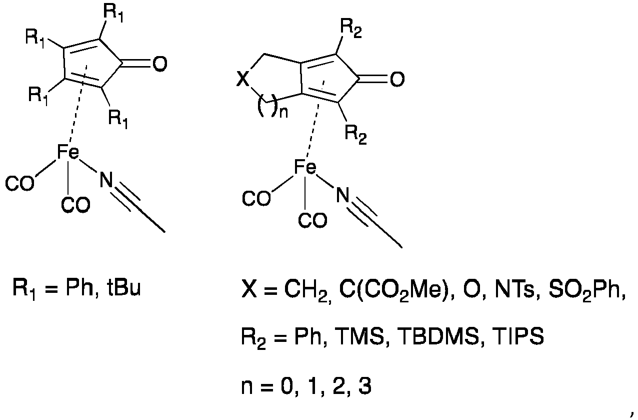 dans lesquelles
Ts = tosyle 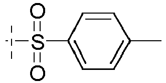
TMS = triméthylsilyle 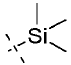
TBDMS = tert-butyldiméthylsilyle 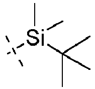
TIPS = triisopropylsilyle 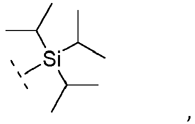 et de préférence répondant à la formule (**B**) suivante : 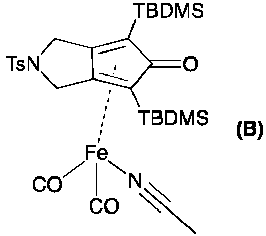 dans laquelle

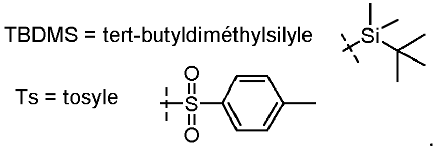

D'autres exemples de catalyseurs pouvant être utilisés sont les suivants :

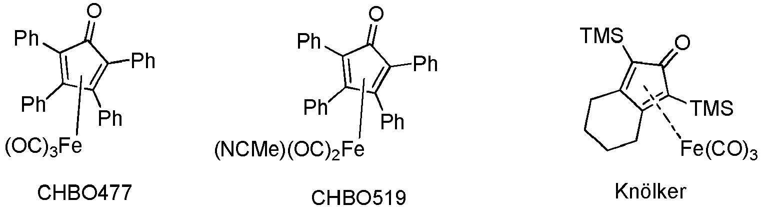

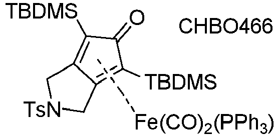

Selon un autre mode de réalisation, l'invention concerne l'utilisation d'un alcool, notamment primaire ou secondaire et d'une imine dans la mise en oeuvre d'un procédé de préparation d'amines tertiaires dans laquelle la préparation d'amines tertiaires est réalisée en présence d'un catalyseur comprenant un complexe de Fer(0), dans laquelle le catalyseur utilisé est soit le complexe de formule (**B**) formé préalablement à la préparation des amines tertiaires, soit le catalyseur formé *in situ* pendant ladite préparation des amines tertiaires par ajout d'oxyde de triméthylamine sur le complexe de formule (**A**) :

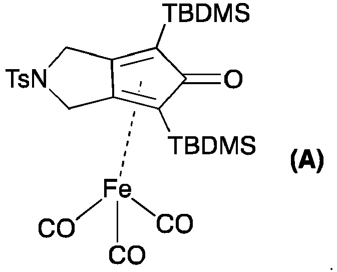

Selon un mode de réalisation particulier, l'invention concerne l'utilisation d'un alcool, notamment primaire ou secondaire et d'une imine dans la mise en oeuvre d'un procédé de préparation d'amines tertiaires, dans laquelle l'alcool de formule (**C**) est choisi parmi l'éthanol, le n-propanol, l'isopropanol, le n-butanol, le sec-butanol, l'isobutanol, l'alcool amylique, l'isopentanol, l'alcool néonpentylique, le méthyl-n-propylcarbinol, l'hexan-1-ol, l'heptan-1-ol, l'octan-1-ol, le nonan-1-ol, le décan-1-ol, l'éthylène glycol.

Selon un autre mode de réalisation particulier, l'imine précitée est choisie parmi

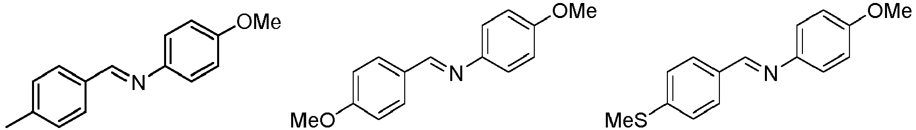

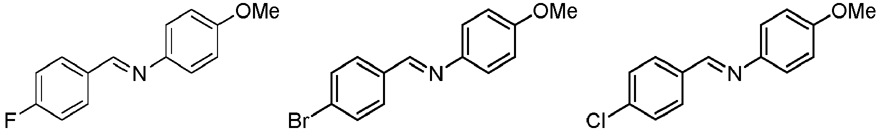

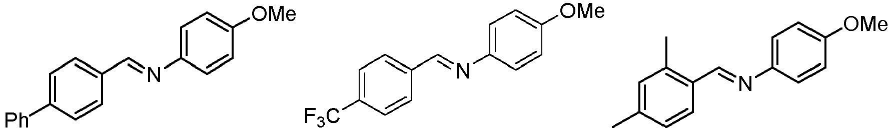

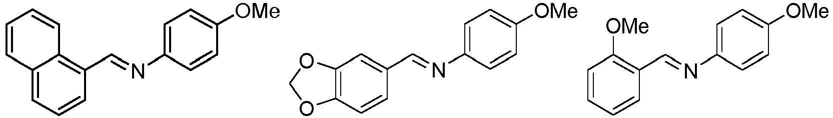

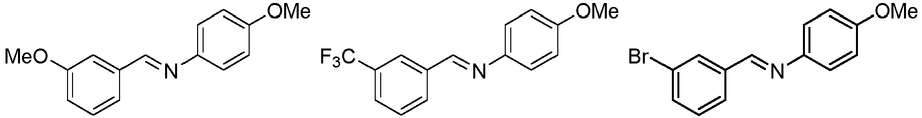

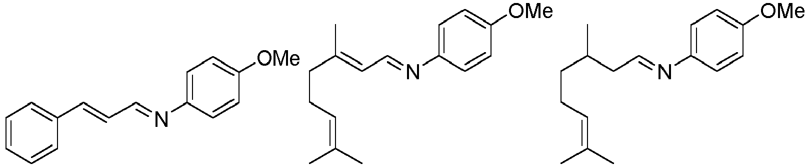

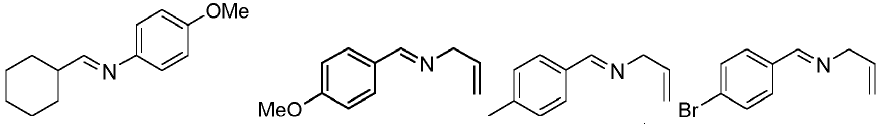

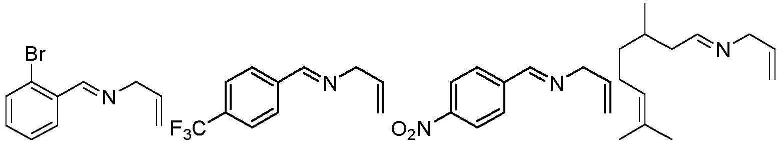

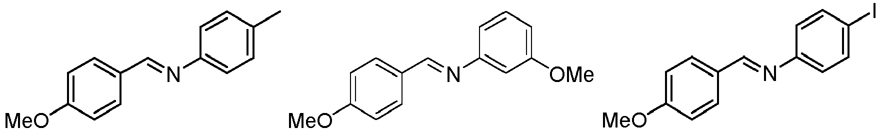

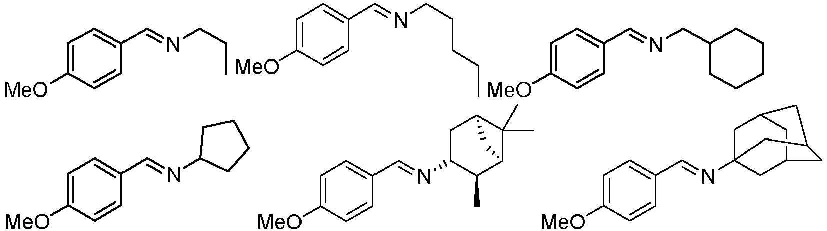

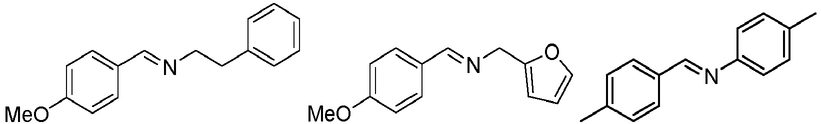

La présente invention concerne également un procédé de préparation tel que défini dans la revendication 3 d'amines tertiaires de formule (**E**) 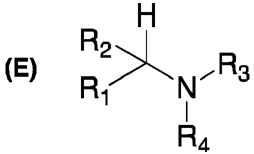
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀, la dite amine tertiaire de formule (**E**) portant notamment trois substituants différents entre eux et différents d'un atome d'hydrogène ;
comprenant une étape d'alkylation d'un alcool de formule (**C**)

   **(C)** R₄OH
dans laquelle R₄ est tel que défini ci-dessus, sur une imine de formule (**D**) 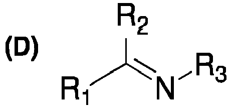
dans laquelle R₁, R₂ et R₃ sont tels que définis ci-dessus, pour obtenir le composé de formule (**E**) tel que défini ci-dessus.

Selon un mode de réalisation particulier, la présente invention concerne un procédé de préparation d'amines tertiaires de formule (**E**) 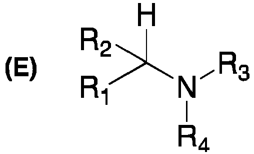
dans laquelle R₁ représente un aryle, allyle, alkyle en C₁ à C₁₀, ou cycloalkyle en C₃ à C₁₀,
R₂ représente un hydrogène,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀;
comprenant une étape d'alkylation d'un alcool de formule (**C**)

   **(C)** R₄OH
dans laquelle R₄ est tel que défini ci-dessus,
sur une imine de formule (**D**) 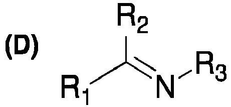
dans laquelle R₁, R₂ et R₃ sont tels que définis ci-dessus,
pour obtenir le composé de formule (**E**) tel que défini ci-dessus.

Selon un autre mode de réalisation particulier, la présente invention concerne un procédé de préparation d'amines tertiaires de formule (**E**) 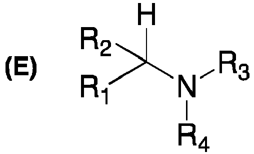
dans laquelle R₁ et R₂ représentent un aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀;
comprenant une étape d'alkylation d'un alcool de formule (**C**)

   **(C)** R₄OH
dans laquelle R₄ est tel que défini ci-dessus,
sur une imine de formule (**D**) 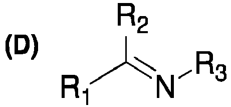
dans laquelle R₁, R₂ et R₃ sont tels que définis ci-dessus,
pour obtenir le composé de formule (**E**) tel que défini ci-dessus.

Selon un mode de réalisation particulier, la présente invention concerne un procédé de préparation d'amines tertiaires de formule (**E**) 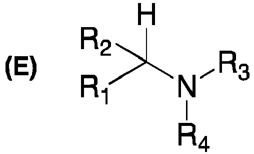
dans laquelle R₁ représente un aryle, allyle, alkyle en C₁ à C₁₀, ou cycloalkyle en C₃ à C₁₀,
R₂ représente un hydrogène,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀ ;
comprenant une étape d'alkylation d'un alcool de formule (**C**)

   **(C)** R₄OH
dans laquelle R₄ est tel que défini ci-dessus,
sur une imine de formule (**D**) 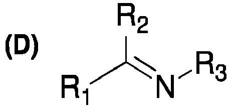
dans laquelle R₁, R₂ et R₃ sont tels que définis ci-dessus,
pour obtenir le composé de formule (**E**) tel que défini ci-dessus.

Selon un mode de réalisation particulier, la présente invention concerne un procédé de préparation d'amines tertiaires de formule (**E**) 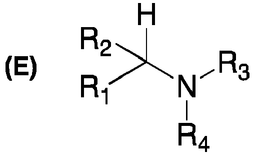
dans laquelle R₁ et R₂ représentent un aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀ ;
comprenant une étape d'alkylation d'un alcool de formule (**C**)

   **(C)** R₄OH
dans laquelle R₄ est tel que défini ci-dessus,
sur une imine de formule (**D**) 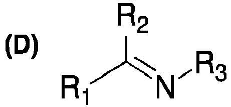
dans laquelle R₁, R₂ et R₃ sont tels que définis ci-dessus,
pour obtenir le composé de formule (**E**) tel que défini ci-dessus.

Selon un autre mode de réalisation, l'invention concerne un procédé de préparation d'amines tertiaires de formule (**E**) 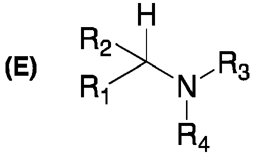
dans laquelle l'amine tertiaire de formule (**E**) porte trois substituants tous différents entre eux et dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀.

Selon un autre mode de réalisation, l'invention concerne un procédé de préparation d'amines tertiaires de formule (**E**), dans lequel l'alcool de formule (**C**) est choisi parmi l'éthanol, le n-propanol, l'isopropanol, le n-butanol, le sec-butanol, l'isobutanol, l'alcool amylique, l'isopentanol, l'alcool néonpentylique, le méthyl-n-propylcarbinol, l'hexan-1-ol, l'heptan-1-ol, l'octan-1-ol, le nonan-1-ol, le décan-1-ol, l'éthylène glycol.

Selon un autre mode de réalisation, l'invention concerne un procédé de préparation d'amines tertiaires de formule (**E**), dans lequel l'étape d'alkylation est catalysée, notamment par un complexe de Fer(0), choisi parmi les formules suivantes : 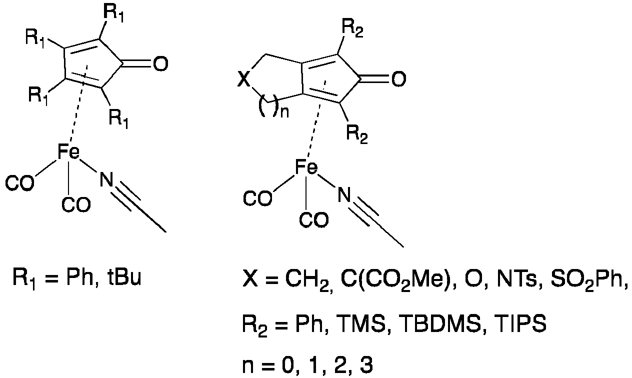 dans lesquelles
Ts = tosyle 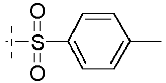
TMS = triméthylsilyle 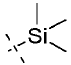
TBDMS = tert-butyldiméthylsilyle 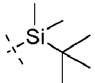
TIPS = triisopropylsilyle 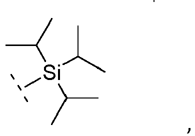 et de préférence répondant à la formule (**B**) suivante : 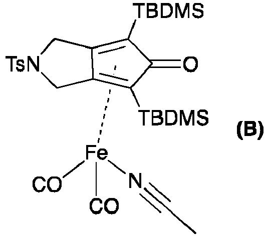 dans laquelle

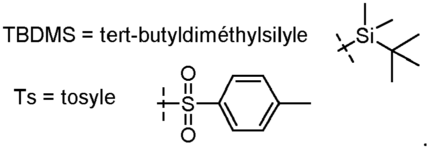

Selon un autre mode de réalisation, l'invention concerne un procédé de préparation d'amines tertiaires de formule (**E**), dans lequel l'étape d'alkylation est catalysée par le complexe de formule (**B**).

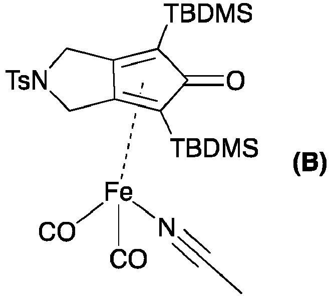

Selon un autre mode de réalisation, l'invention concerne un procédé de préparation d'amines tertiaires de formule (**E**), dans lequel l'étape d'alkylation est catalysée soit par le complexe de formule (**B**) formé préalablement à la préparation des amines tertiaires, soit par le catalyseur formé *in situ* pendant ladite préparation des amines tertiaires par ajout d'oxyde de triméthylamine sur le complexe de formule (**A**).

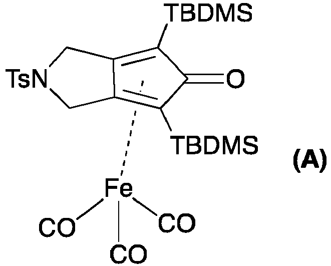

Selon un autre mode de réalisation, l'invention concerne un procédé de préparation d'amines tertiaires de formule (**E**), dans lequel l'étape d'alkylation est effectuée à une température de 80 °C à 130 °C, et de préférence à 110 °C.

Selon un autre mode de réalisation, l'invention concerne un procédé de préparation d'amines tertiaires de formule (**E**), dans lequel l'étape d'alkylation est effectuée dans un solvant organique.

Ce dit solvant organique peut être :
- un solvant unique choisi parmi l'éthanol, l'éthylène glycol, le tétrahydrofurane, le dichloroéthane, le toluène, le méthoxycyclopentane, le diéthyléther.
- un mélange de solvants choisis parmi les solvants précités ou
- l'alcool de formule (**C**) utilisé comme solvant et comme réactif dans le procédé de préparation des amines tertiaires de formule (**E**).

Selon un autre mode de réalisation, l'invention concerne un procédé de préparation d'amines tertiaires de formule (**E**), dans lequel l'étape d'alkylation est effectuée dans l'éthanol ou l'éthylène glycol utilisé comme solvant et comme réactif, ou dans un mélange de solvants composé notamment de THF et d'éthanol ou d'éthylène glycol, l'éthanol ou l'éthylène glycol étant alors utilisé comme solvant et comme réactif.

L'utilisation de l'éthanol, et plus généralement de l'alcool de formule (**C**), à la fois comme solvant et comme réactif dans ce procédé de préparation d'amines tertiaires de formule (**E**) permet de réaliser la réaction en présence d'un large excès de l'un des deux réactifs, ce qui favorise la réaction. Le second intérêt est de s'affranchir de l'utilisation d'un autre solvant, ce qui rend la réaction plus simple à mettre en oeuvre et plus économique.

Selon un autre mode de réalisation, l'invention concerne un procédé de préparation d'amines tertiaires de formule (**E**), dans lequel l'alcool primaire de formule (**C**), notamment l'éthanol ou l'éthylène glycol est également utilisé comme solvant de l'étape d'alkylation, notamment à un nombre d'équivalents supérieur à 10 équivalents, et de préférence 85 équivalents.

L'utilisation d'un solvant composé de l'alcool primaire de formule (**C**) en mélange avec un autre solvant dans le procédé de préparation d'amines tertiaires de formule (**E**) peut se faire selon les proportions solvant/alcool primaire (**C**) allant de 1/1 à 13/1.

Lorsque l'alcool primaire de formule (**C**) est utilisé à la fois comme solvant et comme réactif dans le procédé de préparation d'amines tertiaires de formule (**E**), au moins 10 équivalents d'alcool primaire de formule (**C**) sont utilisés, et de préférence 85 équivalents.

Lorsqu'un solvant organique autre que l'alcool primaire de formule (**C**) est utilisé, 3 équivalents d'alcool primaire de formule (**C**) sont utilisés en tant que réactif.

Selon un autre mode de réalisation, l'invention concerne un procédé de préparation d'amines tertiaires de formule (**E**), dans lequel la concentration en imine de formule (**D**) est comprise de 0.05 M à 0.4 M, en particulier de 0.1 M, 0.2 M, 0.3 M, 0.4 M, et est de préférence de 0.2 M.

Selon un autre mode de réalisation, l'invention concerne un procédé de préparation d'amines tertiaires de formule (**E**), dans lequel la quantité en catalyseur est comprise de 1 à 20 mol%, en particulier de 1 à 5 mol%, 5 à 10 mol%, 10 à 15 mol%, 15 à 20 mol%, et est de préférence de 5 mol%, par rapport à la quantité molaire d'imine.

Selon un mode de réalisation préféré, l'invention concerne un procédé de préparation d'amines tertiaires de formule (**E**), dans lequel l'étape d'alkylation est effectuée à une température de 110°C pendant 24 heures dans l'éthanol avec une concentration en imine de formule (**D**) de 0.2 M et une quantité de catalyseur de 5 mol%.

Selon un autre mode de réalisation, l'invention concerne un procédé de préparation d'amines tertiaires de formule (**E**), dans lequel l'imine de formule (**D**) est soit formée préalablement à la préparation des amines tertiaires, soit formée *in situ* pendant ladite préparation des amines tertiaires par un procédé comprenant la mise en contact d'un aldéhyde ou d'une cétone de formule (**F**) 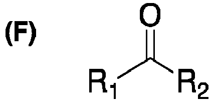
dans laquelle R₁, R₂ représentent un hydrogène, aryle, allyle, alkyle en C₁ à C₁₀, ou cycloalkyle en C₃ à C₁₀,
et d'une amine de formule (**G**)

   **(G)** R₃-NH₂
dans laquelle R₃ représente un aryle, allyle, alkyle en C₁ à C₁₀, cycloalkyle en C₃ à C₁₀, un dérivé carbonylé en C₁ à C₁₀, ou un formiate en C₃ à C₁₀.

Selon le mode de réalisation ci-dessus, l'invention concerne un procédé de préparation d'amines tertiaires de formule (**E**), comprenant une étape de préparation d'amines tertiaires de formule (**E**) comprenant une étape d'alkylation d'un alcool de formule (**C**) sur une imine de formule (**D**) dans lequel l'imine de formule (**D**) est soit formée préalablement à la préparation des amines tertiaires, soit formée *in situ* pendant ladite préparation des amines tertiaires par un procédé comprenant la mise en contact d'un aldéhyde ou d'une cétone de formule (**F**), et d'une amine de formule (**G**), l'étape d'alkylation étant réalisée en présence d'un catalyseur de formule (**B**) soit déjà formé, soit formé *in situ* dans une première étape préalable à l'étape d'alkylation.

Selon un autre mode de réalisation, l'invention concerne un procédé de préparation d'amines tertiaires de formule (**E**), comprenant une étape de préparation d'amines tertiaires de formule (**E**) 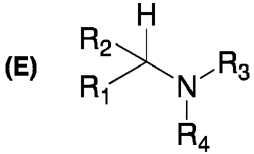
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀ ;
comprenant une étape d'alkylation d'un alcool de formule (**C**)

   **(C)** R₄OH
dans laquelle R₄ est tel que défini ci-dessus,
sur une imine de formule (**D**) 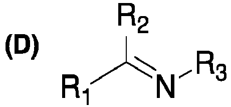
dans laquelle R₁, R₂ et R₃ sont tels que définis ci-dessus,
en présence d'un catalyseur de formule (**B**) 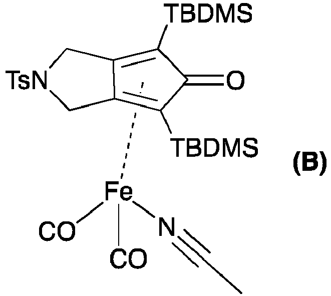
pour obtenir le composé de formule (**E**) tel que défini ci-dessus, ou
comprenant une première étape de préparation du catalyseur
comprenant une étape d'ajout d'oxyde de triméthylamine sur le complexe de formule (**A**) 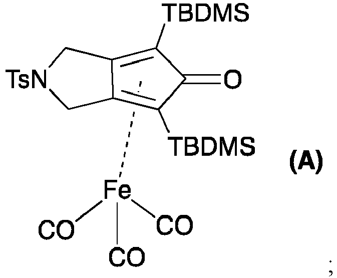
et une seconde étape d'alkylation d'un alcool de formule (**C**)

   **(C)** R₄OH
dans laquelle R₄ représente un alkyle en C₂ à C₁₀, ou un cycloalkyle en C₃ à C₁₀,
sur une imine de formule (**D**) 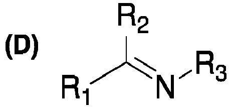
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀, ₑₙ présence d'un catalyseur de formule (**B**) préparé à l'étape précédente et tel que défini ci-dessus,
pour obtenir le composé de formule (**E**) 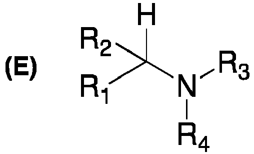
dans laquelle R₁, R₂, R₃ et R₄ sont tels que définis ci-dessus, ou
comprenant une première étape de préparation d'imines de formule (**D**) 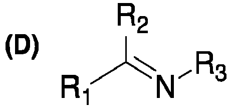
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
comprenant une étape de mise en contact d'un aldéhyde ou d'une cétone de formule (**F**) 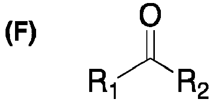
dans laquelle R₁ et R₂ sont tels que définis ci-dessus,
et d'une amine de formule (**G**)

   **(G)** R₃-NH₂
dans laquelle R₃ est tel que défini ci-dessus,
pour obtenir l'imine de formule (**D**) telle que défini ci-dessus ;
et une seconde étape d'alkylation d'un alcool de formule (**C**)

   **(C)** R₄OH
dans laquelle R₄ représente un alkyle en C₂ à C₁₀, ou un cycloalkyle en C₃ à C₁₀,
sur une imine de formule (**D**) préparé à l'étape précédente et telle que définie ci-dessus,
en présence d'un catalyseur de formule (**B**) 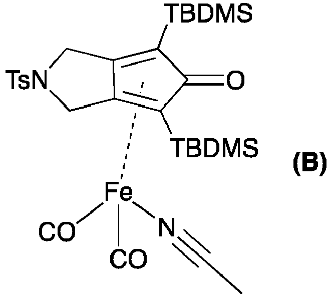
pour obtenir le composé de formule (**E**) 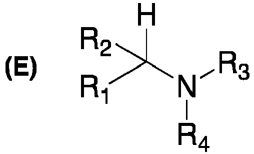
dans laquelle R₁, R₂, R₃ et R₄ sont tels que définis ci-dessus, ou
comprenant une étape préalable de préparation d'imines de formule (**D**) 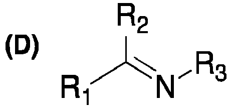
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
comprenant une étape de mise en contact d'un aldéhyde ou une cétone de formule (**F**) 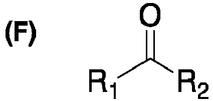
dans laquelle R₁ et R₂ sont tels que définis ci-dessus,
et d'une amine de formule (**G**)

   **(G)** R₃-NH₂
dans laquelle R₃ est tel que défini ci-dessus,
pour obtenir l'imine de formule de formule (**D**) telle que défini ci-dessus ;
une étape préalable de préparation du catalyseur
comprenant une étape d'ajout d'oxyde de triméthylamine sur le complexe de formule (**A**) : 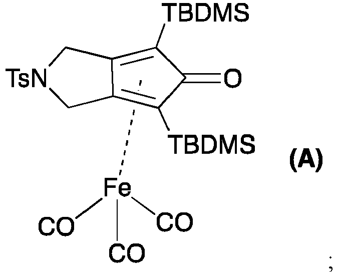
les deux susdites étapes préalables pouvant intervenir dans un ordre quelconque,
et une étape d'alkylation d'un alcool de formule (**C**)

   **(C)** R₄OH
dans laquelle R₄ représente un alkyle en C₂ à C₁₀, ou cycloalkyle en C₃ à C₁₀,
sur une imine de formule (**D**) préparée à l'étape précédente 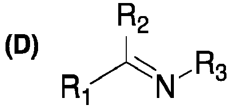
dans laquelle R₁, R₂ et R₃ sont tels que définis ci-dessus,
en présence du catalyseur de formule (**B**) préparé à l'étape précédente et tel que défini ci-dessus,
pour obtenir le composé de formule (**E**) 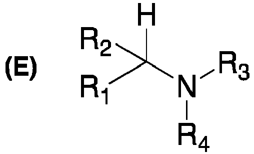
dans laquelle R₁, R₂, R₃ et R₄ sont tels que définis ci-dessus.

Selon un autre mode de réalisation, l'invention concerne un procédé de préparation d'amines tertiaires de formule (**E**), comprenant une étape de préparation d'amines tertiaires de formule (**E**) 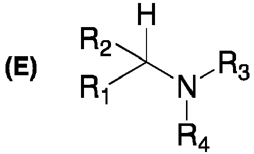
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀ ;
comprenant une étape d'alkylation d'un alcool de formule (C)

   **(C)** R₄OH
dans laquelle R₄ est tel que défini ci-dessus,
sur une imine de formule (**D**) 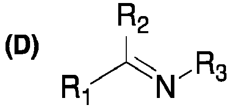
dans laquelle R₁, R₂ et R₃ sont tels que définis ci-dessus,
en présence d'un catalyseur de formule (**B**) 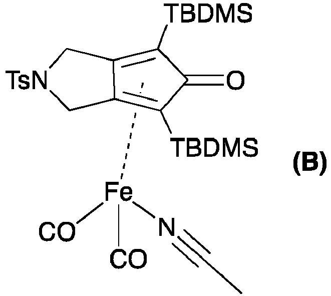
pour obtenir le composé de formule (**E**) tel que défini ci-dessus.

Selon un mode préféré, l'invention concerne un procédé de préparation d'amines tertiaires de formule (**E**), comprenant une étape d'alkylation d'un alcool primaire de formule (**C**) sur une imine de formule (**D**) en présence d'un catalyseur de formule (**B**) formé préalablement à la préparation des amines tertiaires.

Selon un mode préféré, l'invention concerne un procédé de préparation d'amines tertiaires de formule (**E**), comprenant une étape d'alkylation d'un alcool primaire de formule (**C**) sur une imine de formule (**D**) en présence d'un catalyseur de formule (**B**), l'imine de formule (**D**) et le catalyseur de formule (**B**) étant formés préalablement à la préparation des amines tertiaires.

Selon un autre mode de réalisation, l'invention concerne un procédé de préparation d'amines tertiaires de formule (**E**), comprenant une première étape de préparation du catalyseur,
comprenant une étape d'ajout d'oxyde de triméthylamine sur le complexe de formule (**A**) 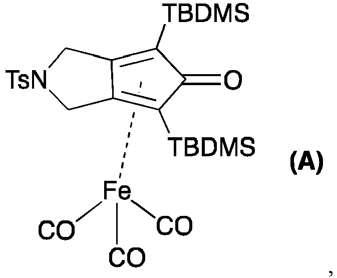
et une seconde étape d'alkylation d'un alcool de formule (**C**)

   **(C)** R₄OH
dans laquelle R₄ représente un alkyle en C₂ à C₁₀, ou un cycloalkyle en C₃ à C₁₀,
sur une imine de formule (**D**) 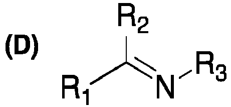
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀, en présence d'un catalyseur de formule (**B**) préparé à l'étape précédente et tel que défini ci-dessus,
pour obtenir le composé de formule (**E**) 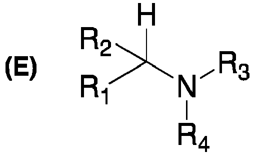
dans laquelle R₁, R₂, R₃ et R₄ sont tels que définis ci-dessus.

Selon un autre mode de réalisation, l'invention concerne un procédé de préparation d'amines tertiaires de formule (**E**), comprenant une première étape de préparation d'imines de formule (**D**) 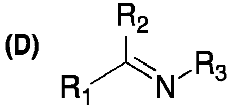
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
comprenant une étape de mise en contact d'un aldéhyde ou d'une cétone de formule (**F**) 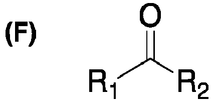
dans laquelle R₁ et R₂ sont tels que définis ci-dessus,
et d'une amine de formule (**G**)

   **(G)** R₃-NH₂
dans laquelle R₃ est tel que défini ci-dessus,
pour obtenir l'imine de formule (**D**) telle que défini ci-dessus ;
et une seconde étape d'alkylation d'un alcool de formule (**C**)

   **(C)** R₄OH
dans laquelle R₄ représente un alkyle en C₂ à C₁₀, ou un cycloalkyle en C₃ à C₁₀
sur une imine de formule (**D**) préparé à l'étape précédente et telle que définie ci-dessus,
en présence d'un catalyseur de formule (**B**) 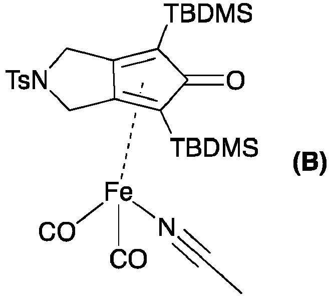
pour obtenir le composé de formule (**E**) 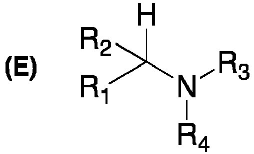
dans laquelle R₁, R₂, R₃ et R₄ sont tels que définis ci-dessus.

Selon un autre mode de réalisation, l'invention concerne un procédé de préparation d'amines tertiaires de formule (**E**), comprenant une étape préalable de préparation d'imines de formule (**D**) 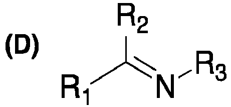
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
comprenant une étape de mise en contact d'un aldéhyde ou une cétone de formule (**F**) 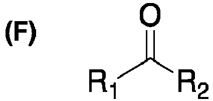
dans laquelle R₁ et R₂ sont tels que définis ci-dessus,
et d'une amine de formule (**G**)

   **(G)** R₃-NH₂
dans laquelle R₃ est tel que défini ci-dessus,
pour obtenir l'imine de formule de formule (**D**) telle que défini ci-dessus ;
une étape préalable de préparation du catalyseur,
comprenant une étape d'ajout d'oxyde de triméthylamine sur le complexe de formule (**A**) : 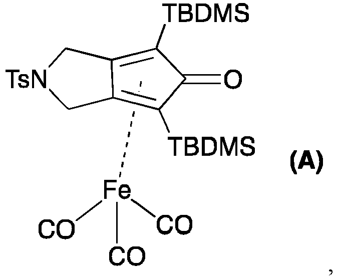
les deux susdites étapes préalables pouvant intervenir dans un ordre quelconque,
et une étape d'alkylation d'un alcool primaire ou secondaire de formule (**C**)

   **(C)** R₄OH
dans laquelle R₄ représente un alkyle en C₂ à C₁₀, ou cycloalkyle en C₃ à C₁₀
sur une imine de formule (**D**) préparée à l'étape précédente 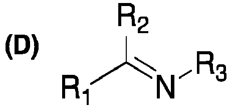
dans laquelle R₁, R₂ et R₃ sont tels que définis ci-dessus,
en présence du catalyseur de formule (**B**) préparé à l'étape précédente et tel que défini ci-dessus,
pour obtenir le composé de formule (**E**) 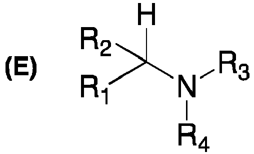
dans laquelle R₁, R₂, R₃ et R₄ sont tels que définis ci-dessus.

Selon un mode préféré de l'invention, l'imine de formule (**D**) est choisie parmi :

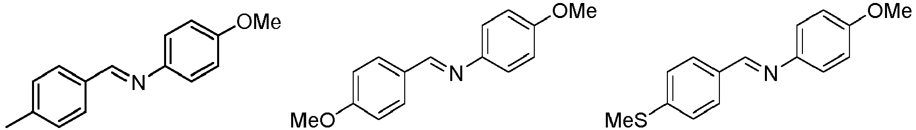

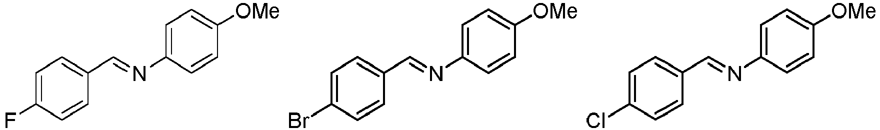

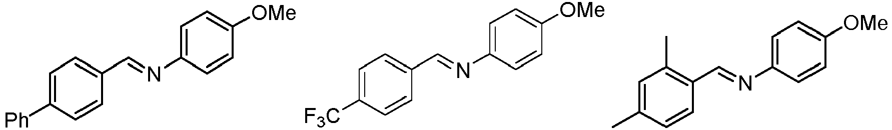

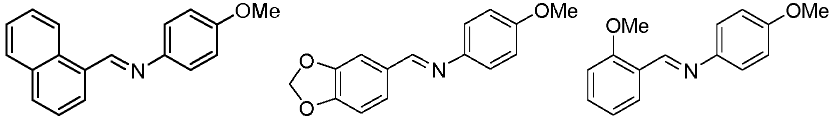

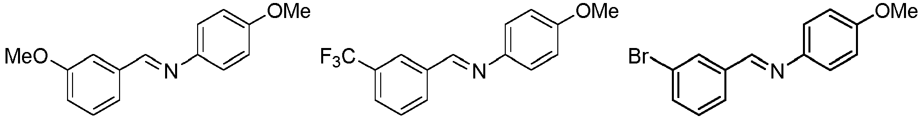

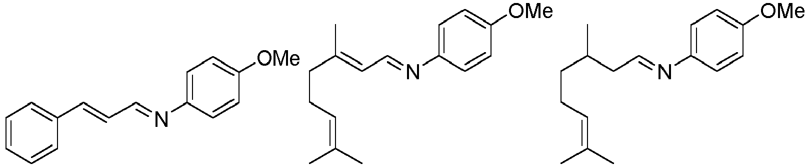

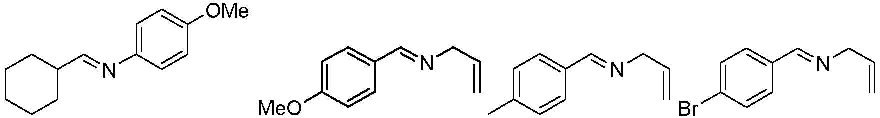

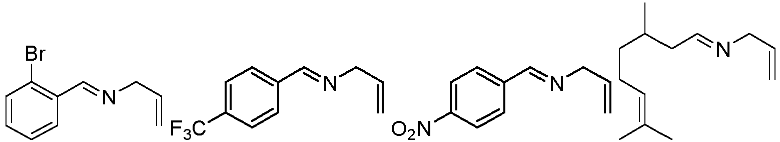

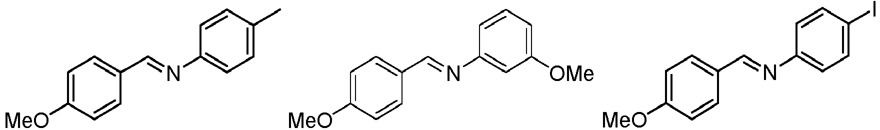

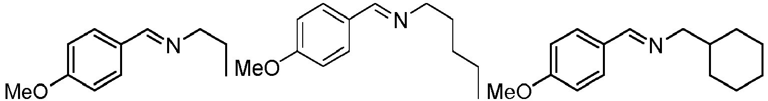

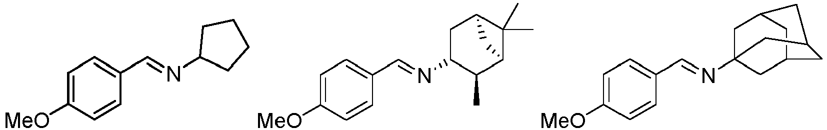

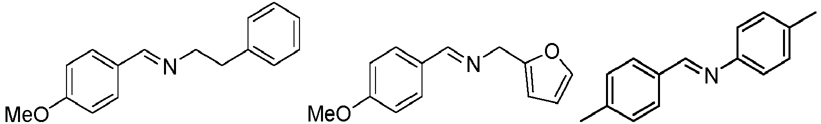

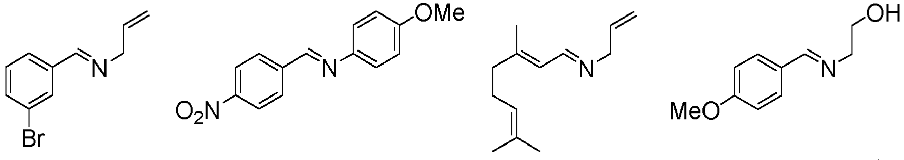

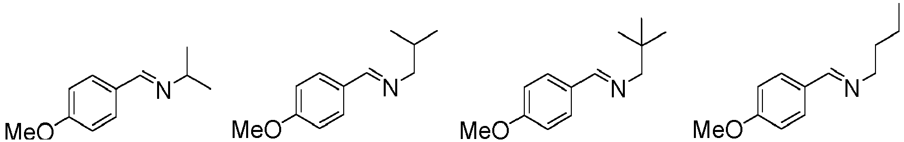

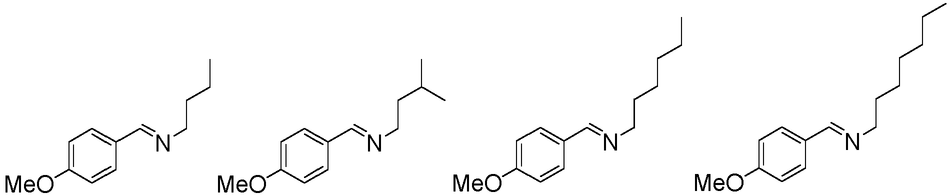

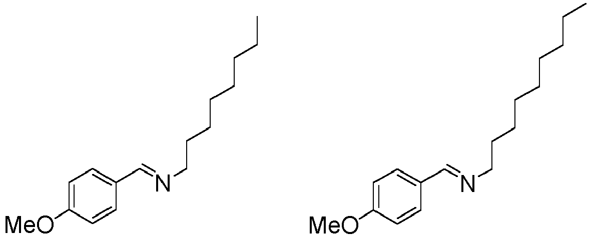

La présente invention concerne également le procédé sus-mentionné, dans lequel ledit amine tertiaire de formule (E) répond à l'une des formules suivantes:

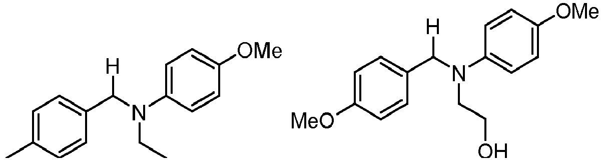

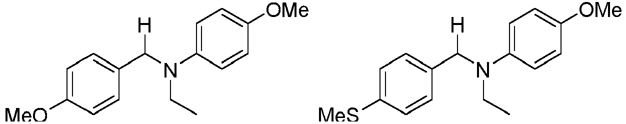

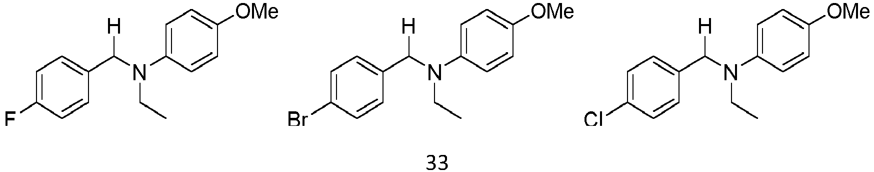

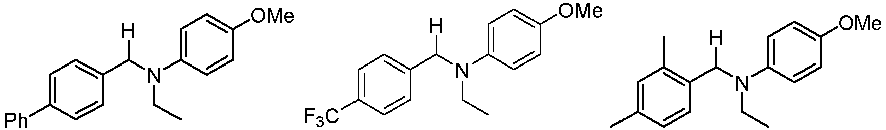

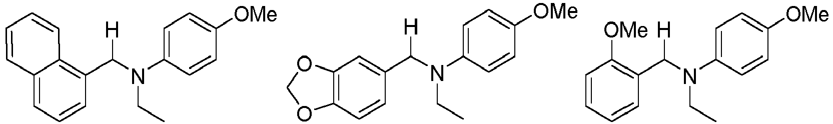

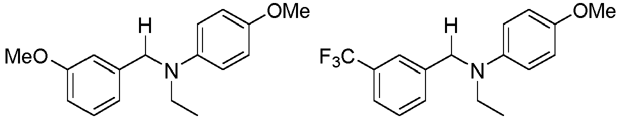

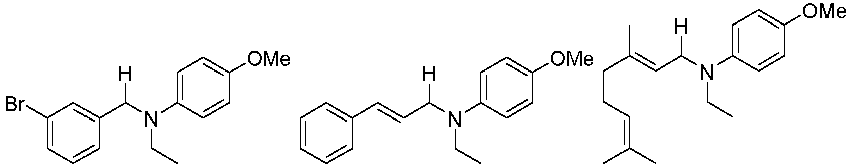

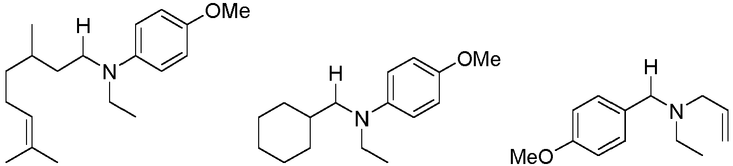

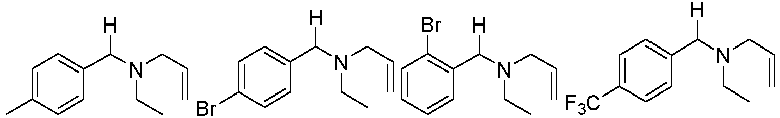

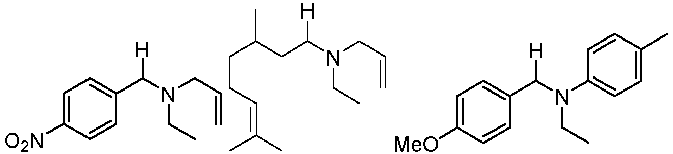

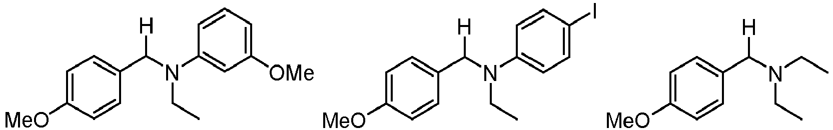

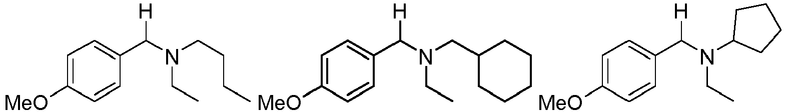

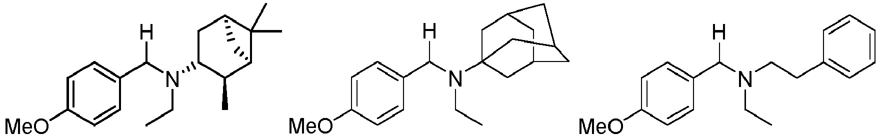

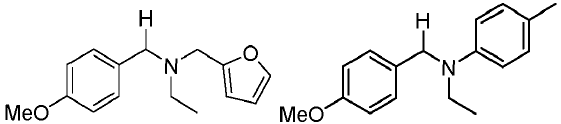

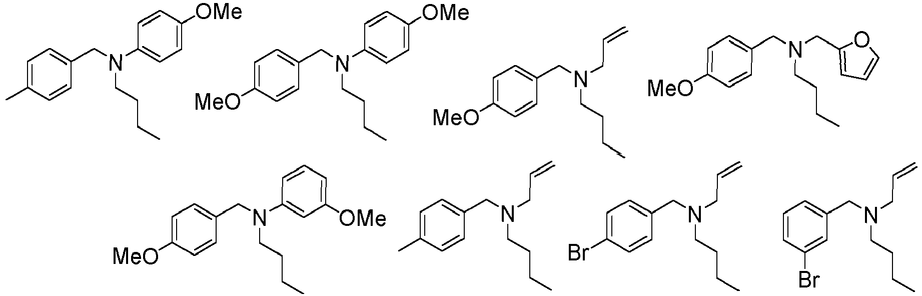

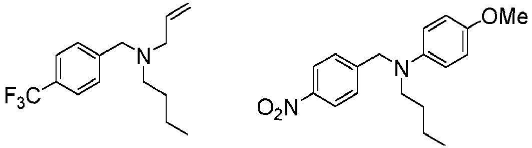

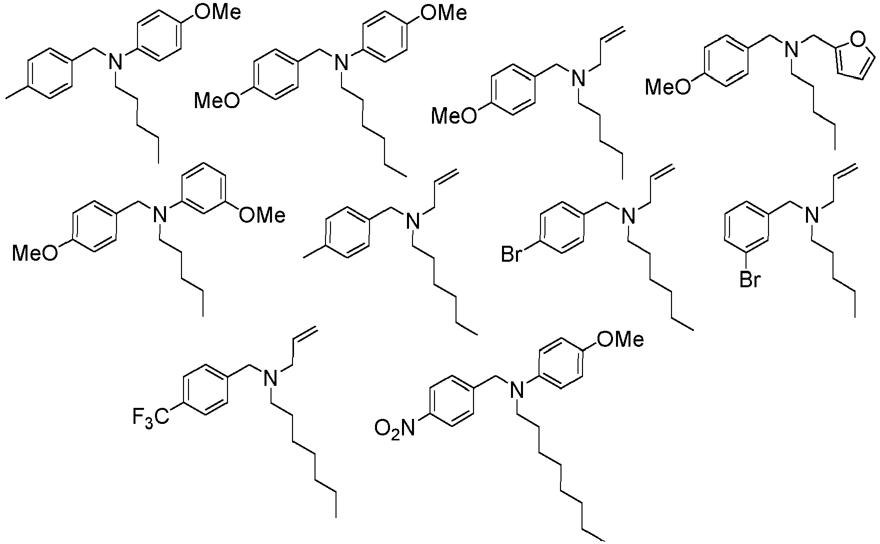

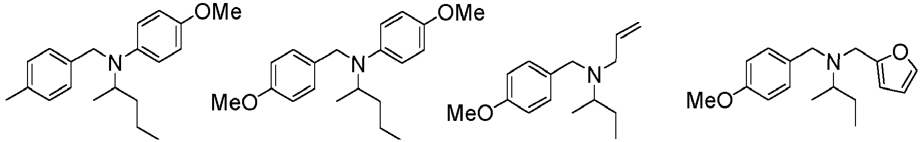

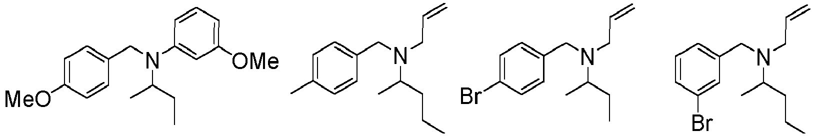

Des

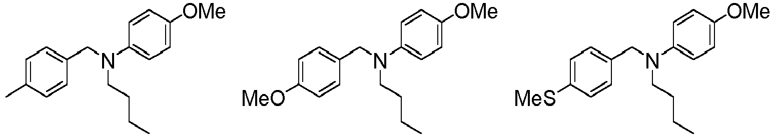

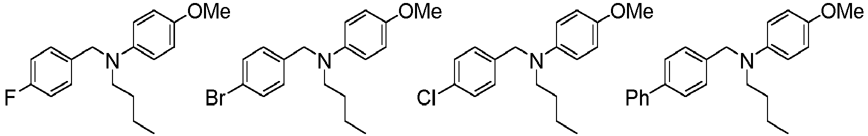

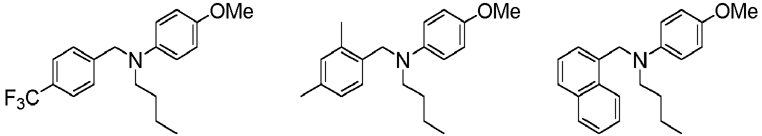

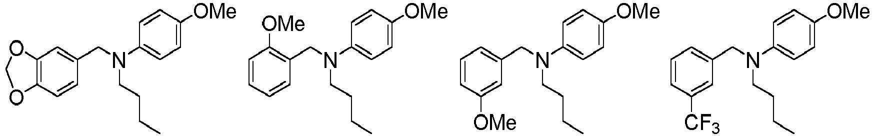

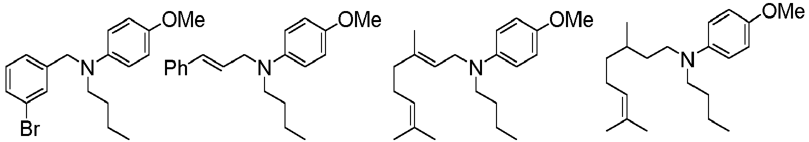

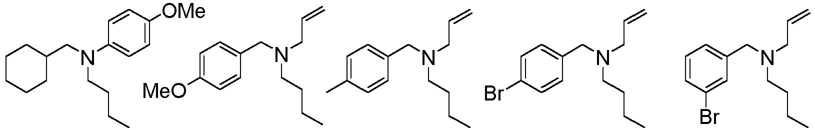

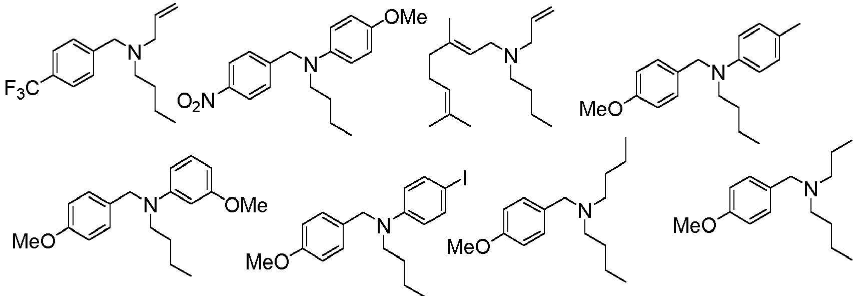

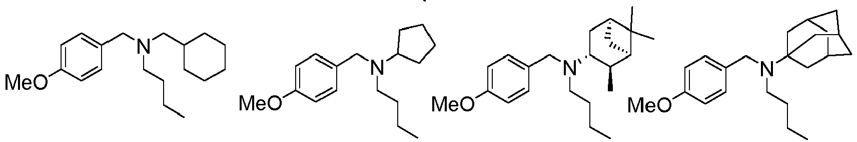

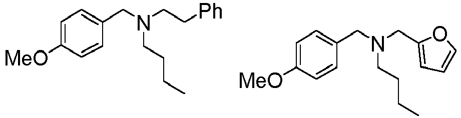

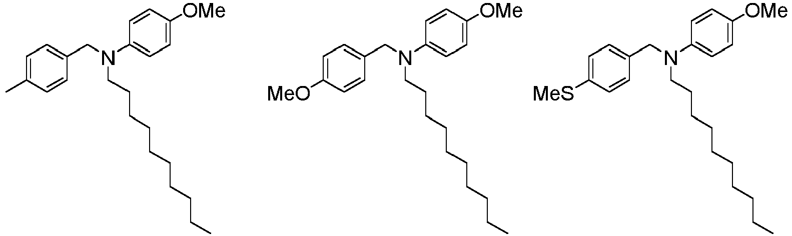

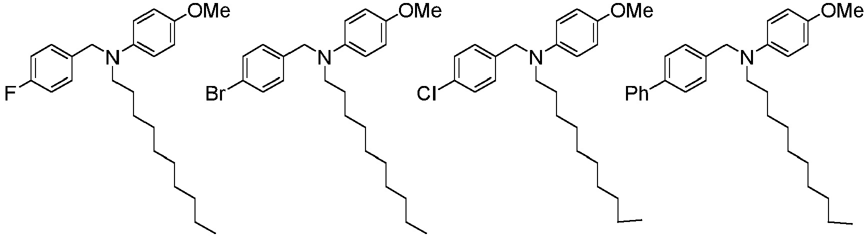

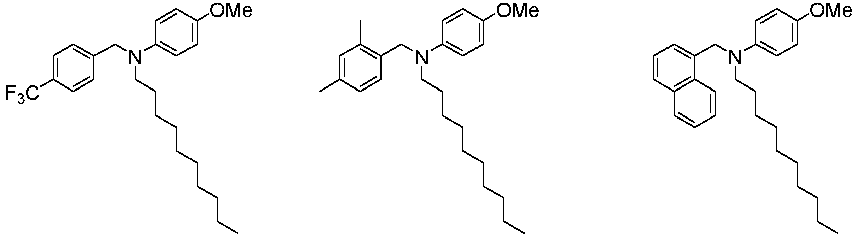

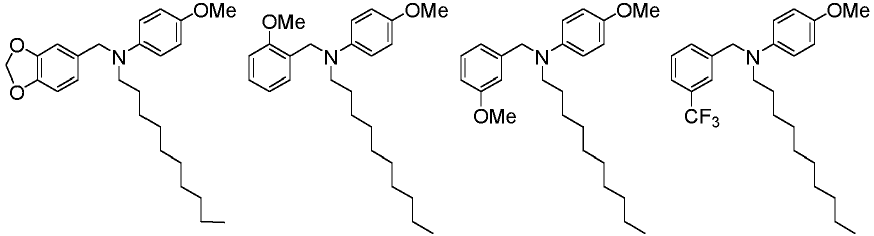

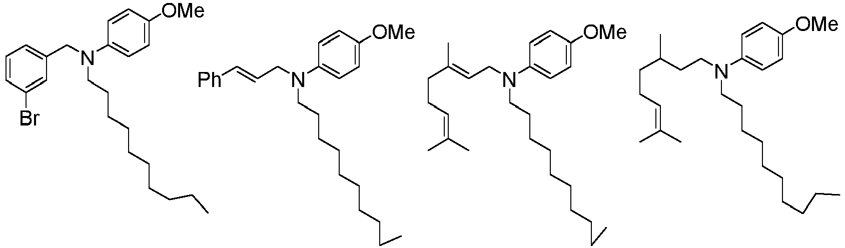

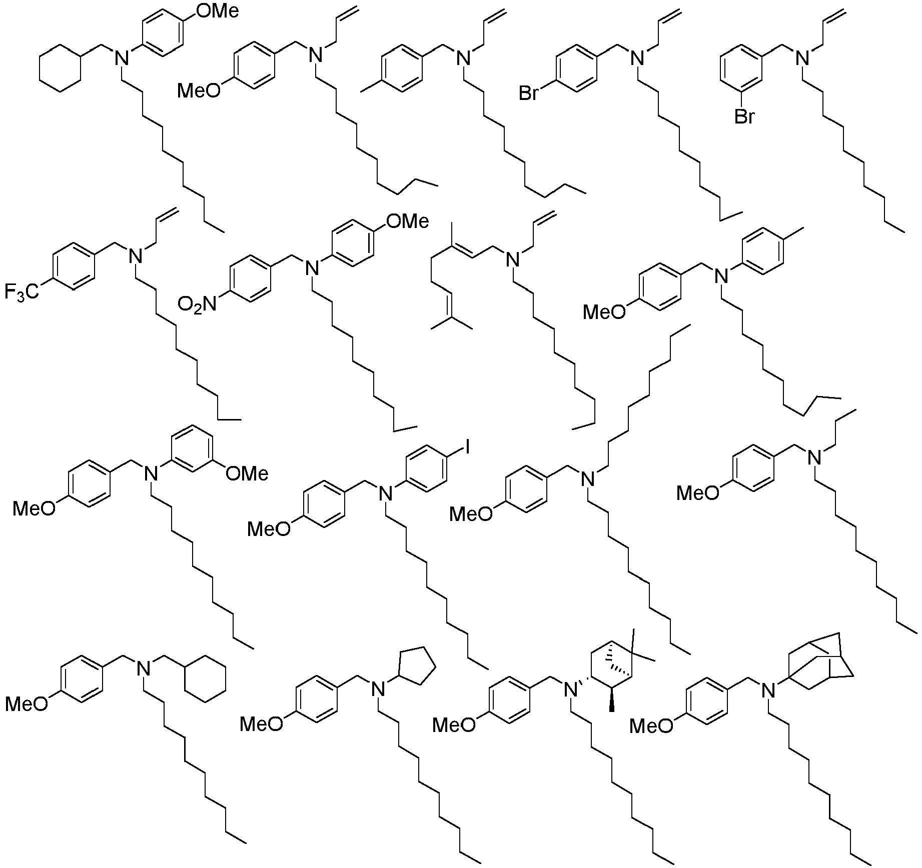

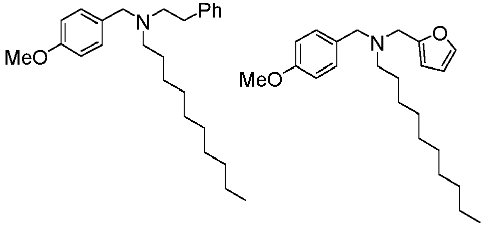

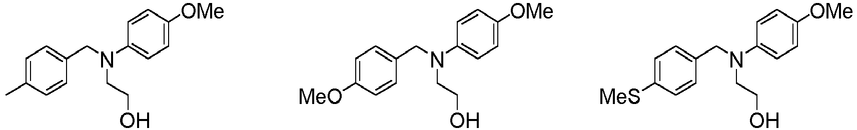

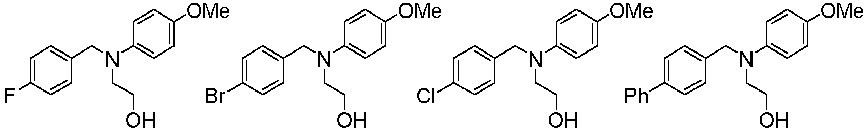

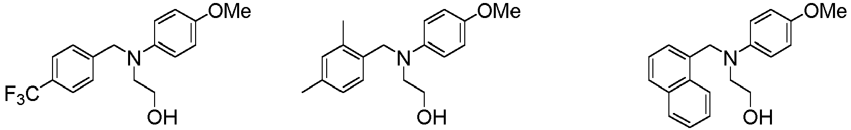

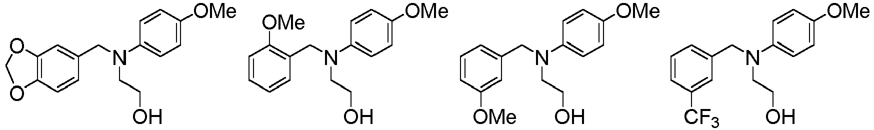

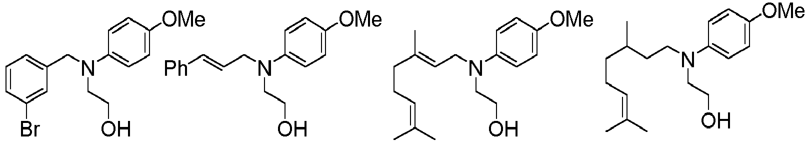

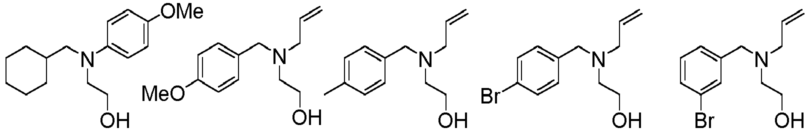

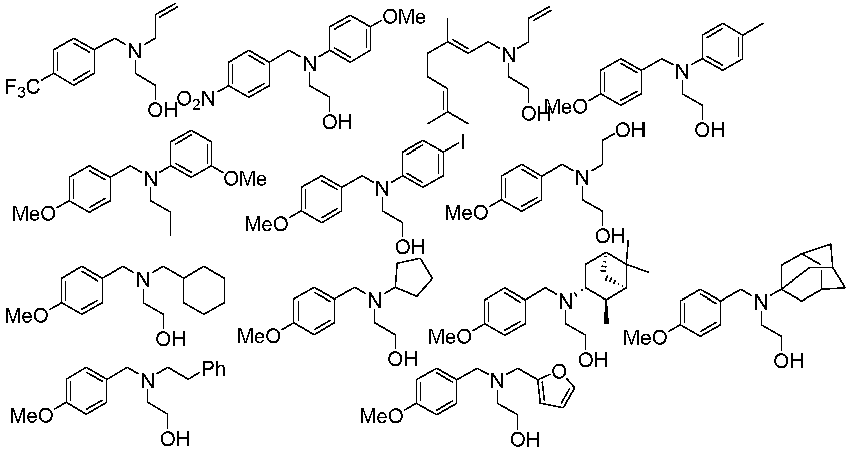

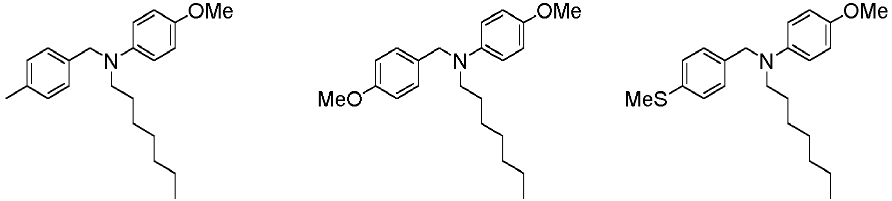

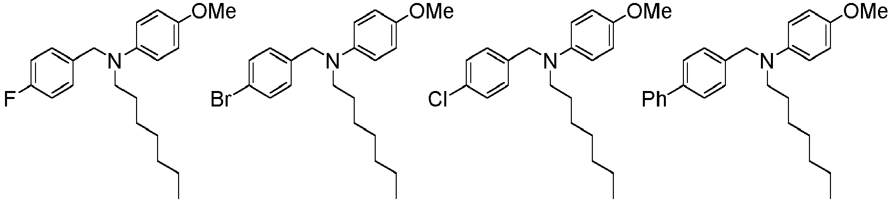

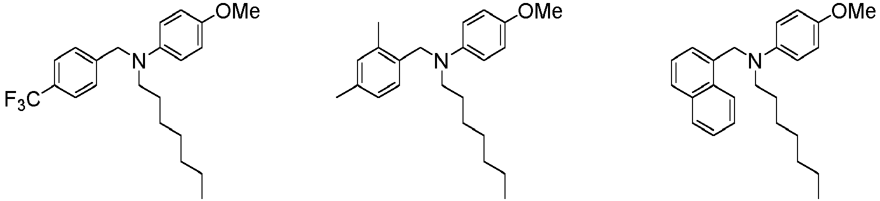

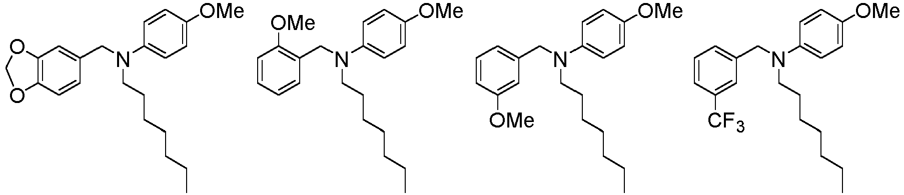

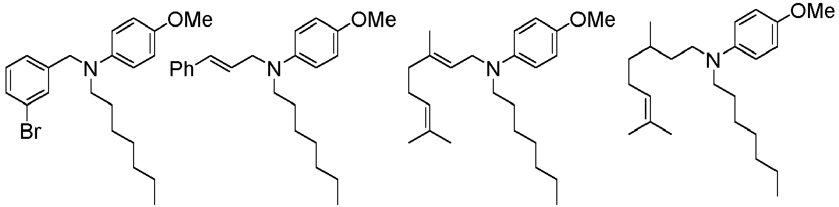

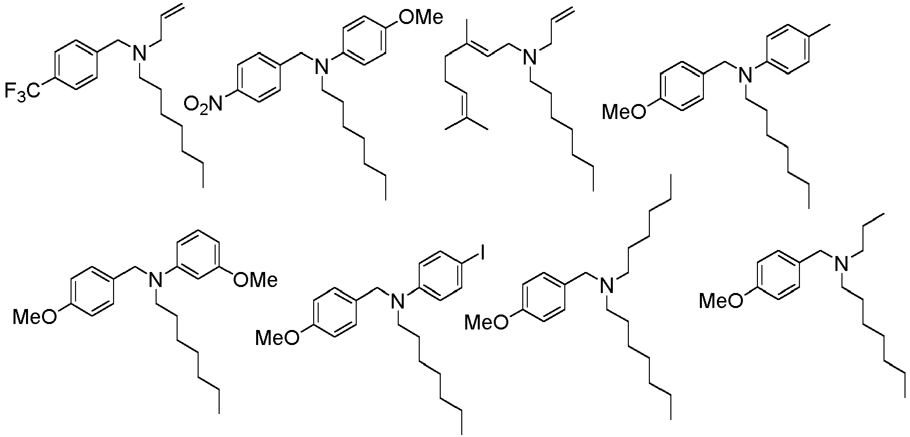

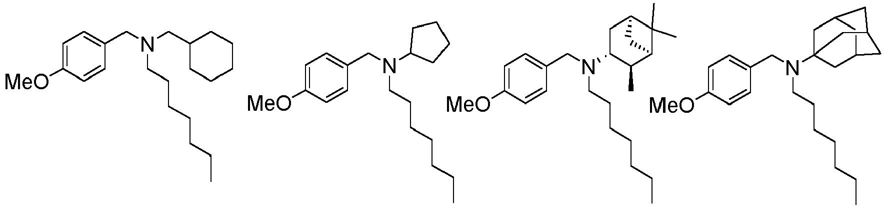

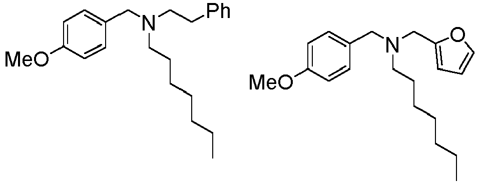

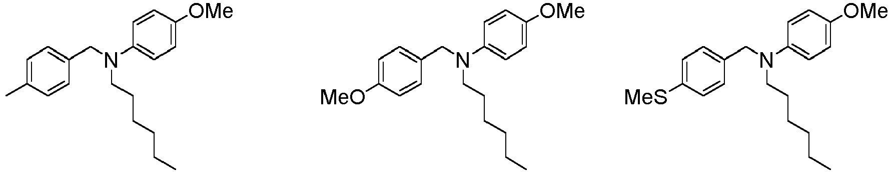

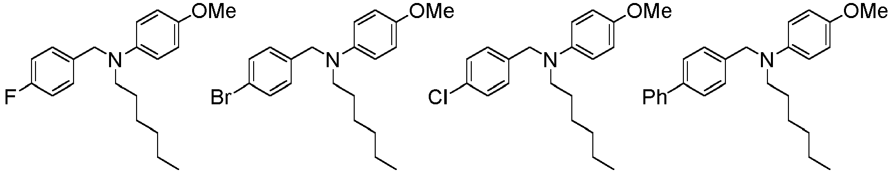

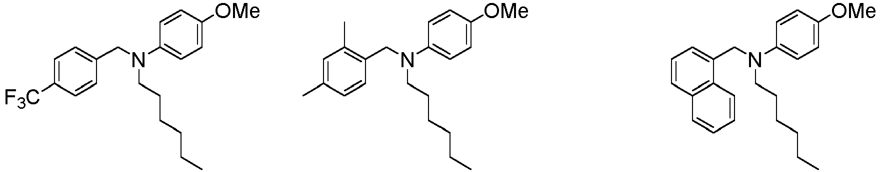

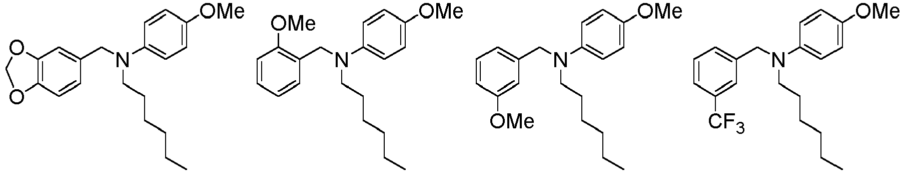

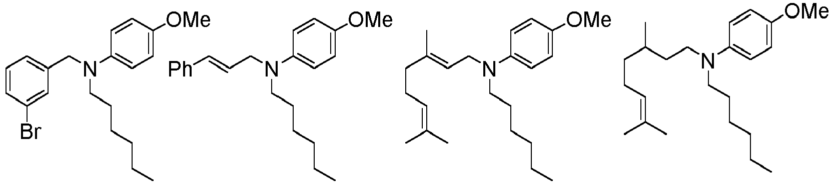

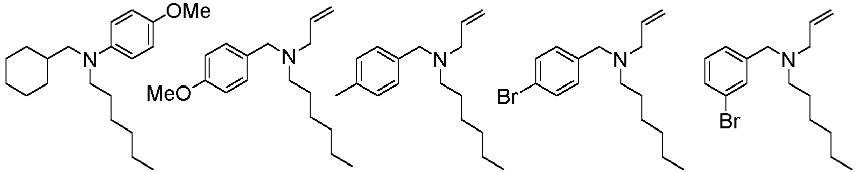

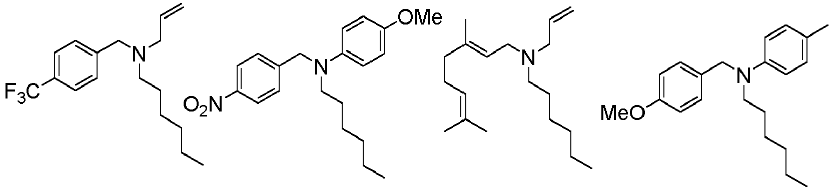

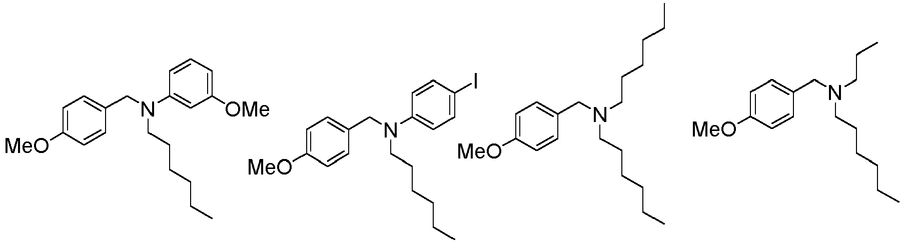

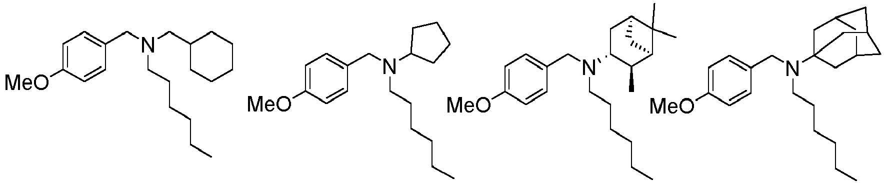

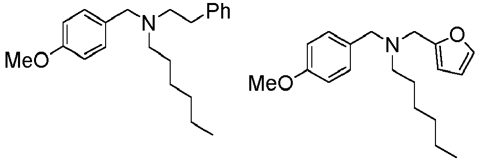

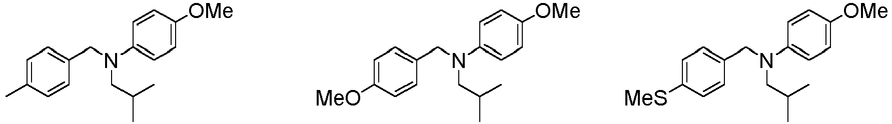

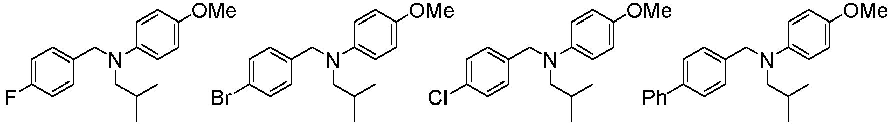

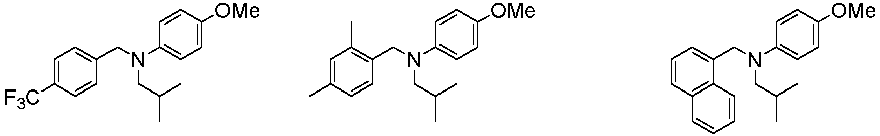

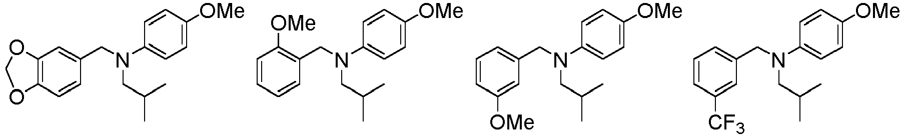

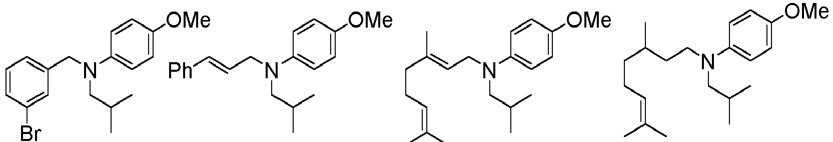

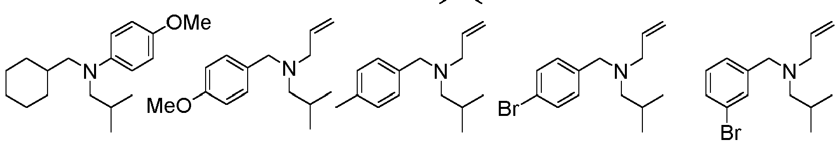

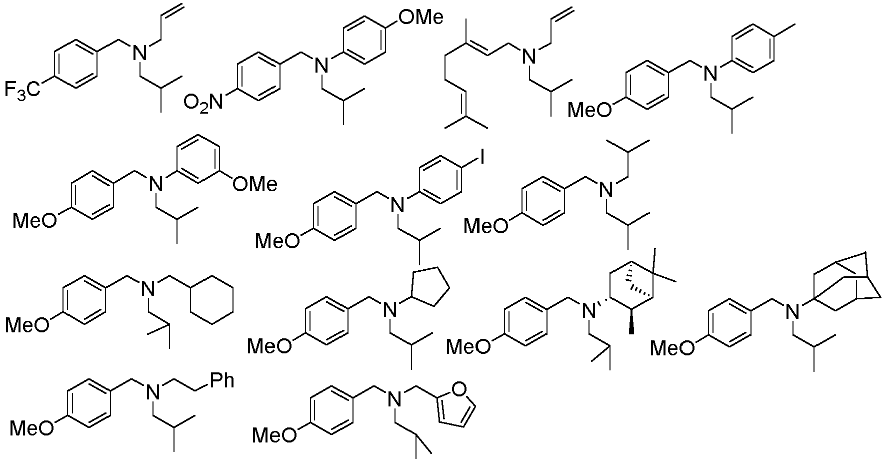

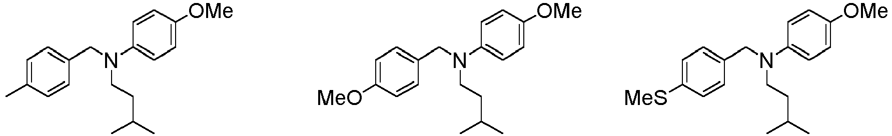

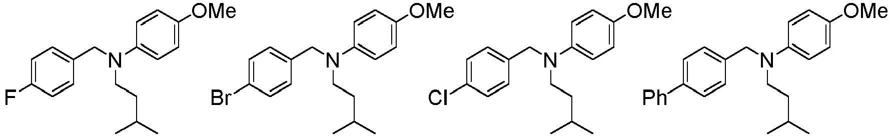

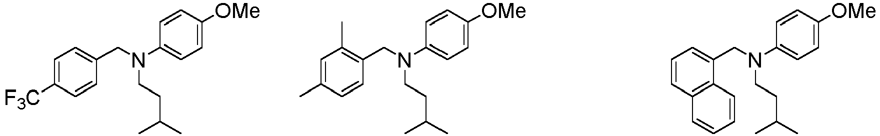

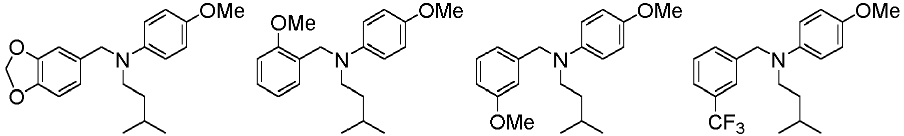

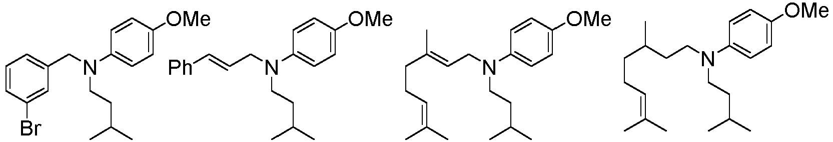

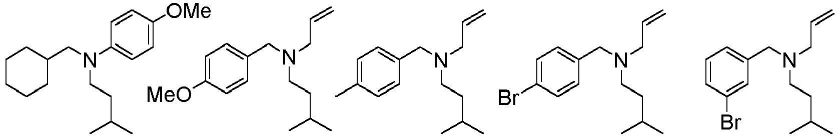

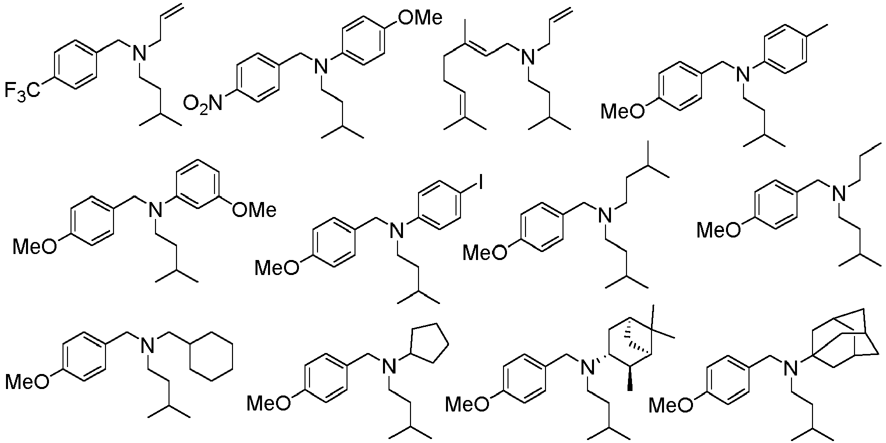

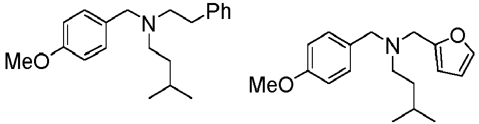

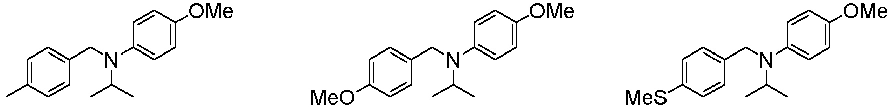

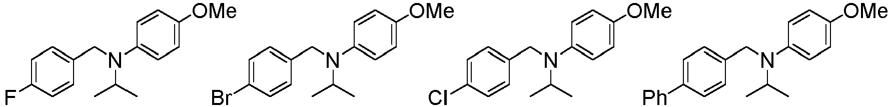

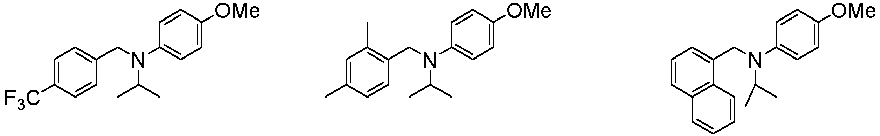

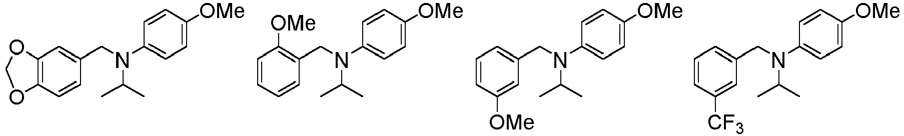

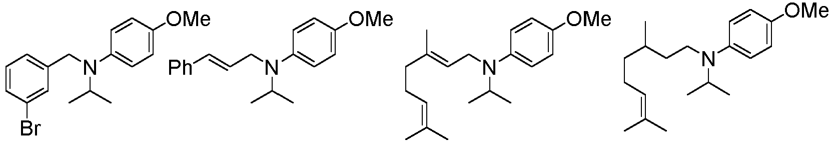

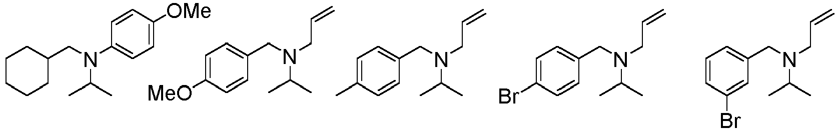

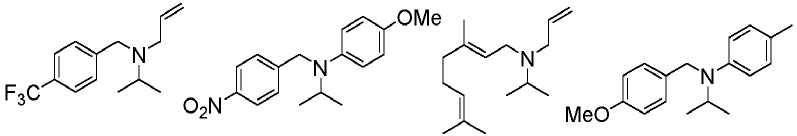

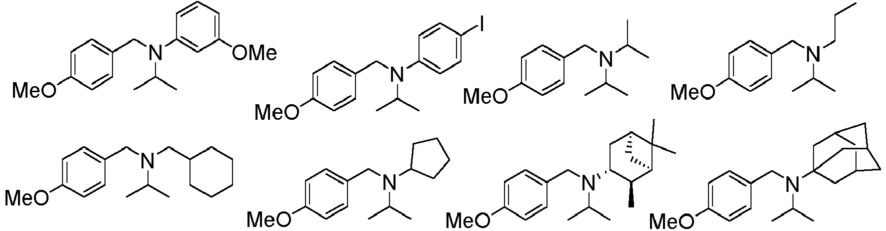

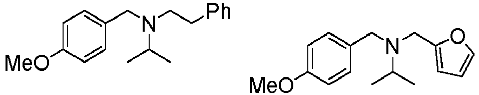

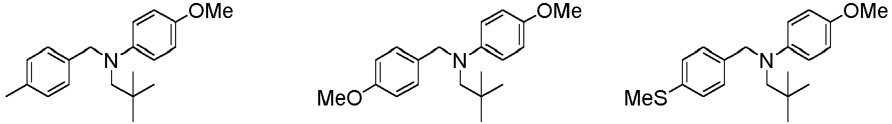

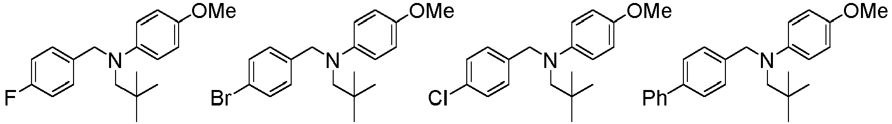

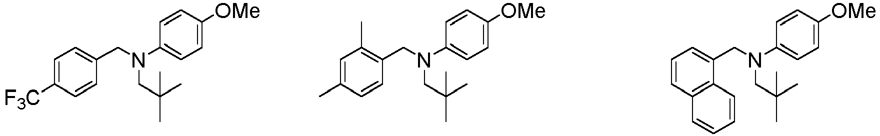

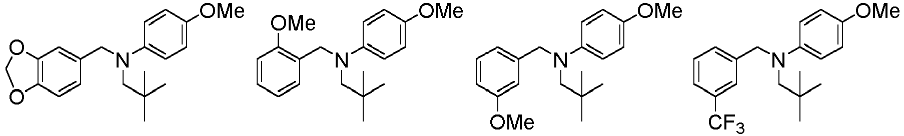

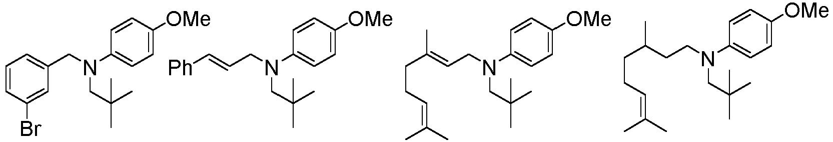

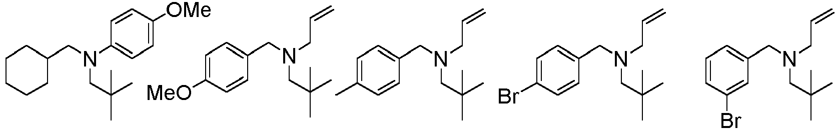

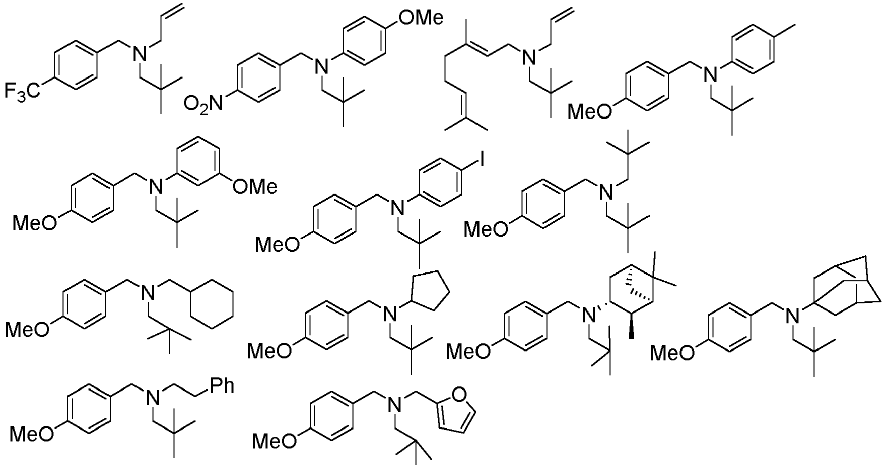

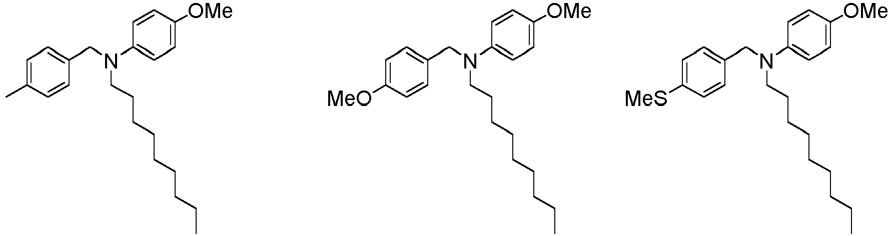

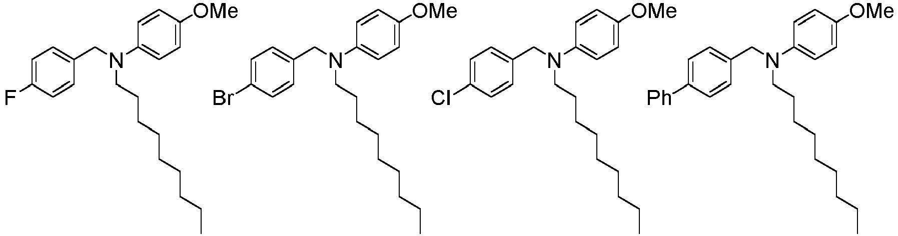

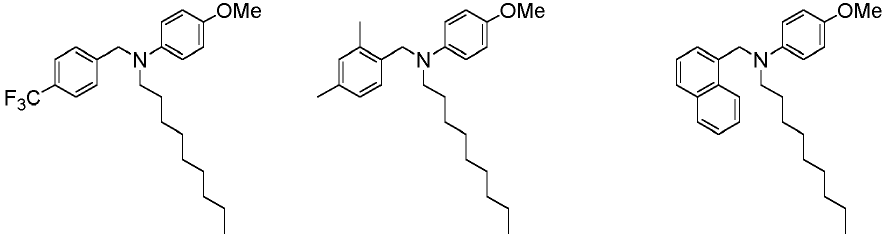

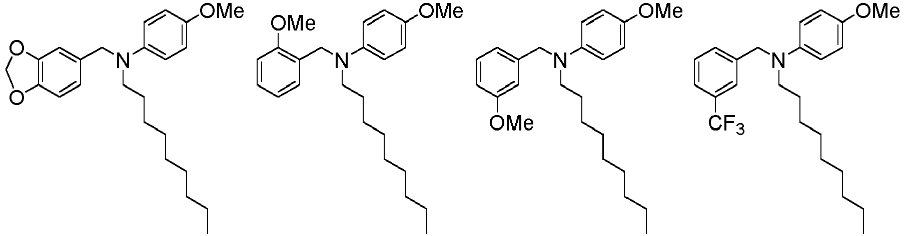

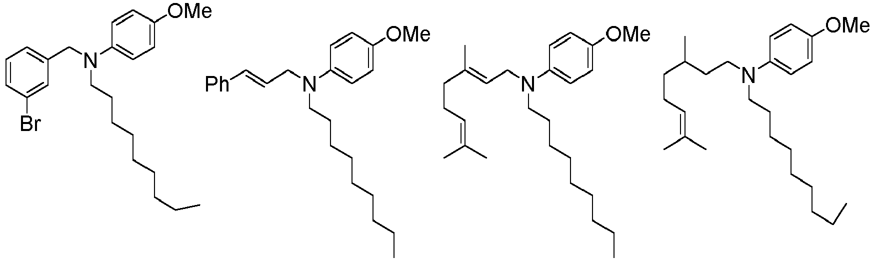

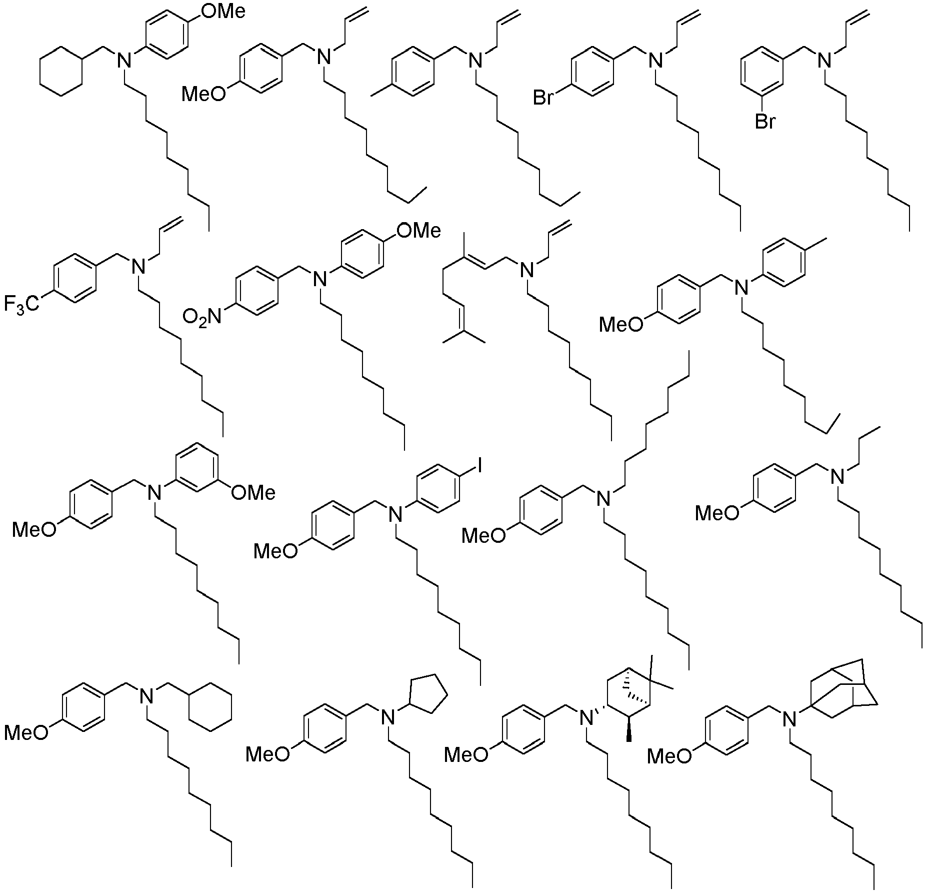

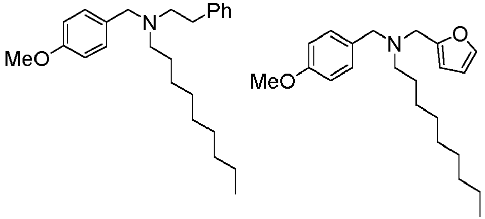

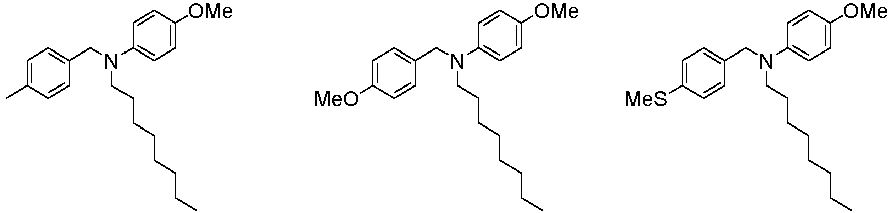

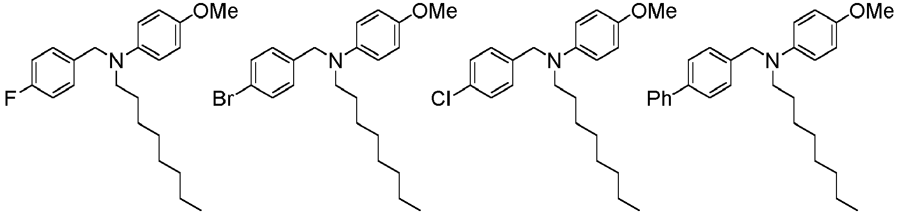

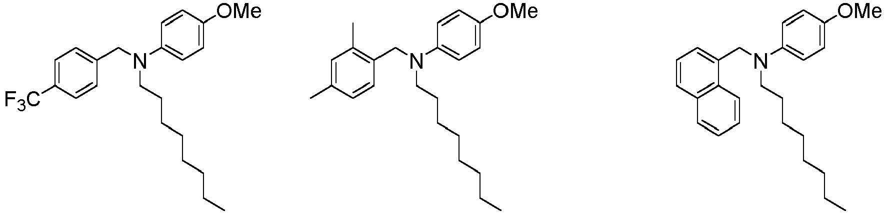

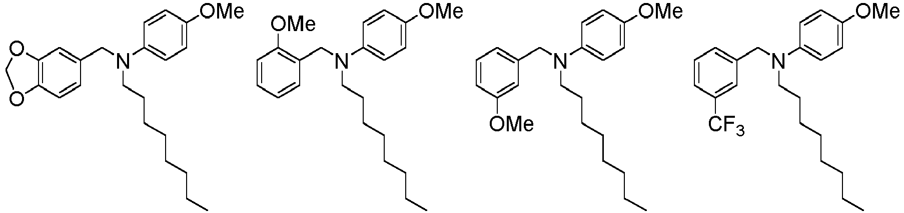

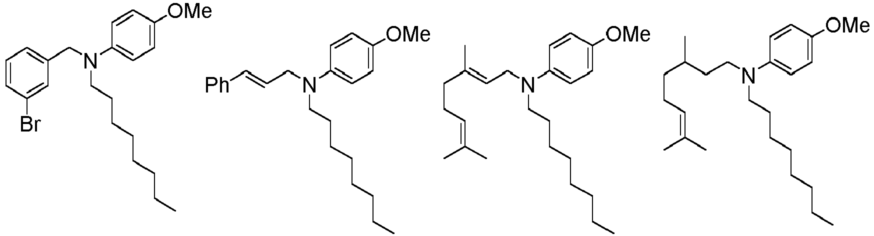

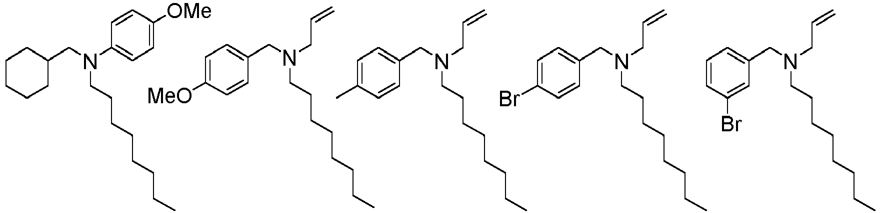

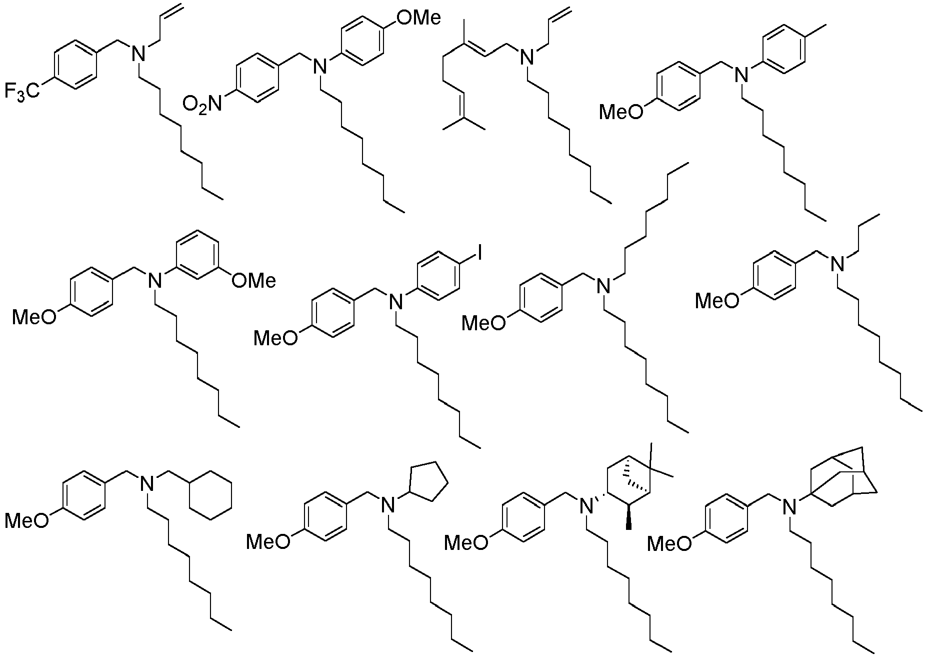

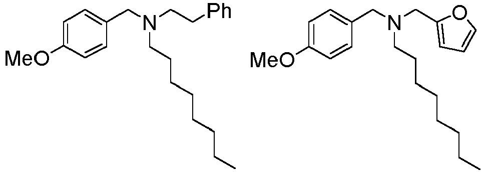

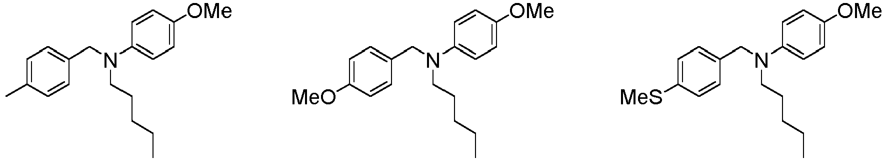

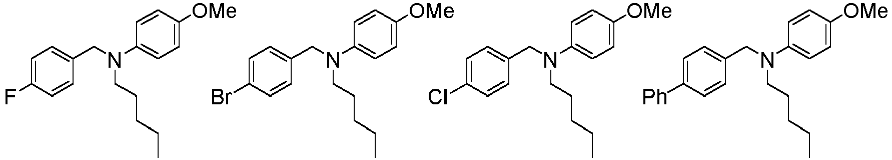

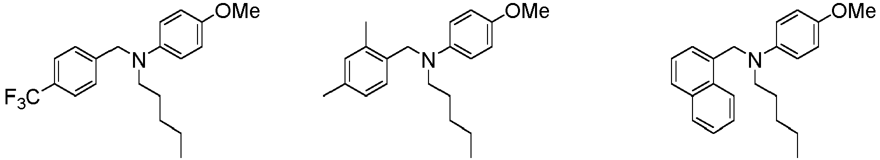

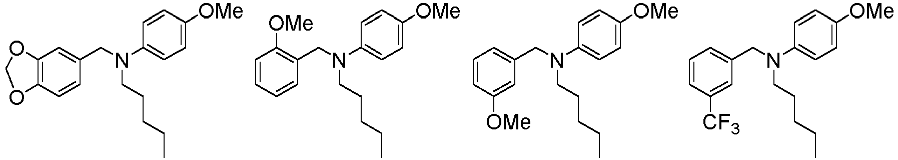

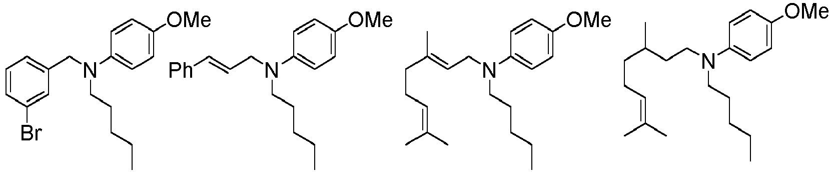

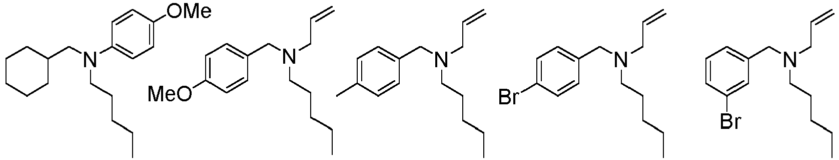

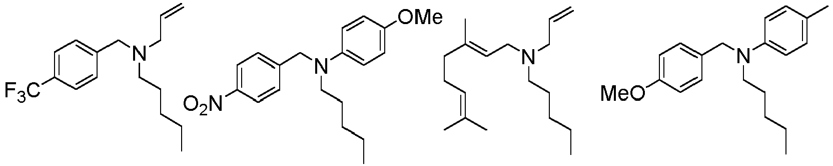

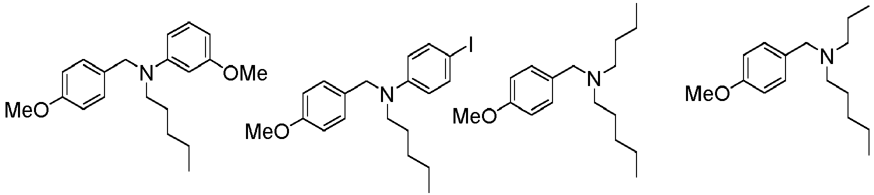

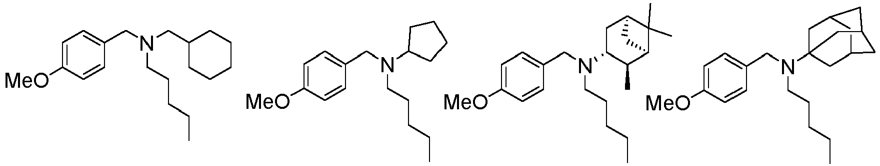

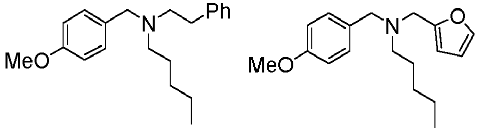

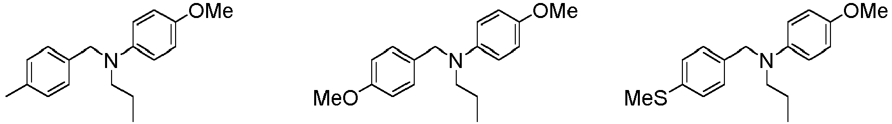

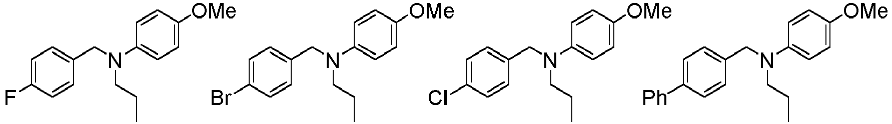

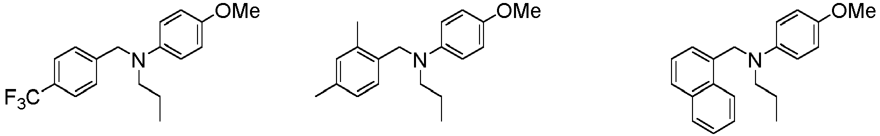

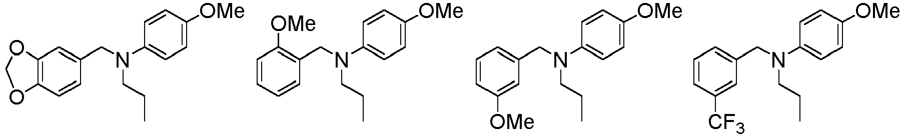

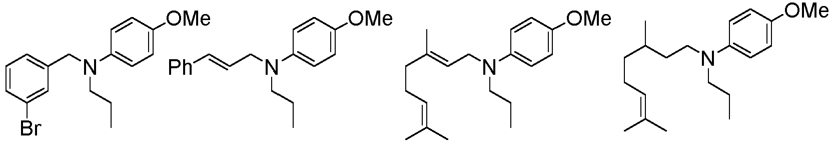

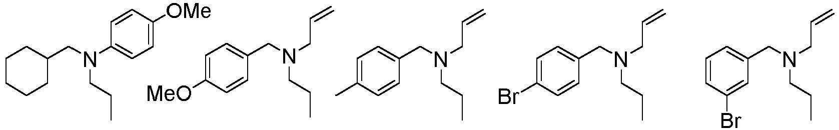

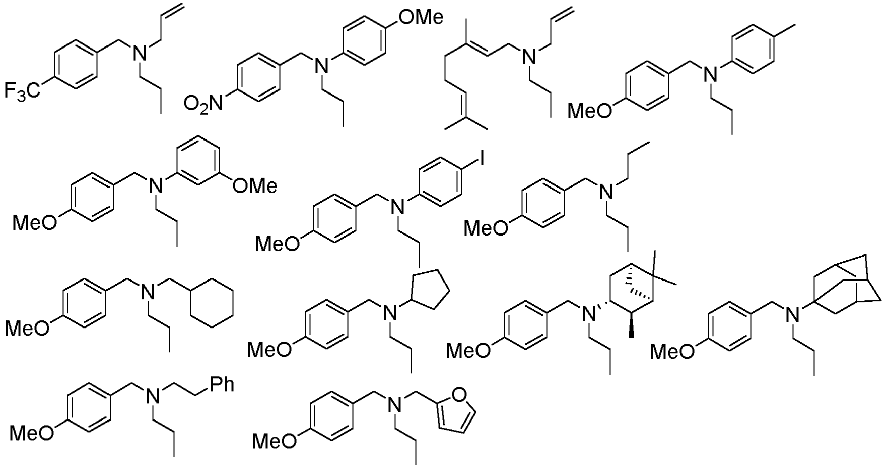

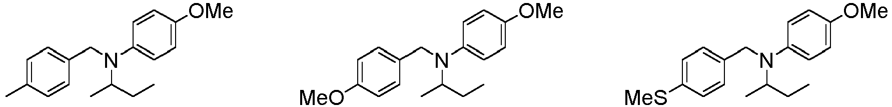

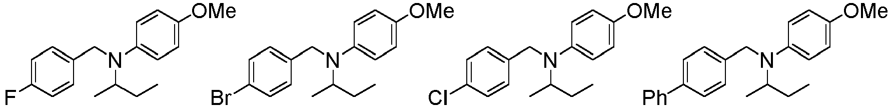

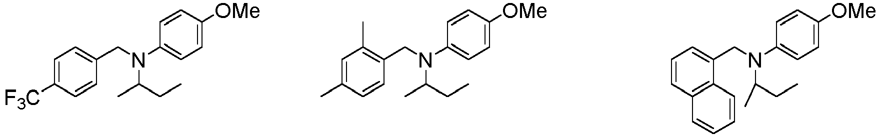

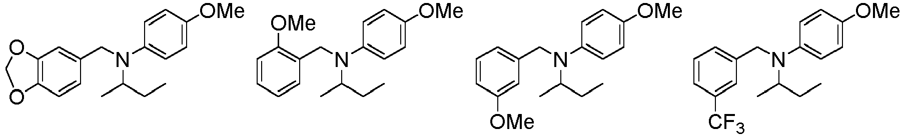

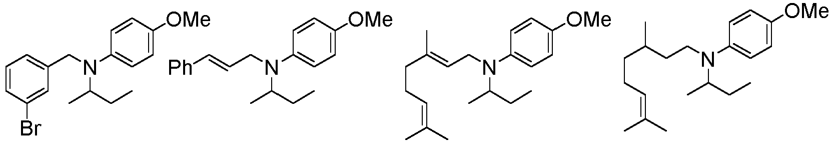

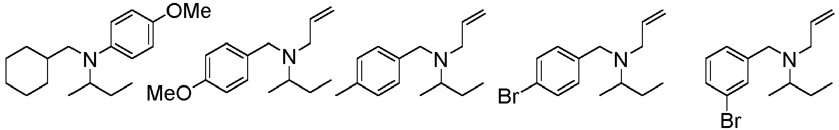

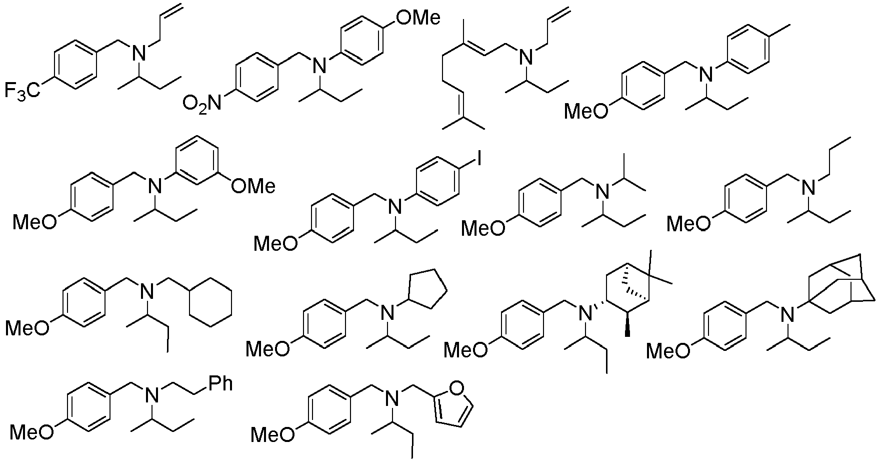

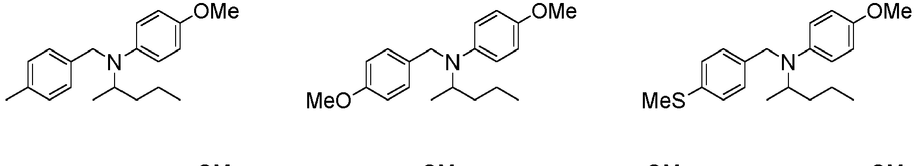

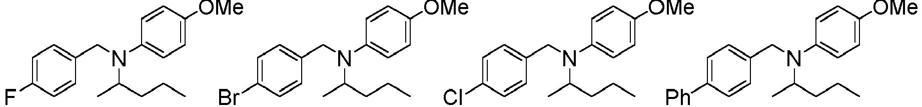

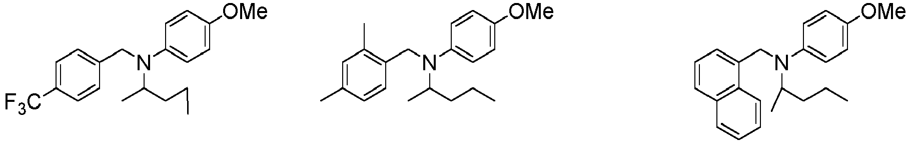

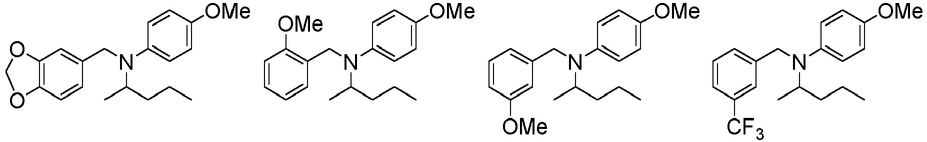

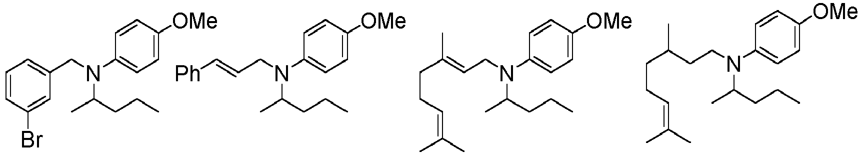

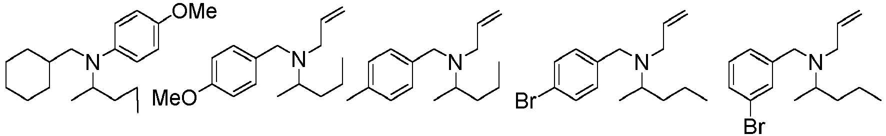

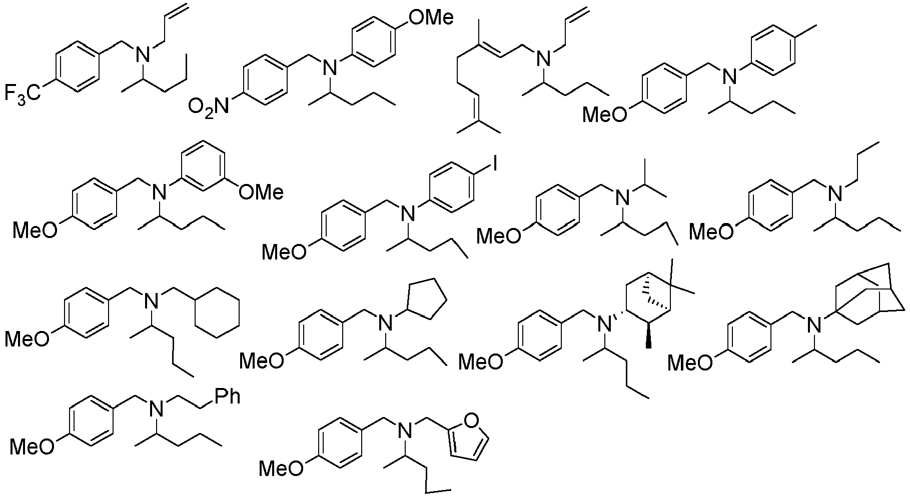

Les amines à longue chaines carbonées, notamment tertiaires, présentent un grand intérêt industriel. Elles sont utilisées dans la synthèse de composés chimiques, en tant que surfactants (agents anticorrosion, détergents, agents de flottaison, adoucissants, assouplissants, antistatiques, germicides, insecticides, dispersant, antiagglomérants, émulsifiants, lubrifiants, agents de traitement de l'eau, additifs alimentaires, cosmétiques, etc.).

Les amines à longue chaines carbonées, notamment allyliques, sont des éléments fondamentaux de la chimie organique et leur synthèse est un objectif industriel et synthétique important. Le fragment allylamine peut être rencontré dans des produits naturels, mais souvent, l'allylamine est transformée en une gamme de produits par fonctionnalisation, réduction ou oxydation de l'insaturation. Ainsi, les amines peuvent être utilisées comme matières premières pour la synthèse de nombreux composés tels que les acides aminées, les alcaloïdes, et les dérivés de glucidiques.

Les amines tertiaires constituent des intermédiaires importants pour la préparation de sels d'ammonium quaternaire dissymétrique de formule 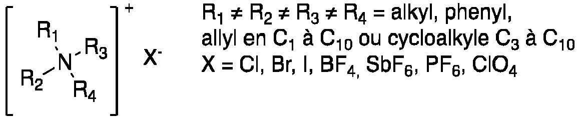 afin d'accéder à des agents actifs que ce soit pour des usages pharmaceutiques ou cosmétiques. Ils sont utilisés en tant que tensioactifs, biocides pour le traitement de l'eau, agent de flottation, détergent à essence, inhibiteurs de la corrosion, additifs de traitement du caoutchouc ou émulsifiants pour herbicides.

### Exemples :

### Exemple 1 : Étude du catalyseur

**Tableau 1. Tests avec différents catalyseurs de Fe**

| 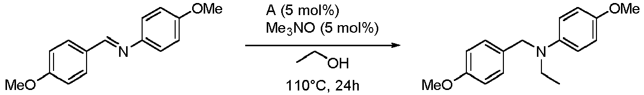 | | | |
|---|---|---|---|
| **Catalyseur.** | **Additif** | **Ratio GC (départ/réduction/alkylation)** | **Rendement [%]** |
| A | Me₃NO | 0/0/**100** | 97 |
| B | - | 0/0/**100** | 99 |

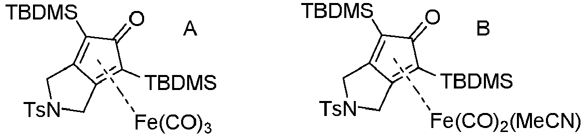

### Exemple 2 : Étude du solvant

**Tableau 2. Tests avec différents solvants**

| 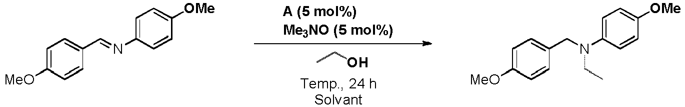 | | |
|---|---|---|
| **Solvant** | **Température [°C]** | **Ratio GC (départ/réduction/alkylation/dialkylation)** |
| DCE/EtOH (svt/3 éq) | 80 | 18/72/**10**/0 |
| THF/EtOH (svt/3 éq) | 80 | 27/65/**8**/0 |
| THF/EtOH (1/1) | 80 | 15/1/**79**/5 |
| THF/EtOH (1/1) | 110 | 23/5/**72**/0 |
| CPME/EtOH (1/2) | 110 | 73/18/**9**/0 |

### Exemple 3 : Étude de l'alcool

**Tableau 3. Etude de divers alcools comme agents alkylants**

| 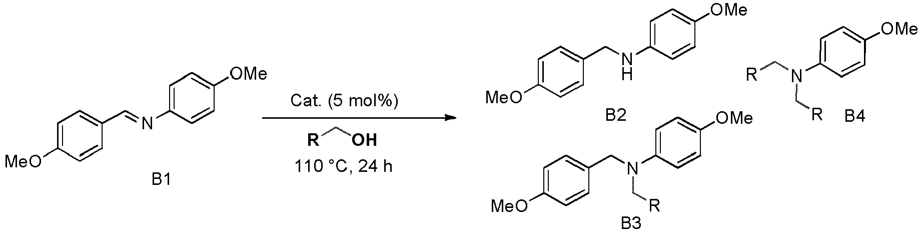 | | | |
|---|---|---|---|
| **Catalyseur** | **Alcool** | **Ratio GC (départ/réduction/alkylation/ dialkylation)** | **Rendement [%]** |
| B | éthylène glycol | 0/0/**100**/0 | 18 |
| B | éthanol | 0/0/100/0 | 99 |

### Exemple 4 : Imines aromatiques

**Tableau 4. Exemplification avec divers aldéhydes aromatiques substitués en position para**

| 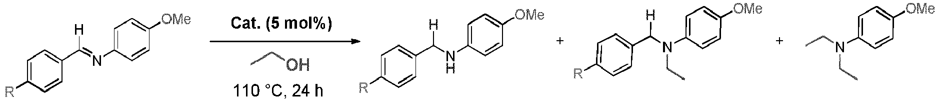 | | | |
|---|---|---|---|
| **Produit** | **Catalyseur** | **Ratio GC (départ/réduction/alkylation/dialkylation)** | **Rendement alkylation [%]** |
| 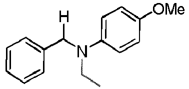 | A/Me₃NO B | 16/23/**48**/13 | 28 |
| | | 5/20/7**4**/1 | 68 |
| 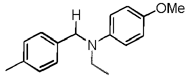 | A/Me₃NO B | 0/0/**100**/0 | 88 |
| | | 0/0/**88**/12 | 55 |
| 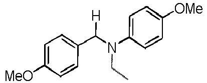 | A/Me₃NO B | 0/0/**100**/0 | 97 |
| | | 0/0/**100**/0 | 99 |
| 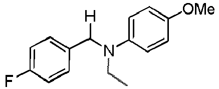 | A/Me₃NO B | 35/25/**40**/0 | 33 |
| | | 45/37/**18**/0 | 15 |
| | B à 130°C | 6/39/**55**/0 | 50 |
| 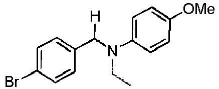 | A/Me₃NO B | 3/50/**35**/12 | 26 |
| | | 8/13/**75**/4 | 68 |
| 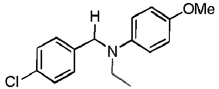 | A/Me₃NO B | 48/33/**19**/0 | 12 |
| | | 0/0/**86**/14 | 74 |
| 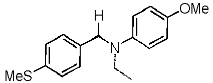 | A/Me₃NO B | 34/19/**43**/4 | 54 |
| | | 0/0/**84**/16 | 85 |
| 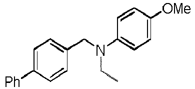 | A/Me₃NO B | - | - |
| | | 0/29/**41**/30 | 38 |
| 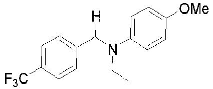 | B | 36/39/**25**/0 | 20 |

**Tableau 5. Exemplification avec divers aldéhydes aromatiques**

| 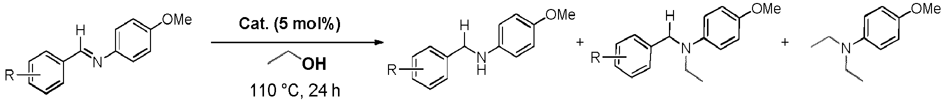 | | | |
|---|---|---|---|
| **Produit** | **Catalyseur** | **Ratio GC (départ/réduction/alkylation/dialkylation)** | **Rendement alkylation [%]** |
| 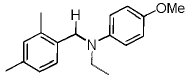 | A/Me₃NO B | - | - |
| | | 19/3/**78**/0 | 70 |
| 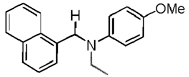 | A/Me₃NO B | - | 70 |
| | | - | 72 |
| 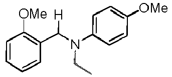 | A/Me₃NO B | 0/0/**83**/17 | 49 |
| | | 0/0/**72**/28 | 55 |
| 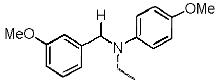 | A/Me₃NO B | 0/0/**92**/8 | 78 |
| | | 0/0/**90**/10 | 70 |
| 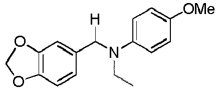 | A/Me₃NO B | - | - |
| | | 5/0/**90**/5 | 78 |
| 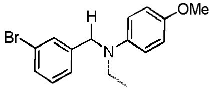 | A/Me₃NO B | - | - |
| | | 0/31/**29**/40 | 21 |
| 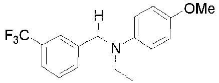 | B | 0/0/**15**/85 | 10 |

### Exemple 5 : Imines allylées

**Tableau 6. Exemplification avec divers aldéhydes conjugués**

| 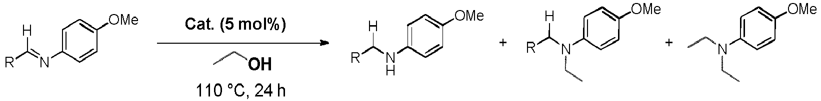 | | | |
|---|---|---|---|
| **Produit** | **Catalyseur** | **Ratio GC (départ/réduction/alkylation/dialkylation)** | **Rendement alkylation [%]** |
| 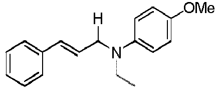 | A/Me₃NO B | - | - |
| | | | 67 |
| 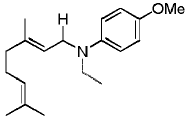 | A/Me₃NO B | - | 48 |
| | | | 30 |

### Exemple 6 : Imines alkylées

**Tableau 7. Exemplification avec divers aldéhydes aliphatiques**

| | | | |
|---|---|---|---|
| 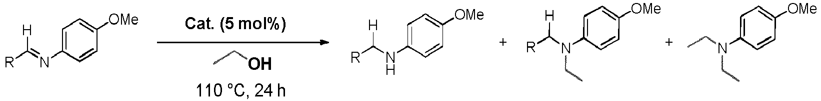 | | | |

| **Produit** | **Catalyseur** | **Ratio GC (départ/réduction/alkylation/dialkylation)** | **Rendement alkylation [%]** |
|---|---|---|---|
| 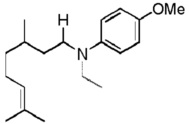 | A/Me₃NO B | - | 52 |
| | | 0/0/**78**/22 | 54 |
| 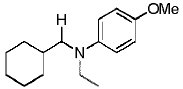 | A/Me₃NO B | - | - |
| | | 0/40/**17**/43 | 14 |

### Exemple 7 : Imines aromatiques

**Tableau 8. Exemplification avec diverses amines aromatiques**

| 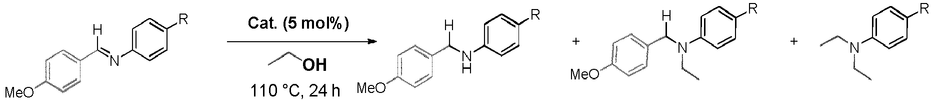 | | |
|---|---|---|
| **Produit** | **Ratio GC (départ/réduction/alkylation/dialkylation)** | **Rendement alkylation [%]** |
| 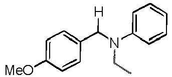 | 0/58/**42**/0 | 30 |
| 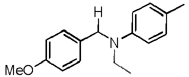 | 0/10/**90**/0 | 88 |
| 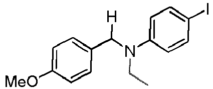 | 45/37/**18**/0 | 12 |
| 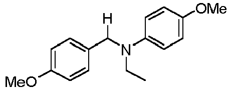 | 0/34/**36**/30 | 18 |

**Tableau 9. Exemplification avec diverses amines aliphatiques**

| 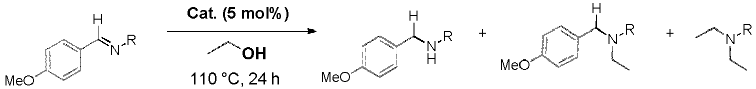 | | |
|---|---|---|
| **Produit** | **Ratio GC (départ/réduction/ alkylation/dialkylation)** | **Rendement alkylation [%]** |
| 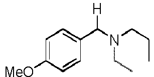 | 0/0/**100**/0 | 85 |
| 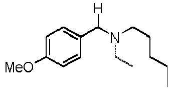 | 0/0/**100**/0 | 85 |
| 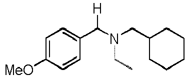 | 0/0/**67**/33 | 51 |
| 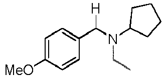 | 0/0/**100**/0 | 75 |
| 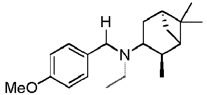 | 0/0/**100**/0 | 47 |
| 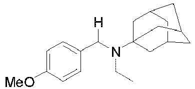 | 39/9/52/0 | 18 |

### Exemple 8 : Imines allylées

**Tableau 10. Exemplification avec diverses amines aromatiques substituées**

| 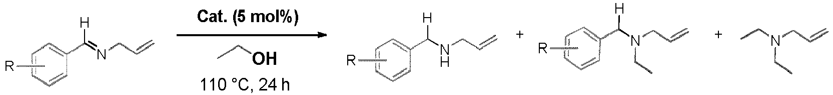 | | | |
|---|---|---|---|
| **Produit** | **Catalyseur** | **Ratio GC (départ/réduction/alkylation/dialkylation)** | **Rendement alkylation [%]** |
| 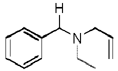 | A/Me₃NO B | - | - |
| | | 0/0/**100**/0 | 57 |
| 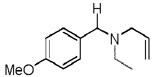 | A/Me₃NO B | - | - |
| | | 0/0/**100**/0 | 85 |
| 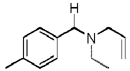 | A/Me₃NO B | - | - |
| | | Pas conversion totale | 38 |
| 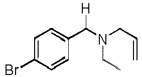 | A/Me₃NO B | - | - |
| | | 0/0/**100**/0 | 88 |
| 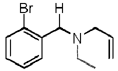 | A/Me₃NO B | - | - |
| | | 0/0/**100**/0 | 72 |
| 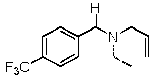 | A/Me₃NO B | - | - |
| | | 0/0/**100**/0 | 55 |
| 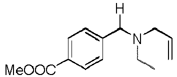 | A/Me₃NO B | - | - |
| | | 0/8/**92**/0 | 76 |
| 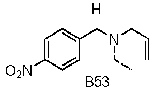 | B | 0/0/**100**/0 | 15 |

**Tableau 11. Exemplification avec diverses amines allyliques ou aliphatiques**

| | | | |
|---|---|---|---|
| 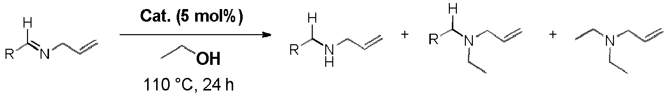 | | | |

| **Produit** | **Catalyseur** | **Ratio GC (départ/réduction/alkylation/dialkylation)** | **Rendement alkylation [%]** |
|---|---|---|---|
| 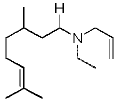 | B | 0/0/**100**/0 | 22 |

### Exemple 9 : Autres imines

**Tableau 12. Exemplification avec diverses imines**

| 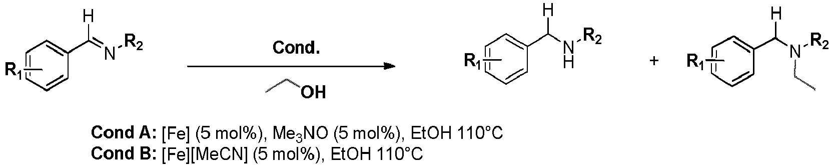 | | | |
|---|---|---|---|
| **Produit** | **Condition** | **Ratio GC (départ/réduction/ alkylation/dialkylation)** | **Rendement alkylation** |
| 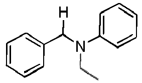 | A | - | - |
| | B | 23/42/**35**/0 | 30 |
| 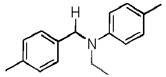 | A | - | - |
| | B | 0/4/**96**/0 | 78 |
| 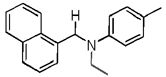 | A | - | - |
| | B | 0/47/**35**/18 | 35 |
| 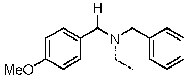 | A | - | - |
| | B | 0/0/**90**/10 ou 0/0/**83**/17 | 71 |
| 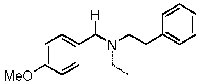 | A | - | - |
| | B | 0/27/**73**/0 (ou 27 de dép) | 53 |
| 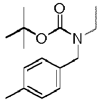 | A | - | - |
| | B | 0/0/**100**/0 | 29 |
| 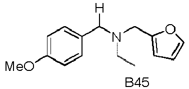 | B | 0/0/**87**/13 | 80 |

### Exemple 10 : Version intermoléculaire

**Tableau 13. Optimisation sur les substrats modèles en version intermoléculaire**

| | | | | |
|---|---|---|---|---|
| 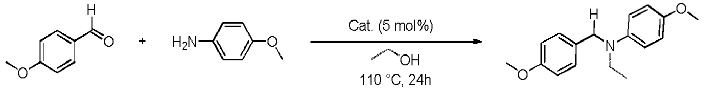 | | | | |

| **Catalyseur** | **Additif** | **Température [°C]** | **Ratio GC(départ/réduction/alkylation/dialkylation)** | **Rendement [%]** |
|---|---|---|---|---|
| B | - | 110 | 0/0/**68**/32 | 65 |

**Tableau 14. Exemplification de la réaction en version intermoléculaire**

| 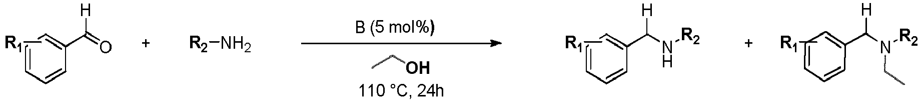 | | |
|---|---|---|
| **Produit** | **Ratio GC (départ/réduction/ alkylation/dialkylation)** | **Rendement alkylation [%]** |
| 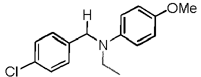 | 18/0/**34**/48 ou 0/18/**34**/48 | 10 |
| 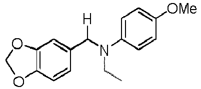 | 48/0/**34**/19 | 26 |
| 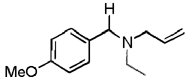 | 0/0/**54**/46 | 35 |
| 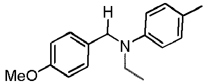 | 51/0/**29**/20 | 19 |

### Réactions d'alkylations des imines (Conditions A)

Dans la boîte à gants, le catalyseur A (0.05 équiv), l'oxyde de triméthylamine (0.05 équiv), de l'éthanol préalablement distillé puis dégazé (0.05 M) sont introduits dans un tube équipé d'un agitateur magnétique. Le mélange réactionnel est agité à température ambiante pendant 30 minutes. Le substrat (50 mg, 1 équiv) est ensuite ajouté, le tube est scellé avec un bouchon en téflon et sorti de boîte à gants. Par la suite, le tube réactionnel est immergé dans un bain préalablement chauffé à 110 °C et agité pendant 24 heures. Après un retour à température ambiante, la réaction est stoppée par l'ajout de méthanol (1 ml) et d'hydroxyde de sodium (1 ml, 1 M). Les phases organiques sont extraites avec 3 × 5 ml d'éther diéthylique, lavées avec une solution saturée de chlorure de sodium (5 ml), puis séchées sur du sulfate de magnésium et concentrées sous pression réduite. Le produit brut est purifié par chromatographie sur colonne de gel de silice (12 g de SiO₂ déjà traité par 5% de triéthylamine, cyclohexane/acétate d'éthyle, 95/5) afin d'obtenir l'alkyle amine désirée.

### Réactions d'alkylations des imines (Conditions B)

Dans un tube de catalyse (10 ml), équipé d'un agitateur magnétique, le substrat (50 mg, 1 équiv), le catalyseur B (0.05 équiv) et de l'éthanol préalablement distillé puis dégazé (0.05 M) sont introduits. Le tube est mis sous argon puis scellé avec un bouchon en téflon. Par la suite, le tube réactionnel est immergé dans un bain préalablement chauffé à 110 °C et agité pendant 24 heures. Après un retour à température ambiante, la réaction est stoppée par l'ajout de méthanol (1 ml) et d'hydroxyde de sodium (1 ml, 1 M). Les phases organiques sont extraites avec 3 × 5 ml d'éther diéthylique, lavées avec une solution saturée de chlorure de sodium (5 ml), puis séchées sur du sulfate de magnésium et concentrées sous pression réduite. Le produit brut est purifié par chromatographie sur colonne de gel de silice (12 g de SiO₂ déjà traité par 5% de triéthylamine, cyclohexane/acétate d'éthyle, 95/5) afin d'obtenir l'alkyle amine désirée.

## Revendications

1. Utilisation d'un alcool de formule (**C**)
**(C)** R₄OH
dans laquelle R₄ représente un alkyle en C₂ à C₁₀, ou un cycloalkyle en C₃ à C₁₀, le dit alcool de formule (**C**) étant notamment l'éthanol,
et d'une imine de formule (**D**) 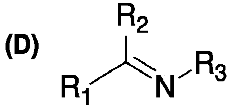
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
dans la mise en oeuvre d'un procédé de préparation d'amines tertiaires de formule (**E**) 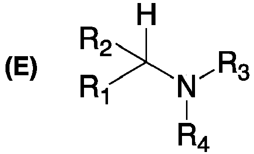
dans laquelle R₁, R₂, R₃, R₄ sont tels que définis ci-dessus,
dans laquelle la préparation d'amines tertiaires est réalisée en présence d'un catalyseurcomprenant un complexe de Fer(0).

2. Utilisation selon la revendication 1, dans laquelle le catalyseur comprenant un complexe de fer (0) est choisi parmi les formules suivantes : 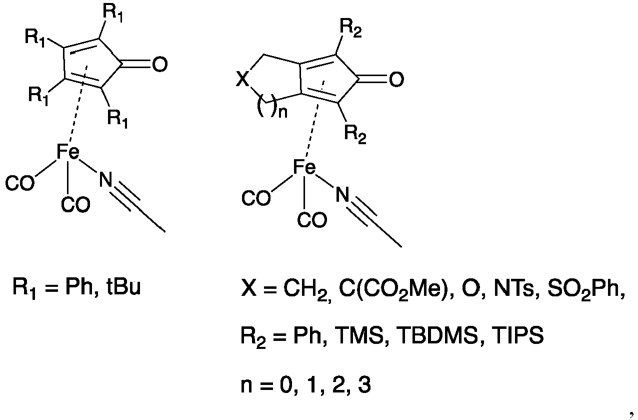 dans lesquelles
Ts = tosyle 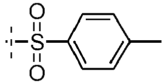
TMS = triméthylsilyle 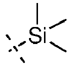
TBDMS = tert-butyldiméthylsilyle 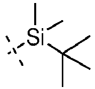
TIPS = triisopropylsilyle

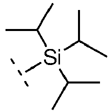

3. Utilisation selon la revendication 1 ou 2, dans laquelle ledit catalyseur répond à la formule (**B**) suivante : 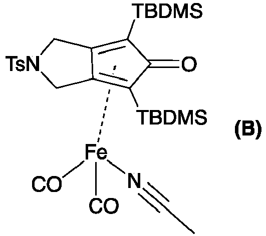 dans laquelle

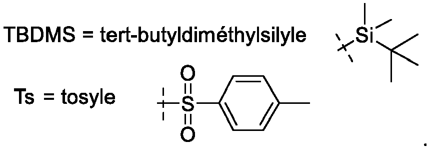

4. Procédé de préparation d'amines tertiaires de formule (**E**) 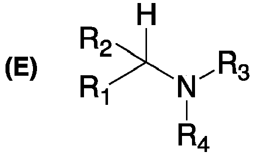
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀, la dite amine tertiaire de formule (**E**) portant notamment trois substituants différents entre eux et différents d'un atome d'hydrogène;
comprenant une étape d'alkylation d'un alcool de formule (**C**)
**(C)** R₄OH
dans laquelle R₄ est tel que défini ci-dessus, le dit alcool de formule (**C**) étant notamment l'éthanol,
sur une imine de formule (**D**) 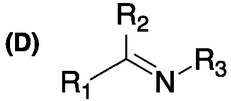
dans laquelle R₁, R₂ et R₃ sont tels que définis ci-dessus,
pour obtenir le composé de formule (**E**) tel que défini ci-dessus,
dans lequel l'étape d'alkylation est catalysée par un complexe de Fer(0).

5. Procédé selon la revendication 4, dans lequel le complexe de Fer(0) est choisi parmi les formules suivantes : 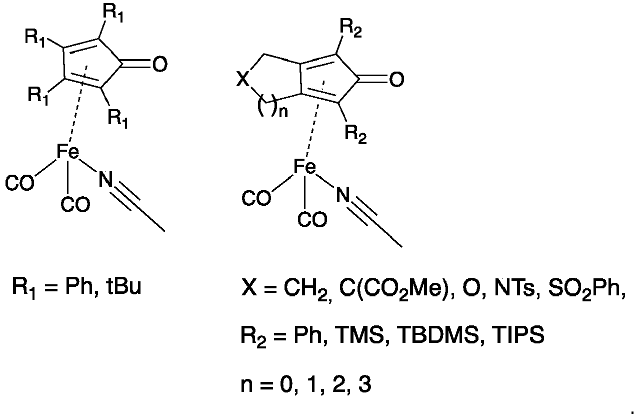 dans lesquelles
Ts = tosyle 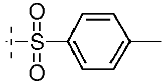
TMS = triméthylsilyle 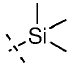
TBDMS = tert-butyldiméthylsilyle 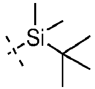
TIPS = triisopropylsilyle

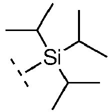

6. Procédé selon la revendication 4 ou 5, dans lequel ledit catalyseur répond à la formule (**B**) suivante : 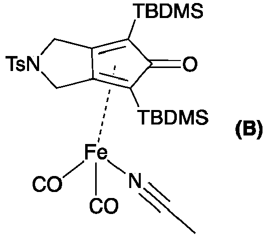 dans laquelle

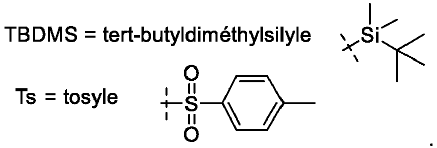

7. Procédé selon l'une des revendications 4 à 6, dans lequel le catalyseur utilisé est soit le complexe de formule (**B**) formé préalablement à la préparation des amines tertiaires, soit le catalyseur formé *in situ* pendant ladite préparation des amines tertiaires par ajout d'oxyde de triméthylamine sur le complexe de formule (**A**)

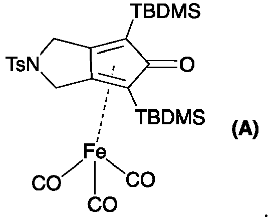

8. Procédé selon l'une des revendications 4 à 7, dans lequel l'étape d'alkylation est effectuée dans un solvant organique, notamment dans l'éthanol ou l'éthylène glycol ou dans un mélange de solvants composé notamment de THF et d'éthanol ou d'éthylène glycol.

9. Procédé selon l'une des revendications 4 à 8, dans lequel l'alcool primaire de formule (C), notamment l'éthanol ou l'éthylène glycol, est également utilisé comme solvant de l'étape d'alkylation, notamment à un nombre d'équivalents supérieur à 10 équivalents, et de préférence 85 équivalents.

10. Procédé selon l'une des revendications 4 à 9, dans lequel l'imine de formule (**D**) est soit formée préalablement à la préparation des amines tertiaires, soit formée *in situ* pendant ladite préparation des amines tertiaires par un procédé comprenant la mise en contact d'un aldéhyde ou d'une cétone de formule (**F**) 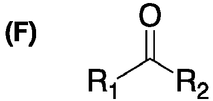
dans laquelle R₁, R₂ représentent un hydrogène, aryle, allyle, alkyle en C₁ à C₁₀, ou cycloalkyle en C₃ à C₁₀,
et d'une amine de formule (**G**)
**(G)** R₃-NH₂
dans laquelle R₃ représente un aryle, allyle, alkyle en C₁ à C₁₀, cycloalkyle en C₃ à C₁₀, un dérivé carbonylé en C₁ à C₁₀, ou un formiate en C₃ à C₁₀.

11. Procédé selon l'une des revendications 5 ou 6, comprenant une étape de préparation d'amines tertiaires de formule (**E**) 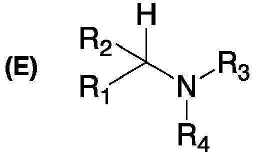
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
R₄ représente un alkyle en C₂ à C₁₀, ou un cycloalkyle en C₃ à C₁₀ ;
comprenant une étape d'alkylation d'un alcool de formule (**C**)
**(C)** R₄OH
dans laquelle R₄ est tel que défini ci-dessus,
sur une imine de formule (**D**) 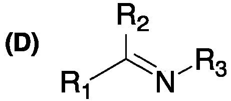
dans laquelle R₁, R₂ et R₃ sont tels que définis ci-dessus,
en présence d'un catalyseur de formule (**B**) 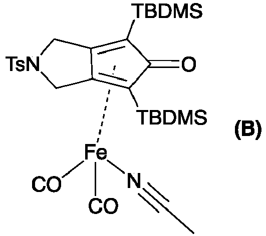
pour obtenir le composé de formule (**E**) tel que défini ci-dessus, ou
comprenant une première étape de préparation du catalyseur,
comprenant une étape d'ajout d'oxyde de triméthylamine sur le complexe de formule (**A**) 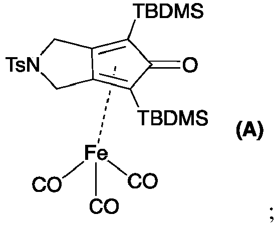
et une seconde étape d'alkylation d'un alcool de formule (**C**)
**(C)** R₄OH
dans laquelle R₄ représente un alkyle en C₂ à C₁₀, ou un cycloalkyle en C₃ à C₁₀, sur une imine de formule (**D**) 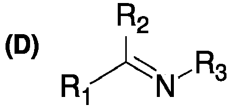
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀ ,en présence d'un catalyseur de formule (**B**) préparé à l'étape précédente et tel que défini ci-dessus,
pour obtenir le composé de formule (**E**) 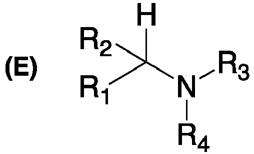
dans laquelle R₁, R₂, R₃ et R₄ sont tels que définis ci-dessus, ou
comprenant une première étape de préparation d'imines de formule (**D**) 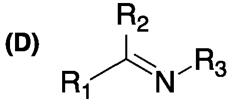
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
comprenant une étape de mise en contact d'un aldéhyde ou d'une cétone de formule (**F**) 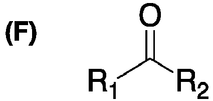
dans laquelle R₁ et R₂ sont tels que définis ci-dessus,
et d'une amine de formule (**G**)
**(G)** R₃-NH₂
dans laquelle R₃ est tel que défini ci-dessus,
pour obtenir l'imine de formule (**D**) telle que défini ci-dessus ;
et une seconde étape d'alkylation d'un alcool de formule (**C**)
**(C)** R₄OH
dans laquelle R₄ représente un alkyle en C₂ à C₁₀, ou un cycloalkyle en C₃ à C₁₀
sur une imine de formule (**D**) préparé à l'étape précédente et telle que définie ci-dessus, en présence d'un catalyseur de formule (**B**) 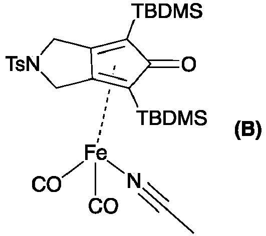
pour obtenir le composé de formule (**E**) 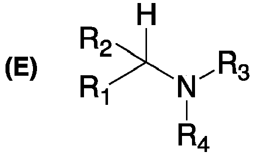
dans laquelle R₁, R₂, R₃ et R₄ sont tels que définis ci-dessus, ou
comprenant une étape préalable de préparation d'imines de formule (**D**) 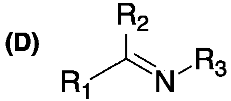
dans laquelle R₁ et R₂ représentent un hydrogène, aryle, allyle, alkyle en C₁ à C₁₀, ou cycloalkyle en C₃ à C₁₀,
R₃ représente un aryle, allyle, alkyle en C₁ à C₁₀, cycloalkyle en C₃ à C₁₀, un dérivé carbonylé en C₁ à C₁₀, ou un formiate en C₃ à C₁₀,
comprenant une étape de mise en contact d'un aldéhyde ou une cétone de formule (**F**) 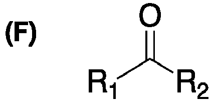
dans laquelle R₁ et R₂ sont tels que définis ci-dessus,
et d'une amine de formule (**G**)
**(G)** R₃-NH₂
dans laquelle R₃ est tel que défini ci-dessus,
pour obtenir l'imine de formule de formule (**D**) telle que défini ci-dessus ;
une étape préalable de préparation du catalyseur,
comprenant une étape d'ajout d'oxyde de triméthylamine sur le complexe de formule (**A**) : 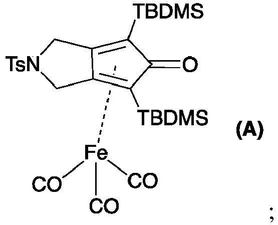
les deux susdites étapes préalables pouvant intervenir dans un ordre quelconque,
et une étape d'alkylation d'un alcool primaire ou secondaire de formule (**C**)
**(C)** R₄OH
dans laquelle R₄ représente un alkyle en C₂ à C₁₀, ou cycloalkyle en C₃ à C₁₀ sur une imine de formule (**D**) préparée à l'étape précédente 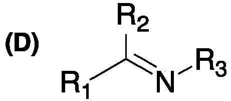
dans laquelle R₁, R₂ et R₃ sont tels que définis ci-dessus,
en présence du catalyseur de formule (**B**) préparé à l'étape précédente et tel que défini ci-dessus,
pour obtenir le composé de formule (**E**) 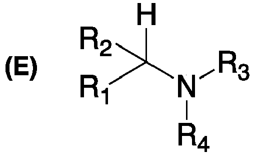
dans laquelle R₁, R₂, R₃ et R₄ sont tels que définis ci-dessus.

12. Procédé selon l'une des revendications 4 à 11, dans lequel ledit amine tertiaire de formule (**E**) répond à l'une des formules suivantes :

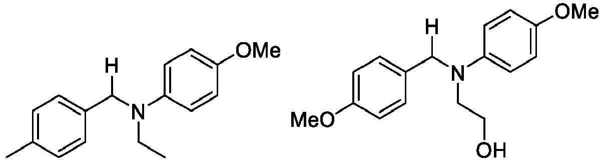

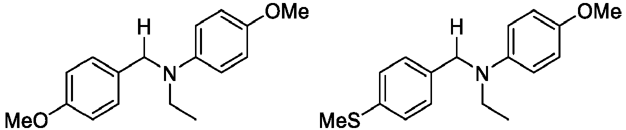

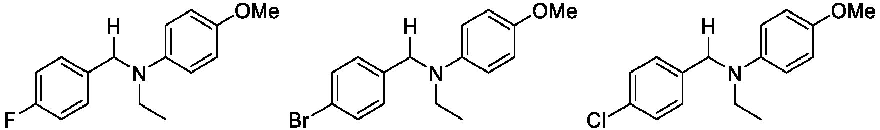

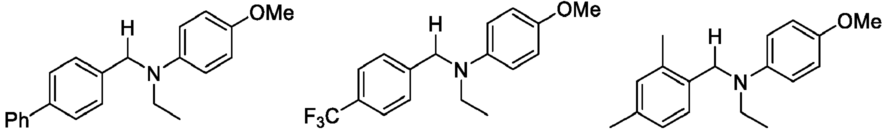

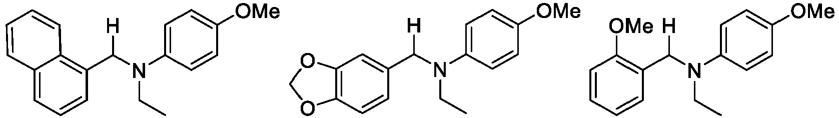

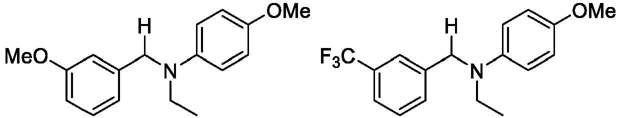

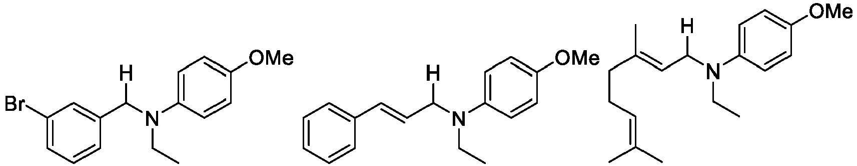

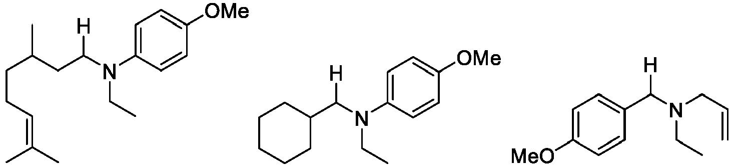

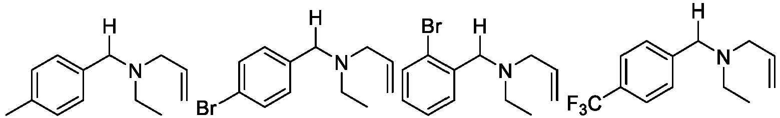

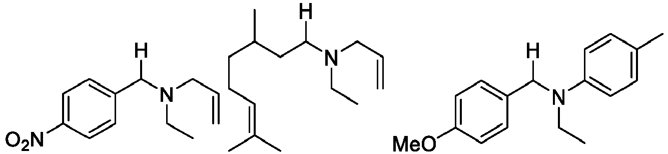

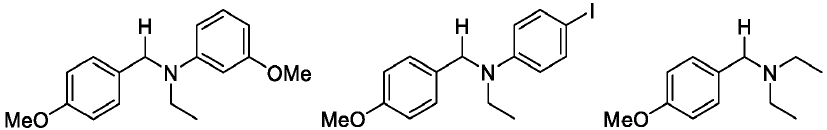

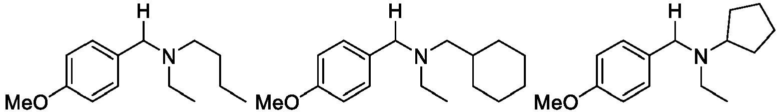

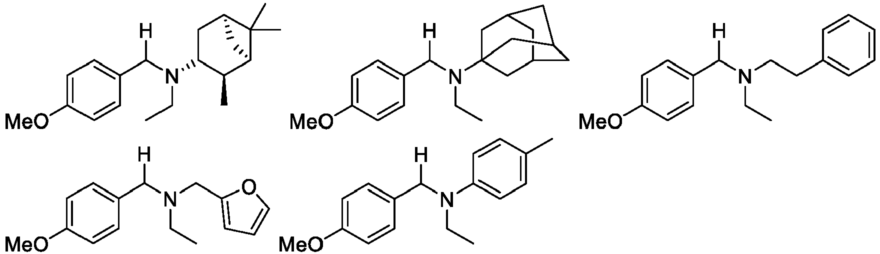

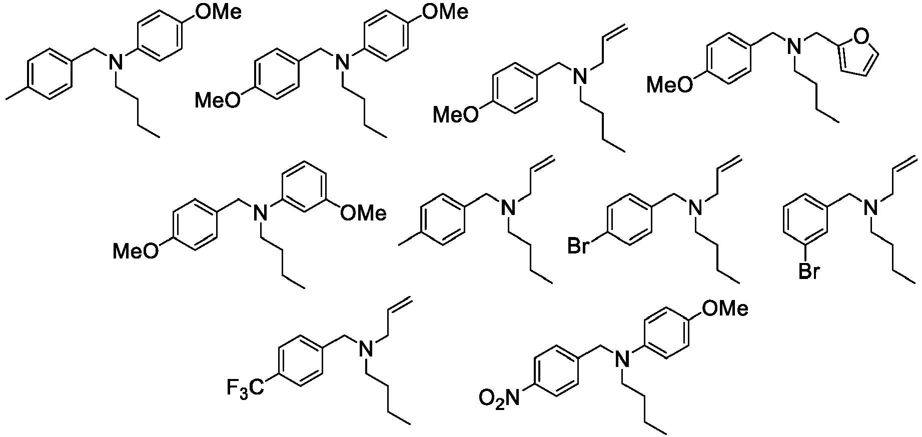

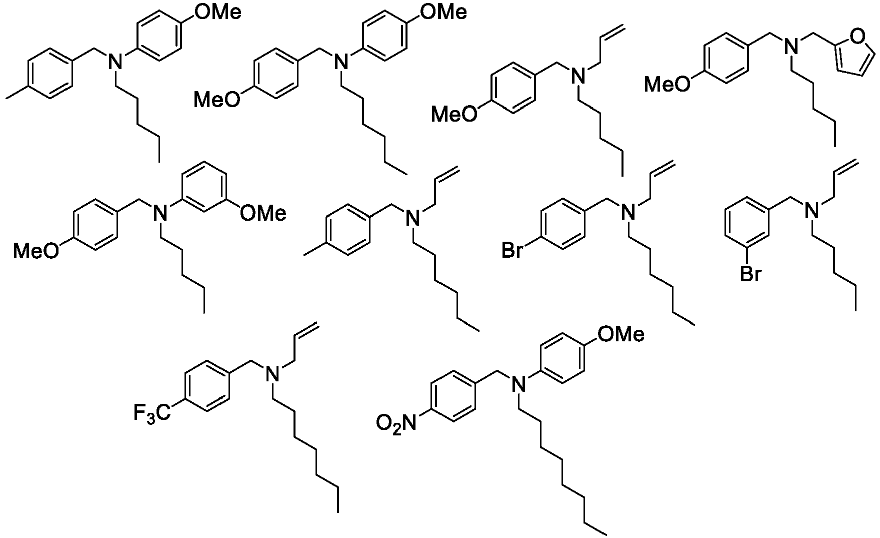

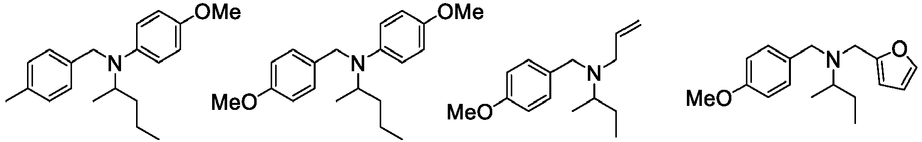

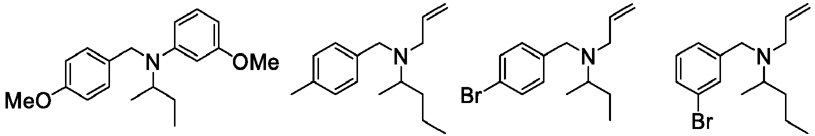

## Patentansprüche

1. Verwendung eines Alkohols mit der Formel (C)
(C) R₄OH
wobei R₄ ein C₂-C₁₀-Alkyl oder ein C₃-C₁₀-Cycloalkyl darstellt, wobei der genannte Alkohol mit der Formel (C) insbesondere Ethanol ist,
und eines Imins mit der Formel (D) 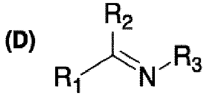
wobei R₁ und R₂ Wasserstoff, Aryl, Allyl, C₁-C₁₀-Alkyl oder C₃-C₁₀-Cycloalkyl darstellen,
R₃ Aryl, Allyl, C₁-C₁₀-Alkyl, C₃-C₁₀-Cycloalkyl, ein C₁-C₁₀-Carbonylderivat oder C₃-C₁₀-Formiat darstellt,
bei der Durchführung eines Verfahrens zur Herstellung von tertiären Aminen mit der Formel (E) 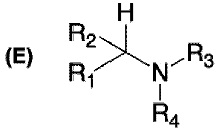
wobei R₁, R₂, R₃, R₄ wie hier im Vorstehenden definiert sind,
wobei die Herstellung der tertiären Amine in Anwesenheit eines Katalysators vorgenommen wird, der einen Eisen(0)-Komplex umfasst.

2. Verwendung nach Anspruch 1, wobei der Katalysator, der einen Eisen(0)-Komplex umfasst, aus den folgenden Formeln ausgewählt wird: 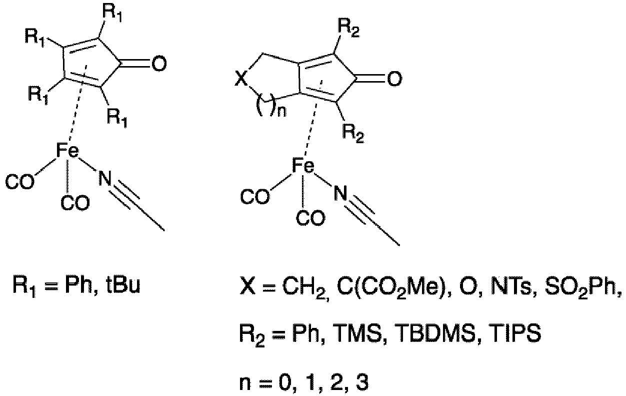 wobei
Ts = Tosyl 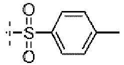
TMS = Trimethylsilyl 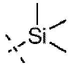
TBDMS = tert.Butyldimethylsilyl 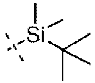
TIPS = Triisopropylsilyl

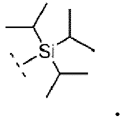

3. Verwendung nach Anspruch 1 oder 2, wobei der genannte Katalysator die folgende Formel (B) hat: 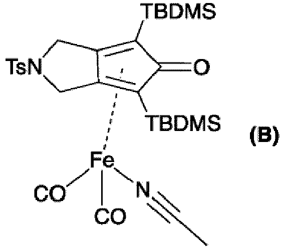 wobei
TBDMS = tert.Butyldimethylsilyl 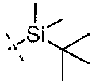
Ts = Tosyl

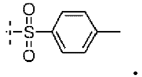

4. Verfahren zur Herstellung von tertiären Aminen mit der Formel (E) 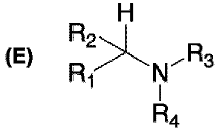
wobei R₁ und R₂ Wasserstoff, Aryl, Allyl, C₁-C₁₀-Alkyl oder C₃-C₁₀-Cycloalkyl darstellen,
R₃ Aryl, Allyl, C₁-C₁₀-Alkyl, C₃-C₁₀-Cycloalkyl, ein C₁-C₁₀-Carbonylderivat oder C₃-C₁₀-Formiat darstellt,
R₄ C₂-C₁₀-Alkyl oder C₃-C₁₀-Cycloalkyl darstellt, wobei das genannte tertiäre Amin mit der Formel (E) insbesondere drei Substituenten trägt, die voneinander verschieden und von einem Wasserstoffatom verschieden sind;
umfassend einen Alkylierungsschritt eines Alkohols mit der Formel (C)
(C) R₄OH
wobei R₄ wie hier im Vorstehenden definiert ist, wobei der genannte Alkohol mit der Formel (C) insbesondere Ethanol ist,
über ein Imin mit der Formel (D) 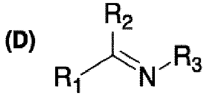
wobei R₁, R₂ und R₃ wie hier im Vorstehenden definiert sind,
um die Verbindung mit der Formel (E) zu erhalten, wie hier im Vorstehenden definiert,
wobei der Alkylierungsschritt durch einen Eisen(0)-Komplex katalysiert wird.

5. Verfahren nach Anspruch 4, wobei der Eisen(0)-Komplex aus den folgenden Formeln ausgewählt wird: 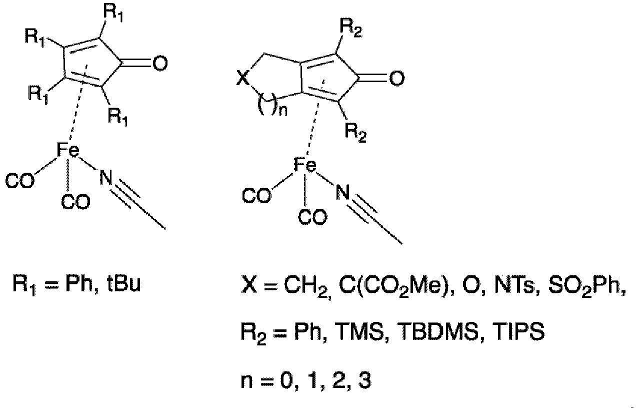 wobei
Ts = Tosyl 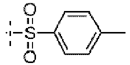
TMS = Trimethylsilyl 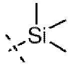
TBDMS = tert.Butyldimethylsilyl 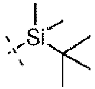
TIPS = Triisopropylsilyl

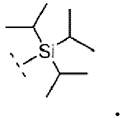

6. Verfahren nach Anspruch 4 oder 5, wobei der genannte Katalysator die folgende Formel (B) hat: 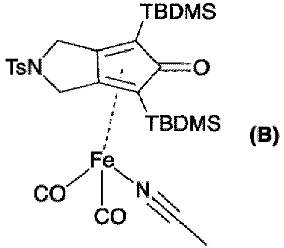 wobei
TBDMS = tert.Butyldimethylsilyl 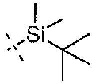
Ts = Tosyl

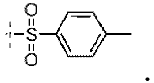

7. Verfahren nach einem der Ansprüche 4 bis 6, wobei der verwendete Katalysator entweder der Komplex mit der Formel (B) ist, der vor der Herstellung der tertiären Amine gebildet wird, oder der Katalysator, der *in situ* während der genannten Herstellung der tertiären Amine durch Zusatz von Trimethylaminoxid zu dem Komplex mit der Formel (A) gebildet wird

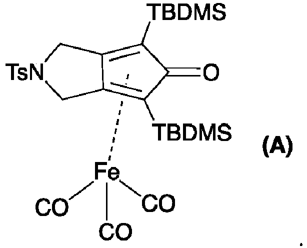

8. Verfahren nach einem der Ansprüche 4 bis 7, wobei der Alkylierungsschritt in einem organischen Lösungsmittel vorgenommen wird, insbesondere in Ethanol oder Ethlenglykol, oder in einer Mischung von Lösungsmitteln, insbesondere bestehend aus THF und Ethanol oder Ethylenglykol.

9. Verfahren nach einem der Ansprüche 4 bis 8, wobei der primäre Alkohol mit der Formel (C), insbesondere Ethanol oder Ethylenglykol, auch als Lösungsmittel des Alkylierungsschritts verwendet wird, insbesondere mit einer Anzahl von Äquivalenten größer als 10 Äquivalente, und vorzugsweise 85 Äquivalenten.

10. Verfahren nach einem der Ansprüche 4 bis 9, wobei das Imin mit der Formel (D) entweder vor der Herstellung der tertiären Amine gebildet wird, oder *in situ* während der genannten Herstellung der tertiären Amine gebildet wird, durch ein Verfahren, umfassend das Inberührungbringen eines Aldehyds oder eines Ketons mit der Formel (F) 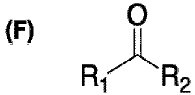
wobei R₁, R₂ Wasserstoff, Aryl, Allyl, C₁-C₁₀-Alkyl oder C₃-C₁₀-Cycloalkyl darstellen,
und eines Amins mit der Formel (G)
(G) R₃-NH₂
wobei R₃ Aryl, Allyl, C₁-C₁₀-Alkyl, C₃-C₁₀-Cycloalkyl, ein C₁-C₁₀-Carbonylderivat oder C₃-C₁₀-Formiat darstellt.

11. Verfahren nach einem der Ansprüche 5 oder 6, umfassend einen Schritt zur Herstellung von tertiären Aminen mit der Formel (E) 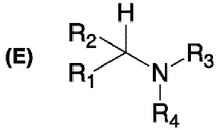
wobei R₁ und R₂ Wasserstoff, Aryl, Allyl, C₁-C₁₀-Alkyl oder C₃-C₁₀-Cycloalkyl darstellen,
R₃ Aryl, Allyl, C₁-C₁₀-Alkyl, C₃-C₁₀-Cycloalkyl, ein C₁-C₁₀-Carbonylderivat oder C₃-C₁₀-Formiat darstellt,
R₄ C₂-C₁₀-Alkyl oder C₃-C₁₀-Cycloalkyl darstellt;
umfassend einen Alkylierungsschritt eines Alkohols mit der Formel (C)
(C) R₄OH
wobei R₄ wie hier im Vorstehenden definiert ist,
über ein Imin mit der Formel (D) 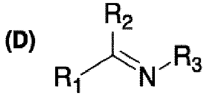
wobei R₁, R₂ und R₃ wie hier im Vorstehenden definiert sind,
in Anwesenheit eines Katalysators mit der Formel (B) 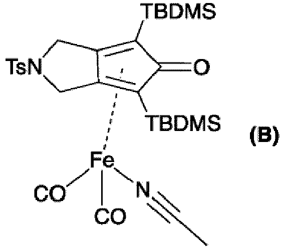
um die Verbindung mit der Formel (E) zu erhalten, wie hier im Vorstehenden definiert, oder
umfassend einen ersten Schritt zur Herstellung des Katalysators,
umfassend einen Schritt zum Zusetzen von Trimethylaminoxid zu dem Komplex mit der Formel (A) 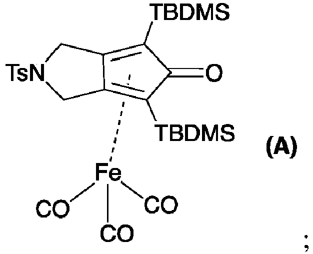
und einen zweiten Alkylierungsschritt eines Alkohols mit der Formel (C)
(C) R₄OH
wobei R₄ C₂-C₁₀-Alkyl oder C₃-C₁₀-Cycloalkyl darstellt,
über ein Imin mit der Formel (D) 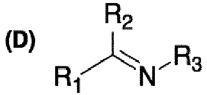
wobei R₁ und R₂ Wasserstoff, Aryl, Allyl, C₁-C₁₀-Alkyl oder C₃-C₁₀-Cycloalkyl darstellen,
R₃ Aryl, Allyl, C₁-C₁₀-Alkyl, C₃-C₁₀-Cycloalkyl, ein C₁-C₁₀-Carbonylderivat oder C₃-C₁₀-Formiat darstellt, in Anwesenheit eines Katalysators mit der Formel (B), der in dem vorhergehenden Schritt hergestellt wird und wie hier im Vorstehenden definiert,
um die Verbindung mit der Formel (E) zu erhalten 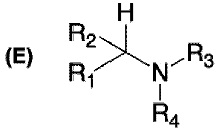
wobei R₁, R₂, R₃ und R₄ wie hier im Vorstehenden definiert sind, oder
umfassend einen ersten Schritt zur Herstellung von Iminen mit der Formel (D) 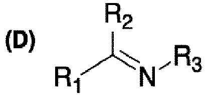
wobei R₁ und R₂ Wasserstoff, Aryl, Allyl, C₁-C₁₀-Alkyl oder C₃-C₁₀-Cycloalkyl darstellen,
R₃ Aryl, Allyl, C₁-C₁₀-Alkyl, C₃-C₁₀-Cycloalkyl, ein C₁-C₁₀-Carbonylderivat oder C₃-C₁₀-Formiat darstellt,
umfassend einen Schritt zum Inberührungbringen eines Aldehyds oder eines Ketons mit der Formel (F) 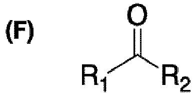
wobei R₁, R₂ wie hier im Vorstehenden definiert sind,
und eines Amins mit der Formel (G)
(G) R₃-NH₂
wobei R₃ wie hier im Vorstehenden definiert ist,
um das Imin mit der Formel (D) zu erhalten, wie hier im Vorstehenden definiert;
und einen zweiten Alkylierungsschritt eines Alkohols mit der Formel (C)
(C) R₄OH
wobei R₄ C₂-C₁₀-Alkyl oder C₃-C₁₀-Cycloalkyl darstellt,
über ein Imin mit der Formel (D), das im vorhergehenden Schritt hergestellt wird und wie hier im Vorstehenden definiert, in Anwesenheit eines Katalysators mit der Formel (B) 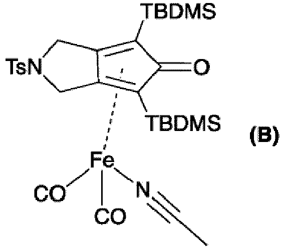
um die Verbindung mit der Formel (E) zu erhalten 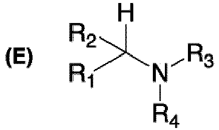
wobei R₁, R₂, R₃ und R₄ wie hier im Vorstehenden definiert sind, oder
umfassend einen vorherigen Schritt zur Herstellung von Iminen mit der Formel (D) 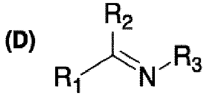
wobei R₁ und R₂ Wasserstoff, Aryl, Allyl, C₁-C₁₀-Alkyl oder C₃-C₁₀-Cycloalkyl darstellen,
R₃ Aryl, Allyl, C₁-C₁₀-Alkyl, C₃-C₁₀-Cycloalkyl, ein C₁-C₁₀-Carbonylderivat oder C₃-C₁₀-Formiat darstellt,
umfassend einen Schritt zum Inberührungbringen eines Aldehyds oder eines Ketons mit der Formel (F) 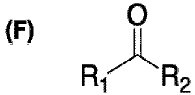
wobei R₁ und R₂ wie hier im Vorstehenden definiert sind,
und eines Amins mit der Formel (G)
(G) R₃-NH₂
wobei R₃ wie hier im Vorstehenden definiert ist,
um das Imin mit der Formel (D) zu erhalten, wie hier im Vorstehenden definiert;
einen vorherigen Schritt zur Herstellung des Katalysators,
umfassend einen Schritt zum Zusetzen von Trimethylaminoxid zu dem Komplex mit der Formel (A) 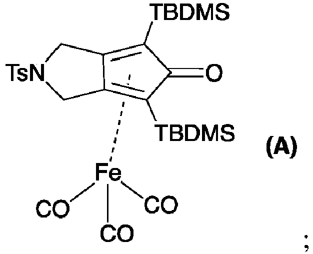
wobei die beiden im Vorstehenden genannten Schritte in einer beliebigen Reihenfolge erfolgen können,
und einen zweiten Alkylierungsschritt eines primären oder sekundären Alkohols mit der Formel (C)
(C) R₄OH
wobei R₄ C₂-C₁₀-Alkyl oder C₃-C₁₀-Cycloalkyl darstellt,
über ein Imin mit der Formel (D), das im vorhergehenden Schritt hergestellt wird 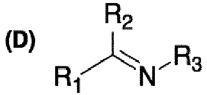
wobei R₁, R₂ und R₃ wie hier im Vorstehenden definiert sind,
in Anwesenheit des Katalysators mit der Formel (B), der im vorhergehenden Schritt hergestellt wird und wie hier im Vorstehenden definiert,
um die Verbindung mit der Formel (E) zu erhalten 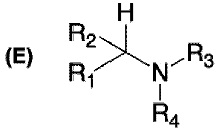
wobei R₁, R₂, R₃ und R₄ wie hier im Vorstehenden definiert sind.

12. Verfahren nach einem der Ansprüche 4 bis 11, wobei das genannte tertiäre Amin mit der Formel (E) eine der folgenden Formeln hat:

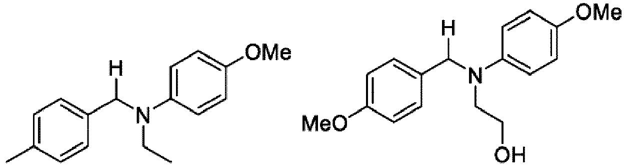

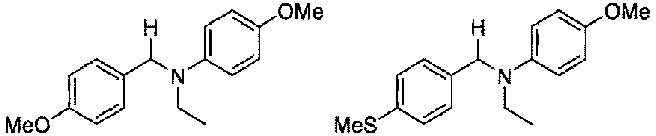

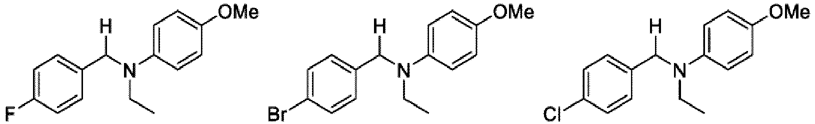

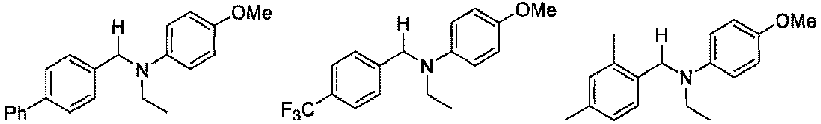

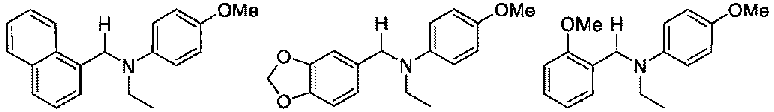

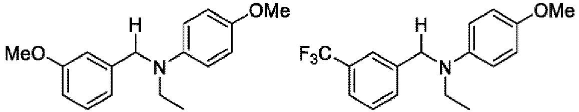

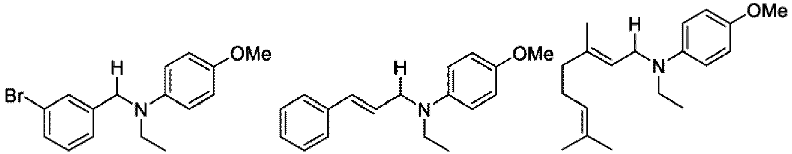

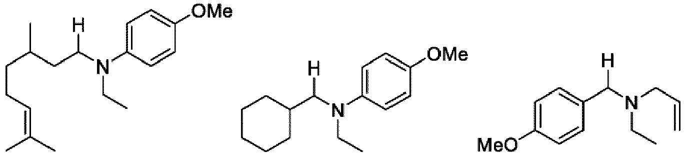

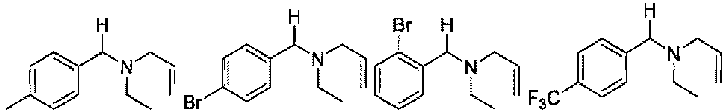

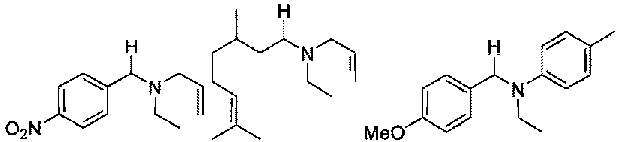

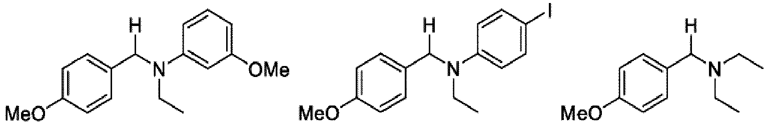

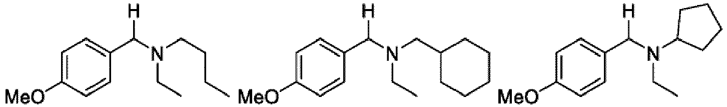

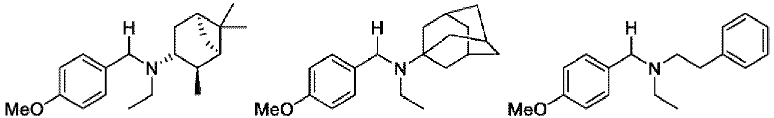

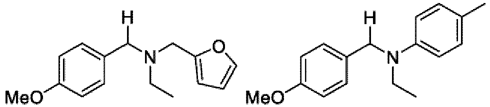

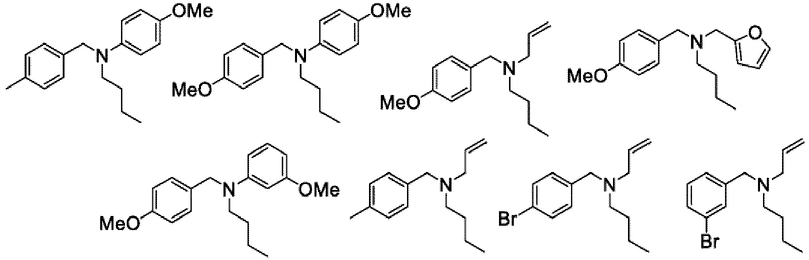

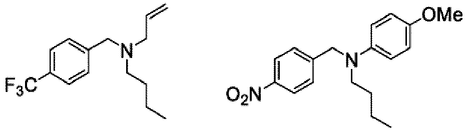

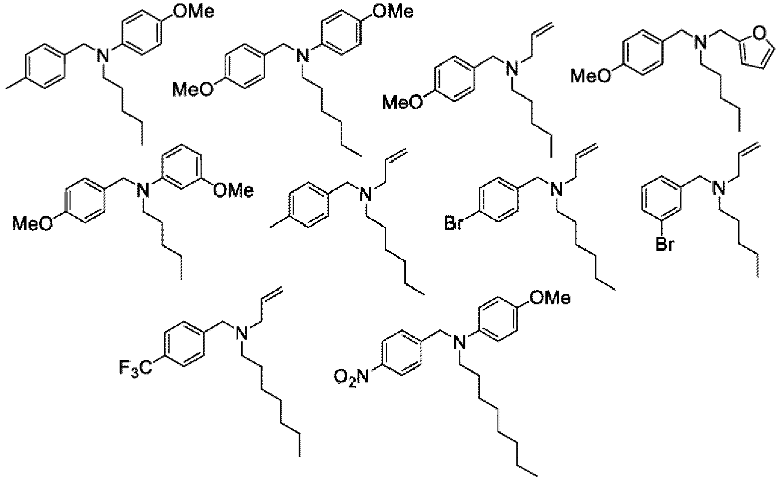

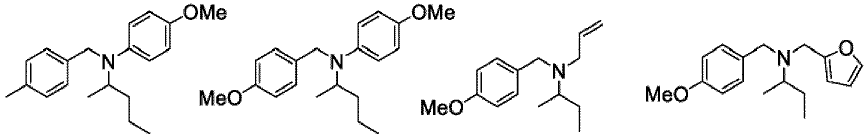

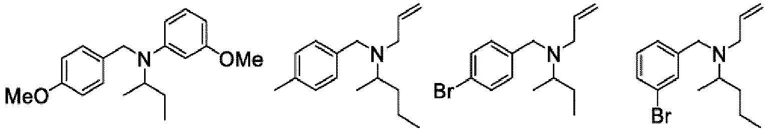

## Claims

1. Use of an alcohol of formula (**C**)
(C) R₄OH
wherein R₄ represents a C₂ to C₁₀ alkyl, or a C₃ to C₁₀ cycloalkyl, said alcohol of formula (C) being in particular ethanol,
and of an imine of formula (**D**) 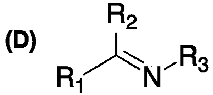
wherein R₁ and R₂ represent a hydrogen, aryl, allyl, C₁ to C₁₀ alkyl, or C₃ to C₁₀ cycloalkyl,
R₃ represents an aryl, allyl, C₁ to C₁₀ alkyl, C₃ to C₁₀ cycloalkyl, C₁ to C₁₀, a C₁ to C₁₀ carbonyl derivative, or a C₃ to C₁₀ formate,
in the implementation of a method of preparation of tertiary amines of formula (**E**) 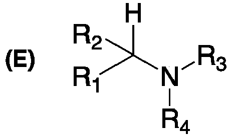
wherein R₁, R₂, R₃, R₄ are as defined above,
wherein the preparation of tertiary amines is performed in the presence of a catalyst comprising an iron(0) complex.

2. Use according to claim 1, wherein the catalyst comprising an iron(0) complex is chosen among the following formulas: 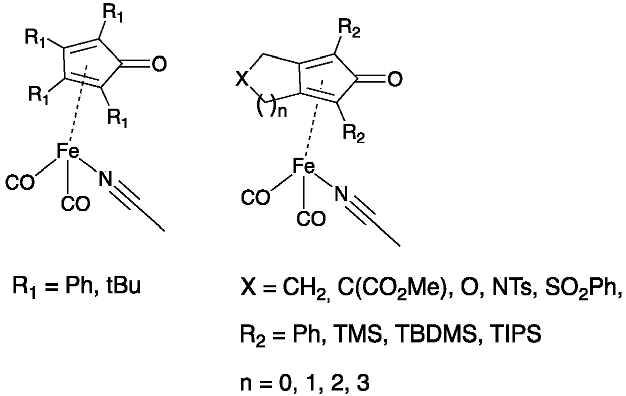 in which
Ts = tosyl 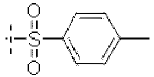
TMS = trimethylsilyl 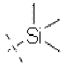
TBDMS = tert-butyldimethylsilyl 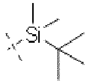
TIPS = triisopropylsilyl

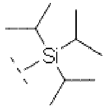

3. Use according to anyone of claims 1 or 2, wherein said catalyst has the following formula (**B**): 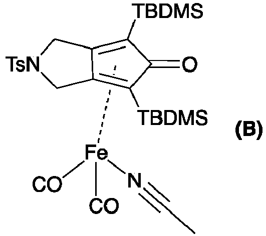 in which

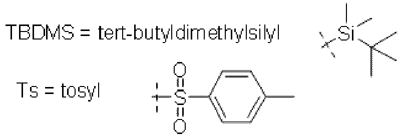

4. Method of preparation of tertiary amines of formula (**E**) 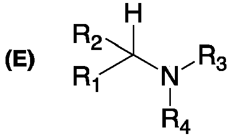
wherein R₁ and R₂ represent a hydrogen, aryl, allyl, C₁ to C₁₀ alkyl, or C₃ to C₁₀ cycloalkyl,
R₃ represents an aryl, allyl, C₁ to C₁₀ alkyl, C₃ to C₁₀ cycloalkyl, C₁ to C₁₀, a C₁ to C₁₀ carbonyl derivative, or a C₃ to C₁₀ formate,
R₄ represents a C₂ to C₁₀ alkyl, or C₃ to C₁₀ cycloalkyl, said tertiary amine of formula (**E**) carrying in particular three different substituents, different from a hydrogen atom;
comprising a step of alkylation of an alcohol of formula (**C**)
(C) R₄OH
wherein R₄ is as defined above, said alcohol of formula (**C**) being in particular ethanol, on an imine of formula (**D**) 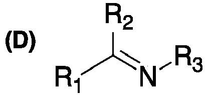
wherein R₁, R₂ and R₃ are as defined above,
to obtain the compound of formula (**E**) as defined above,
wherein the step of alkylation is catalyzed by an iron(0) complex.

5. Method according to claim 4, wherein the iron(0) complex is chosen among the following formulas: 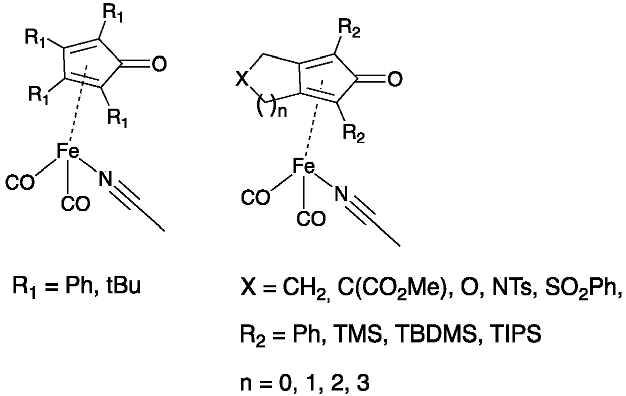 in which
Ts = tosyl 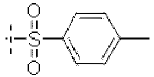
TMS = trimethylsilyl 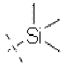
TBDMS = tert-butyldimethylsilyl 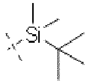
TIPS = triisopropylsilyl

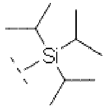

6. Method according to anyone of claims 4 or 5, wherein the catalyst has the following formula (**B**): 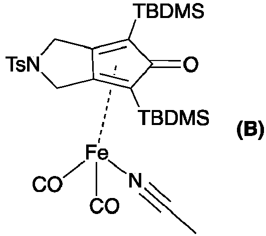 in which

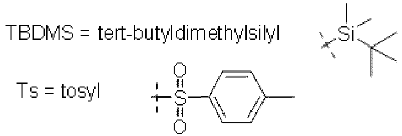

7. Method according to anyone of claims 4 to 6, wherein the used catalyst is either the complex of formula (**B**) formed prior to the preparation of tertiary amines, or is the catalyst formed *in situ* during said preparation of tertiary amines by adding trimethylamine oxide to the complex of formula (**A**)

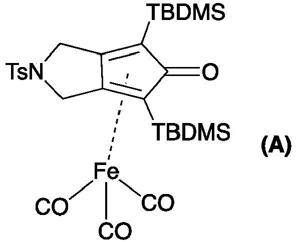

8. Method according to anyone of claims 4 to 7, wherein the step of alkylation is performed in an organic solvent, in particular in ethanol or ethylene glycol or in a mixture of solvents composed in particular of THF and of ethanol or of ethylene glycol.

9. Method according to anyone of claims 4 or 8, wherein the primary alcohol of formula (**C**), in particular ethanol or ethylene glycol, is also used as a solvent for the step of alkylation, in particular in a number of equivalents higher than 10 equivalents, and preferably 85 equivalents.

10. Method according to anyone of claims 4 or 9, wherein the imine of formula (**D**) is either formed prior to the preparation of the tertiary amines, or is formed *in situ* during said preparation of tertiary amines by a method comprising contacting an aldehyde or ketone of formula (**F**) 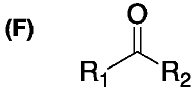
wherein R₁, R₂ represents a hydrogen, aryl, allyl, C₁ to C₁₀ alkyl, or C₃ to C₁₀ cycloalkyl, and an amine of formula (**G**)
**(G)** R₃-NH₂
wherein R₃ represents an aryl, allyl, C₁ to C₁₀ alkyl, C₃ to C₁₀ cycloalkyl, a C₁ to C₁₀ carbonyl derivative or a C₃ to C₁₀ formate.

11. Method according to one of claims 5 or 6, comprising a step of preparing tertiary amines of formula (**E**) 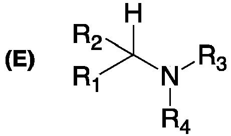
wherein R₁ and R₂ represent a hydrogen, aryl, allyl, C₁ to C₁₀ alkyl, or C₃ to C₁₀ cycloalkyl,
R₃ represents an aryl, allyl, C₁ to C₁₀ alkyl, C₃ to C₁₀ cycloalkyl, a C₁ to C₁₀ carbonyl derivative, or a C₃ to C₁₀ formate,
R₄ represents a C₂ to C₁₀ alkyl, or a C₃ to C₁₀ cycloalkyl;
comprising a step of alkylation of an alcohol of formula (**C**)
(C) R₄OH
wherein R₄ is as defined above,
on an imine of formula (**D**) 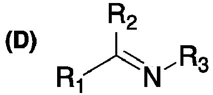
wherein R₁, R₂ and R₃ are as defined above,
in the presence of a catalyst of formula (**B**) 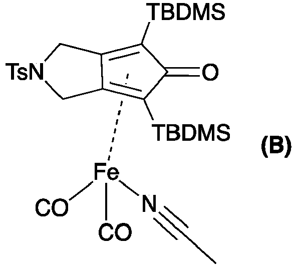
to obtain the compound of formula (**E**) as defined above, or
comprising a first step of preparing of the catalyst
comprising a step of adding trimethylamine oxide on the complex of formula (**A**) 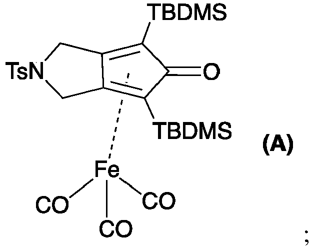
and a second step of alkylation of an alcohol of formula (**C**)
(C) R₄OH
wherein R₄ represents a C₂ to C₁₀ alkyl, or a C₃ to C₁₀ cycloalkyl,
on an imine of formula (**D**) 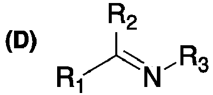
wherein R₁ and R₂ represent a hydrogen, aryl, allyl, C₁ to C₁₀ alkyl or a C₃ to C₁₀ cycloalkyl,
R₃ represents an aryl, allyl, C₁ to C₁₀ alkyl, C₃ to C₁₀ cycloalkyl, a C₁ to C₁₀ carbonyl derivative, or a C₃ to C₁₀ formate, in the presence of a catalyst of formula (**B**) prepared during the preceding step and as defined above,
to obtain the compound of formula (**E**) 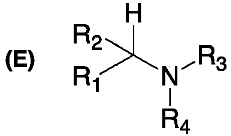
wherein R₁, R₂, R₃ and R₄ are as defined above, or
comprising a first step of preparing imines of formula (**D**) 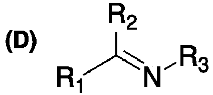
wherein R₁ and R₂ represent a hydrogen, aryl, allyl, C₁ to C₁₀ alkyl, or cycloalkyl C₃ to C₁₀,
R₃ represents an aryl, allyl, C₁ to C₁₀ alkyl, C₃ to C₁₀ cycloalkyl, a C₁ to C₁₀ carbonyl derivative, or a C₃ to C₁₀ formate,
comprising a step of contacting with an aldehyde or a ketone of formula (**F**) 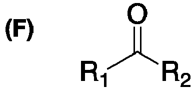
wherein R₁ and R₂ are as defined above,
and an amine of formula (**G**)
**(G)** R₃-NH₂
wherein R₃ is as defined above,
to obtain the imine of formula (**D**) as defined above;
and a second step of alkylation of an alcohol of formula (**C**)
**(C)** R₄OH
wherein R₄ represents a C₂ to C₁₀ alkyl, or a C₃ to C₁₀ cycloalkyl,
on an imine of formula (**D**) prepared in the preceding step and as defined above, in the presence of a catalyst of formula (**B**) 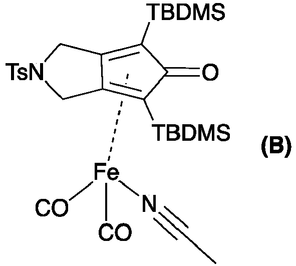
to obtain the compound of formula (**E**) 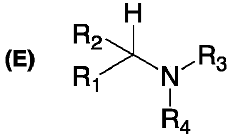
wherein R₁, R₂ , R₃ and R₄ are as defined above, or
comprising a prior step of preparing of imines of formula (**D**) 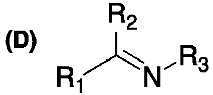
wherein R₁ and R₂ represent a hydrogen, aryl, allyl, C₁ to C₁₀ alkyl, or C₃ to C₁₀ cycloalkyl,
R₃ represents an aryl, allyl, C₁ to C₁₀ alkyl, C₃ to C₁₀ cycloalkyl, a C₁ to C₁₀ carbonyl derivative, or a C₃ to C₁₀ formate,
comprising a step of contacting an aldehyde or ketone of formula (**F**) 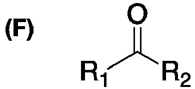
wherein R₁ and R₂ are as defined above,
and an amine of formula (**G**)
**(G) R₃-NH₂**
wherein R₃ is as defined above,
to obtain the imine of formula (**D**) as defined above;
a prior step of preparing the catalyst,
comprising a step of adding trimethylamine oxide to the complex of formula (**A**): 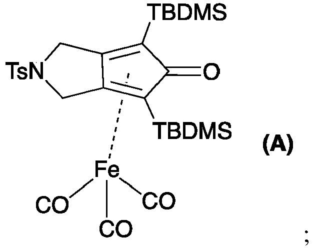
said two prior steps may occur in any order,
and a step of alkylation of an alcohol of formula (**C**)
(**C**) R₄OH
wherein R₄ represents a C₂ to C₁₀ alkyl, or a C₃ to C₁₀ cycloalkyl,
on an imine of formula (**D**) prepared in the previous step 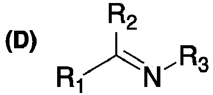
wherein R₁, R₂ and R₃ are as defined above,
in the presence of catalyst of formula (**B**) prepared in the preceding step and as above,
to obtain the compound of formula (**E**) 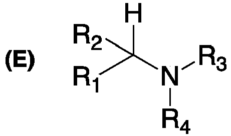
wherein R₁, R₂, R₃ and R₄ are as defined above.

12. Method according to any one of claims 4 to 11, wherein said tertiary amine of formula (**E**) has one of the following formulas:
